(19)

**Europäisches Patentamt**
**European Patent Office**
**Office européen des brevets**

(11)  **EP 4 527 854 A2**

(12)  **EUROPEAN PATENT APPLICATION**

(43) Date of publication:
26.03.2025  Bulletin 2025/13

(21) Application number: 24165507.5

(22) Date of filing: 09.03.2018

(51) International Patent Classification (IPC):
**C07K 16/28** (2006.01)

(52) Cooperative Patent Classification (CPC):
**C07K 16/2827; A61P 35/00; C07K 16/1045;**
**C07K 16/2809;** C07K 2317/21; C07K 2317/31;
C07K 2317/33; C07K 2317/732; C07K 2317/76;
C07K 2317/92

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: 09.03.2017  DK PA201700164
11.07.2017  DK PA201700408

(62) Document number(s) of the earlier application(s) in
accordance with Art. 76 EPC:
**18712124.9 / 3 592 769**

(71) Applicant: **Genmab A/S**
**1560 Copenhagen V (DK)**

(72) Inventors:
• **ALTINTAS, Isil**
**3584 CT Utrecht (NL)**
• **SATIJN, David**
**3584 CT Utrecht (NL)**
• **VAN DEN BRINK, Edward,N,**
**3584 CT Utrecht (NL)**

• **VERZIJL, Dennis**
**3584 CT Utrecht (NL)**
• **RADEMAKER, Rik**
**3584 CT Utrecht (NL)**
• **PARREN, Paul**
**3984 PR Odijk (NL)**
• **DE GOEIJ, Bart**
**3584 CT Utrecht (NL)**

(74) Representative: **Genmab A/S**
**Carl Jacobsens Vej 30**
**2500 Valby (DK)**

Remarks:
•The complete document including Reference
Table(s) and the Sequence Listing(s) can be
downloaded from the EPO website
•The application is published incomplete as filed
(Rule 68(1) EPC).
•This application was filed on 22-03-2024 as a
divisional application to the application mentioned
under INID code 62.
•The references to the drawing(s) no. 3D-F are
deemed to be deleted (Rule 56(4)(6) EPC).

(54)  **ANTIBODIES AGAINST PD-L1**

(57)  The present invention relates to novel antibodies and their use in medicine. In particular, the invention relates to bispecific antibodies capable of binding human PD-L1 and capable of binding human CD3. Novel classes of antibodies capable of binding human PD-L1 are also provided. The invention furthermore relates to uses of the antibodies of the invention and to methods, nucleic acid constructs and host cells for producing antibodies of the invention.

EP 4 527 854 A2

**Description**

**FIELD OF THE INVENTION**

The present invention relates to novel antibodies and their use in medicine. In particular, the invention relates to bispecific antibodies capable of binding human PD-L1 and capable of binding human CD3. Novel classes of antibodies capable of binding human PD-L1 are also provided. The invention furthermore relates to uses of the antibodies of the invention and to methods, nucleic acid constructs and host cells for producing antibodies of the invention.

**BACKGROUND OF THE INVENTION**

Programmed death ligand 1 (PD-L1, PDL1, CD274, B7H1, B7-H1) is a 33 kDa, single-pass type I membrane protein. Three isoforms of PD-L1 have been described, based on alternative splicing. PD-L1 belongs to the immunoglobulin (Ig) superfamily and contains one Ig-like C2-type domain and one Ig-like V-type domain. Freshly isolated T and B cells express negligible amounts of PD-L1 and a fraction (about 16%) of CD14+ monocytes constitutively express PD-L1 (Rietz and Chen, 2004 Am J Transplant 4: 8-14). Interferon-$\gamma$ (IFN-$\gamma$) is known to upregulate PD-L1 on tumor cells (Abiko et al., 2015 Br J Cancer 112:1501-1509; Dong et al., 2002 Nature Medicine 8(8): 793-800).

PD-L1 obstructs anti-tumor immunity by 1) tolerizing tumor-reactive T cells by binding to its receptor PD-1 (CD279) on activated T cells (Dong et al., *supra*; Latchman et al., 2004 Proc Natl Acad Sci USA 101, 10691-6); 2) rendering tumor cells resistant to CD8+ T cell and Fas ligand-mediated lysis by PD-1 signaling through tumor cell-expressed PD-L1 (Azuma et al., 2008 Blood 111, 3635-43); 3) tolerizing T cells by reverse signaling through T cell-expressed CD80 (B7.1) (Butte et al., 2007 Immunity 27, 111-22; Park et al., 2010 Blood 116, 1291-8); and 4) promoting the development and maintenance of induced T regulatory cells (Francisco et al., 2009 J Exp Med 206, 3015-29). PD-L1 is expressed in many human cancers, including melanoma, ovarian, lung and colon cancer (Dong et al., *supra*).

PD-L1 blocking antibodies have shown clinical activity in several cancers known to overexpress PD-L1 (incl. melanoma, NSCLC). For example, atezolizumab is a humanized IgG1 monoclonal antibody against PD-L1. It is currently in clinical trials as an immunotherapy for several indications including various types of solid tumors (see e.g. Rittmeyer et al., 2017 Lancet 389:255-265). Avelumab, a PD-L1 antibody, (Kaufman et al Lancet Oncol. 2016;17(10):1374-1385) has been approved by the FDA for the treatment of adults and pediatric patients 12 years and older with metastatic Merkel cell carcinoma, and is currently in clinical trials in several cancer indications, including bladder cancer, gastric cancer, head and neck cancer, mesothelioma, NSCLC, ovarian cancer and renal cancer. Durvalumab, a PD-L1 antibody, is approved for locally advanced or metastatic urothelial carcinoma indications, and is in clinical development in multiple solid tumors and blood cancers (see e.g. Massard et al., 2016 J Clin Oncol. 34(26):3119-25). Further anti-PD-L1 antibodies have been described in WO2004004771, WO2007005874, WO2010036959, WO2010077634, WO2013079174, WO2013164694, WO2013173223 and WO2014022758.

While significant progress has been made on eradication of cancer, there is still a need for further improvement of antibody-based cancer therapy.

**SUMMARY OF THE INVENTION**

In a first aspect, the invention provides novel anti-PD-L1 antibodies comprising an antigen-binding region capable of binding to human PD-L1. The antibodies of this second aspect of the invention may be monospecific or multispecific, and, if multispecific, said multispecific antibodies may, or may not, comprise an antigen-binding region capable of binding to human CD3$\varepsilon$.

The invention further provides bispecific antibodies comprising an antigen-binding region capable of binding to human PD-L1 and an antigen-binding region capable of binding to human CD3$\varepsilon$ (epsilon). Such a bispecific antibody has a dual effect:

Firstly, through its PD-L1 binding region, the antibody binds PD-L1 expressing tumor cells, while through its CD3-binding region, the antibody binds T cells. The antibody thus brings T cells in close proximity to tumor cells, thereby facilitating tumor cell killing by T cells. Furthermore, without being limited by any specific theory, it is hypothesized that bringing PD-L1-expressing cells and effector T cells in close proximity to each other might initiate the release of interferon-$\gamma$ which in turn could upregulate PD-L1 on tumor cells, thus facilitating recruitment of more antibodies to the tumor and further enhance its killing.

Secondly, the bispecific antibodies of the invention inhibit the binding of human PD-L1 to human PD-1 thus preventing PD-L1 from obstructing anti-tumor immunity through PD-1.

The CD3xPD-L1 bispecific antibodies are particularly useful in therapeutic settings in which specific targeting and T cell-mediated killing of cells that express PD-L1 is desired. The CD3xPD-L1 bispecific antibodies are highly efficient in mediating killing of PD-L1 expressing cells, including, in some embodiments, cells with low PD-L1 copy numbers.

The antibodies of the invention are capable of binding to PD-L1 expressing cells, such as MDA-MB-231, PC3 or HELA cells. Furthermore, the antibodies of the invention inhibit the interaction between PD-L1 and PD-1 and can mediate killing of MDA-MB-231, PC3 and/or HELA cells by purified T-cells or PBMCs.

In a further aspect, the invention relates to the use of the antibodies of the invention in medicine, in particular for the treatment of cancer.

These and other aspects of the invention are described in further detail below.

## BRIEF DESCRIPTION OF THE DRAWINGS

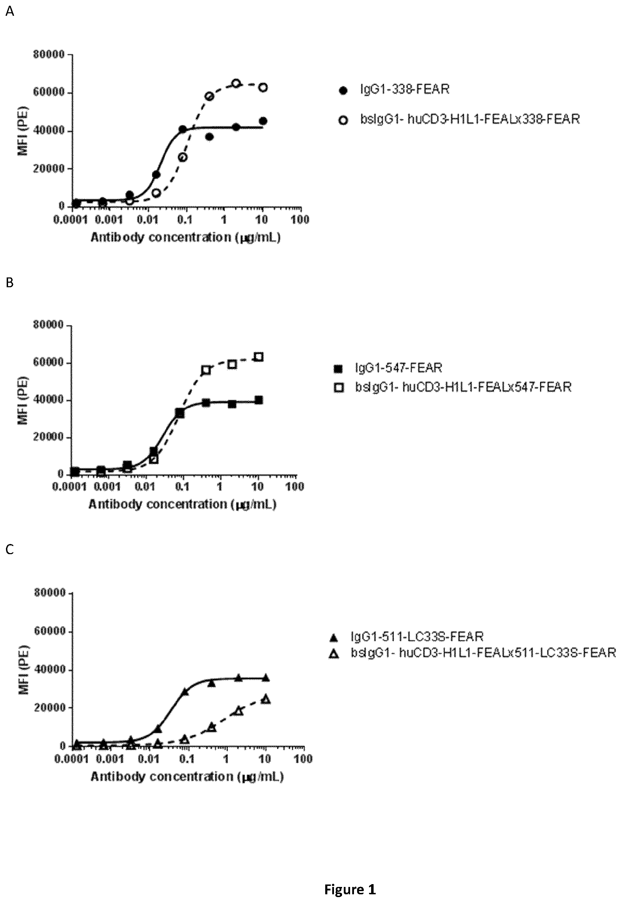

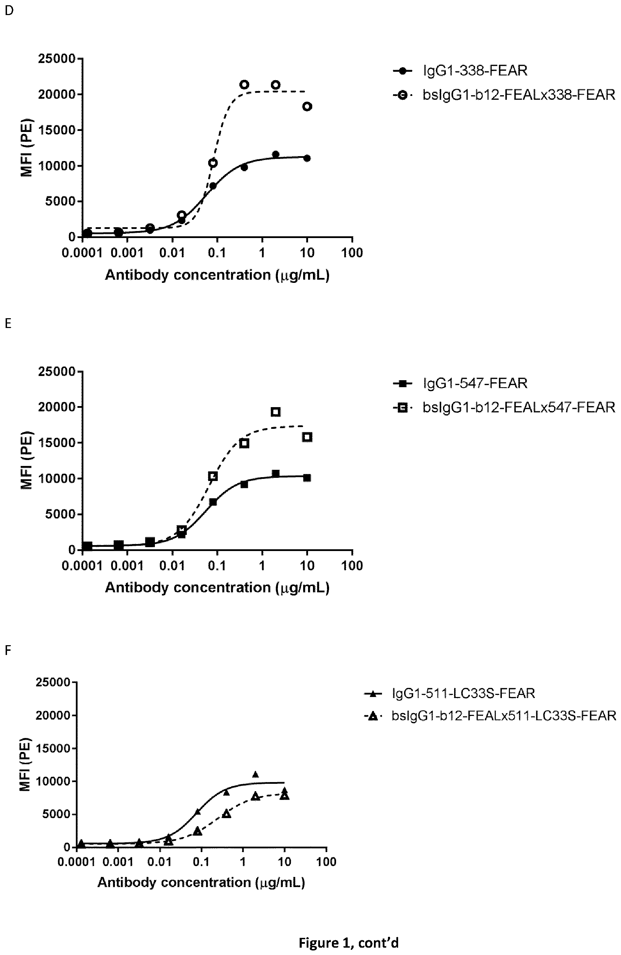

**Figure 1: Binding of bispecific CD3xPD-L1 and b12xPD-L1 antibodies and their monospecific, bivalent PD-L1 counterparts to MDA-MB-231 cells.** (A) Binding of bsIgG1-huCD3-H1L1-FEALx338-FEAR and IgG1-338-FEAR, (B) Binding of bsIgG1-huCD3-H1L1-FEALx547-FEAR and IgG1-547-FEAR, (C) Binding of bsIgG1-huCD3-H1L1-FEALx511-LC33S-FEAR and IgG1-511-LC33S-FEAR, (D)Binding of bsIgG1-b12-FEALx338-FEAR (E) Binding of bsIgG1-b12-FEALx547-FEAR, (F) Binding of bsIgG1-b12-FEALx511-LC33S-FEAR. Data shown are mean fluorescence intensity (MFI) as determined by flow cytometry, for one representative experiment.

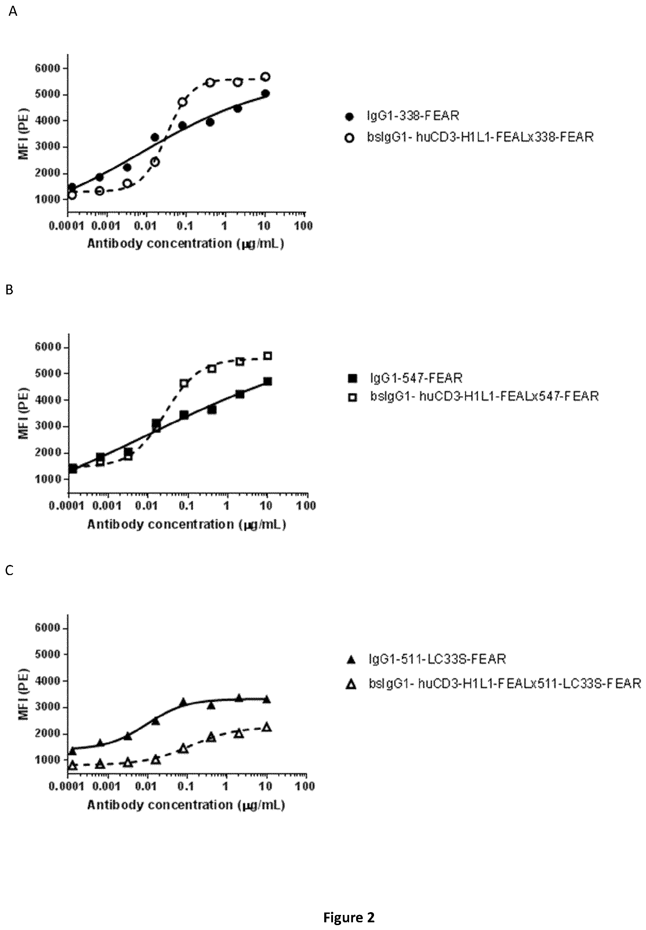

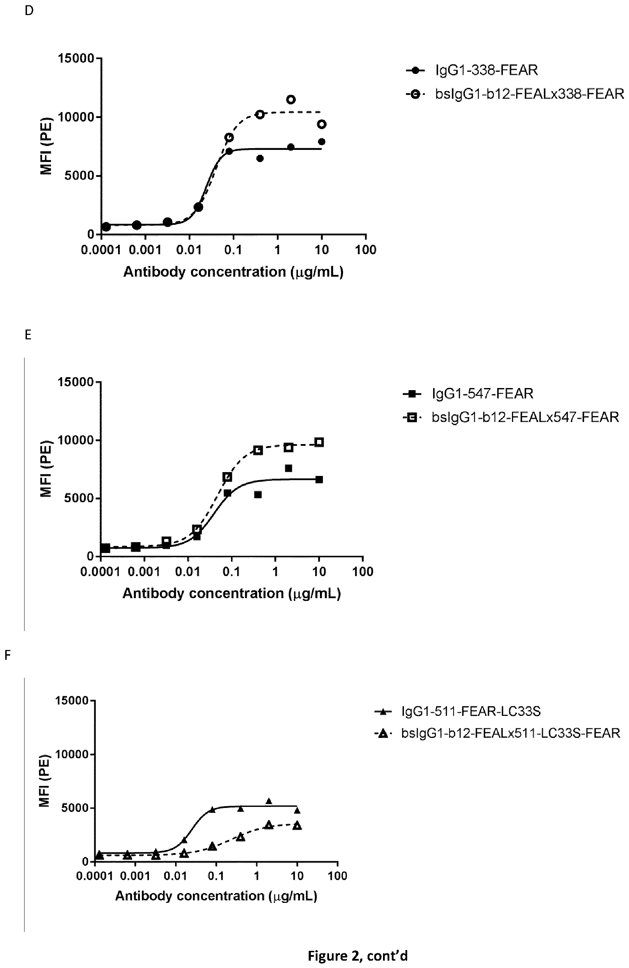

**Figure 2: Binding of bispecific CD3xPD-L1 and b12xPD-L1 antibodies and their monospecific, bivalent PD-L1 counterparts to PC3 cells.** (A) Binding of bsIgG1-huCD3-H1L1-FEALx338-FEAR and IgG1-338-FEAR, (B) Binding of bsIgG1-huCD3-H1L1-FEALx547-FEAR and IgG1-547-FEAR, (C) Binding of bsIgG1-huCD3-H1L1-FEALx511-LC33S-FEAR and IgG1-511-LC33S-FEAR, (D) Binding of bsIgG1-b12-FEALx338-FEAR (E) Binding of bsIgG1-b12-FEALx547-FEAR, (F) Binding of bsIgG1-b12-FEALx511-LC33S-FEAR. Data shown are mean fluorescence intensity (MFI) as determined by flow cytometry, for one representative experiment.

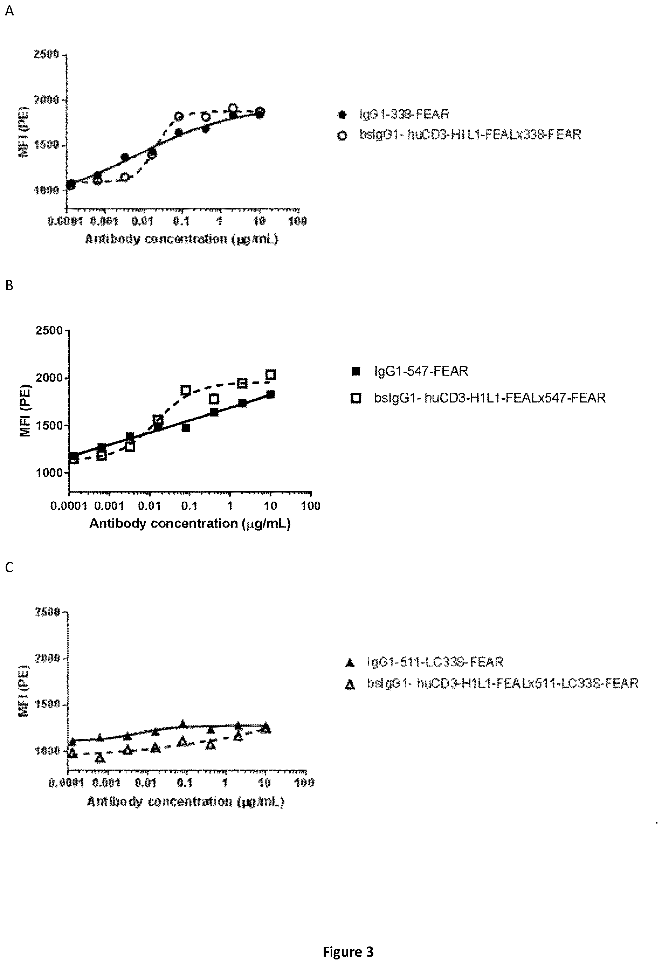

**Figure 3: Binding of bispecific CD3xPD-L1 and b12xPD-L1 antibodies and their monospecific, bivalent PD-L1 counterparts to HELA cells.** (A) Binding of bsIgG1-huCD3-H1L1-FEALx338-FEAR and IgG1-338-FEAR, (B) Binding of bsIgG1-huCD3-H1L1-FEALx547-FEAR and IgG1-547-FEAR, (C) Binding of bsIgG1-huCD3-H1L1-FEALx511-LC33S-FEAR and IgG1-511-LC33S-FEAR, (D) Binding of bsIgG1-b12-FEALx338-FEAR (E) Binding of bsIgG1-b12-FEALx547-FEAR, (F) Binding of bsIgG1-b12-FEALx511-LC33S-FEAR. Data shown are mean fluorescence intensity (MFI) as determined by flow cytometry, for one representative experiment.

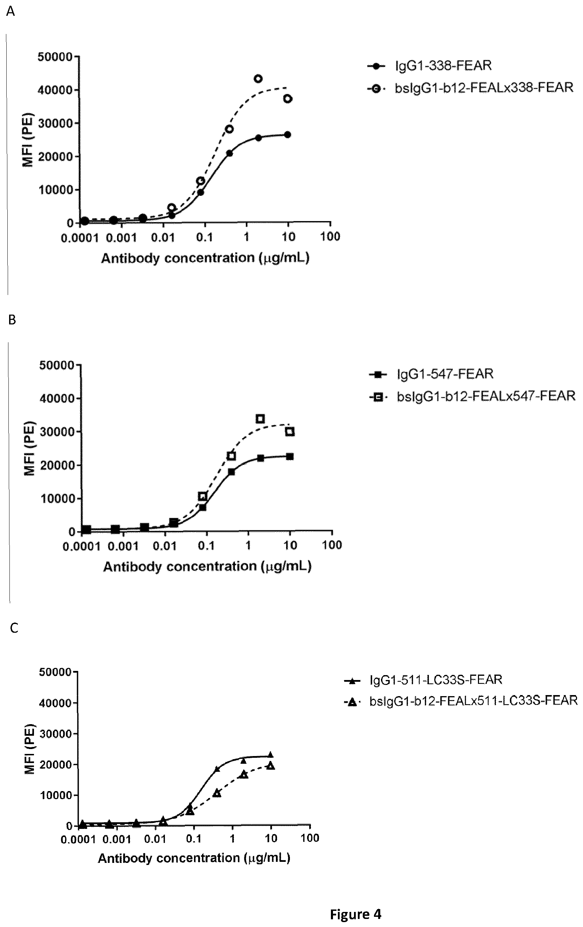

**Figure 4: Binding of bispecific b12xPD-L1 antibodies and their monospecific, bivalent PD-L1 counterparts to SK-MES-1 cells.** (A) Binding of bsIgG1-b12-FEALx338-FEAR and IgG1-338-FEAR, (B) Binding of bsIgG1-b12-FEALx547-FEAR and IgG1-547-FEAR, (C) Binding of bsIgG1-b12-FEALx511-LC33S-FEAR and IgG1-511-LC33S-FEAR. Data shown are mean fluorescence intensity (MFI) as determined by flow cytometry, for one representative experiment.

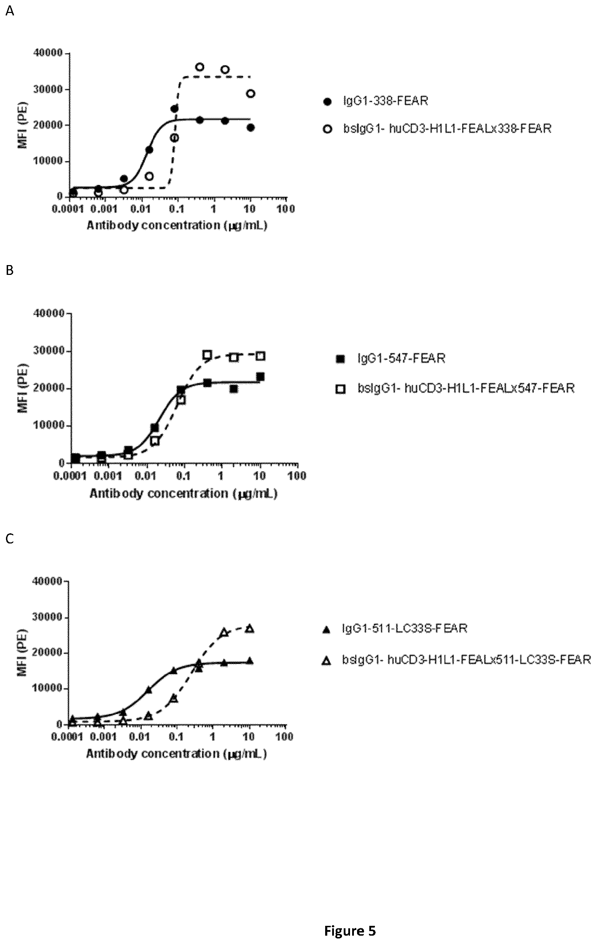

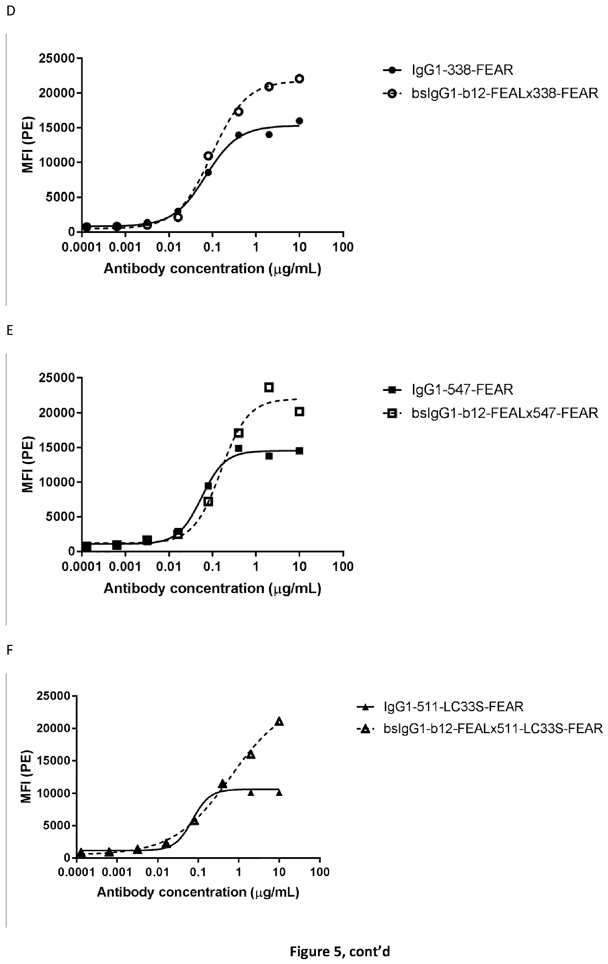

**Figure 5: Binding of bispecific CD3xPD-L1 and b12xPD-L1 antibodies and their monospecific, bivalent PD-L1 counterparts to CHO cells transfected with cynomolgus PD-L1.** (A) Binding of bsIgG1-huCD3-H1L1-FEALx338-FEAR and IgG1-338-FEAR, (B) Binding of bsIgG1-huCD3-H1L1-FEALx547-FEAR and IgG1-547-FEAR, (C) Binding of bsIgG1-huCD3-H1L1-FEALx511-LC33S-FEAR and IgG1-511-LC33S-FEAR, (D) Binding of bsIgG1-b12-FEALx338-FEAR, (E) Binding of bsIgG1-b12-FEALx547-FEAR, (F) Binding of bsIgG1-b12-FEALx511-LC33S-FEARData shown are mean fluorescence intensity (MFI) as determined by flow cytometry, for one representative experiment.

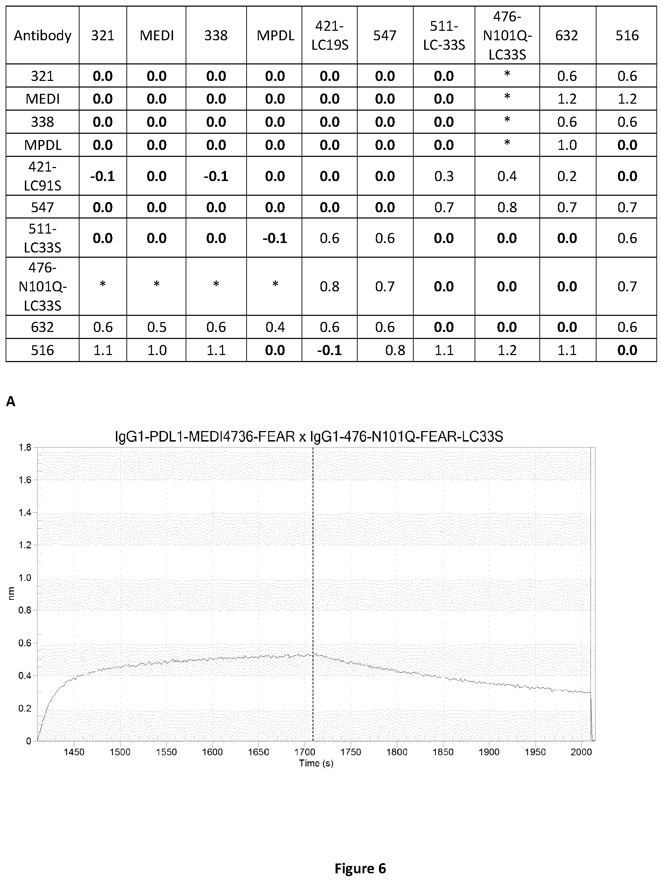

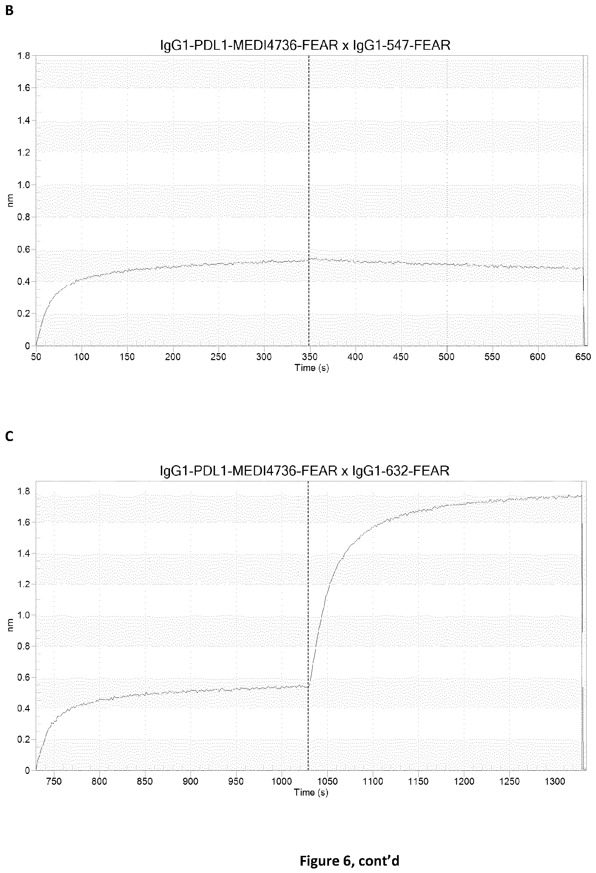

**Figure 6: Antibody cross-block.** Determination of antibody cross-block was performed using biolayer interferometry. All antibodies were produced in a FEAR IgG1 backbone. In the table, responses ≥ 0.1 nm were considered non-blocking antibody pairs (results indicated as plain numbers in the table), responses below 0.1 were considered to be blocking antibody pairs (results indicated as bold numbers in the table), while some responses neither blocking nor non-blocking were indicated to be antibodies showing displacing behavior (results indicated in asterisk (*) in the table). MEDI = MEDI4736; MPDL = MPDL3280A. Representative figures are shown for (A) displacing, (B) blocking and (C) non-blocking antibody pairs.

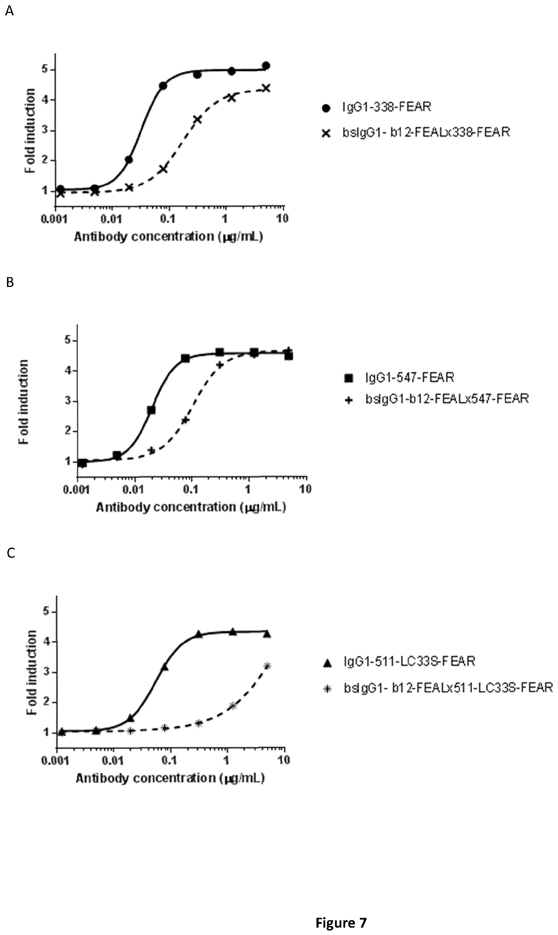

**Figure 7: Effect of bispecific b12xPD-L1 antibodies and their monospecific, bivalent PD-L1 counterparts on the PD-1/PD-L1 interaction.** The effect of (A) bsIgG1-b12-FEALx338-FEAR and IgG1-338-FEAR, (B) bsIgG1-b12-FEALx547-FEAR and IgG1-547-FEAR, (C) bsIgG1-b12-FEALx511-LC33S-FEAR and IgG1-511-LC33S-FEAR was determined in a PD-1/PD-L1 blockade bioassay. Data shown are fold induction relative to control (without antibody added), for one representative experiment.

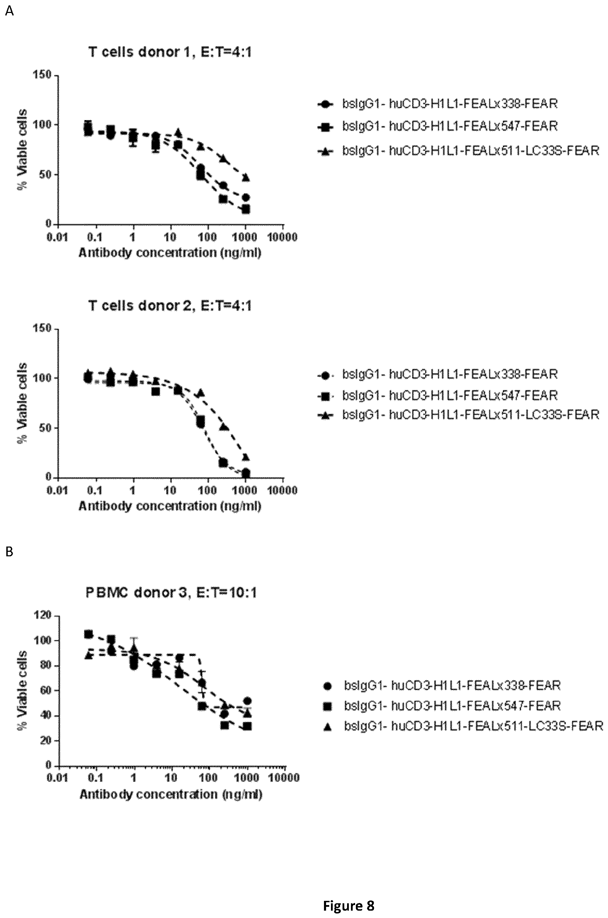

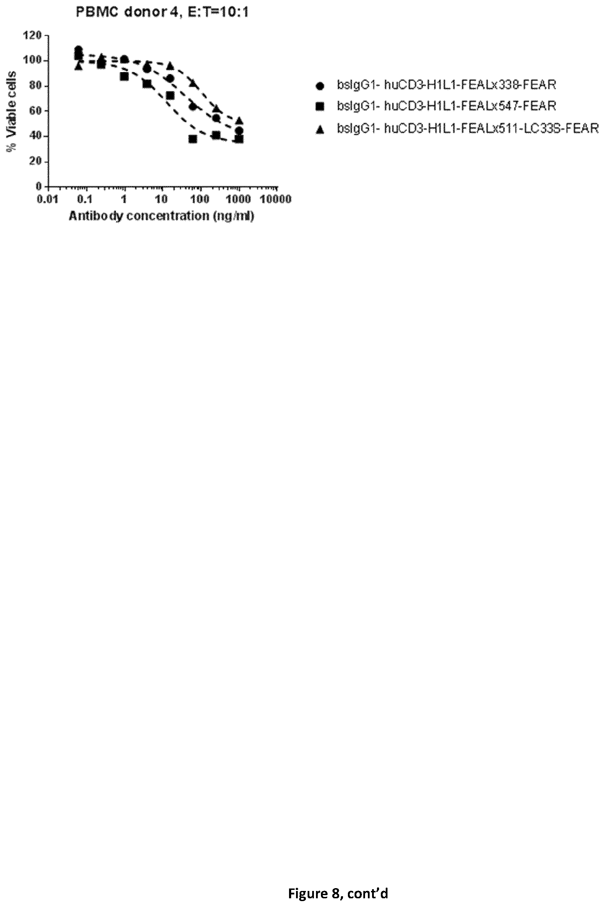

**Figure 8: Induction of cytotoxicity *in vitro* CD3xPD-L1 bispecific antibodies in MDA-MB-231 cells.** MDA-MB-231 cells were incubated bsIgG1-huCD3-H1L1-FEALx338-FEAR, bsIgG1-huCD3-H1L1-FEALx547-FEAR and bsIgG1-huCD3-H1L1-FEALx511-LC33S-FEAR. Purified T cells (A) or PBMCs (B) were used as effector cells. Data shown are % viable cells, for one representative experiment.

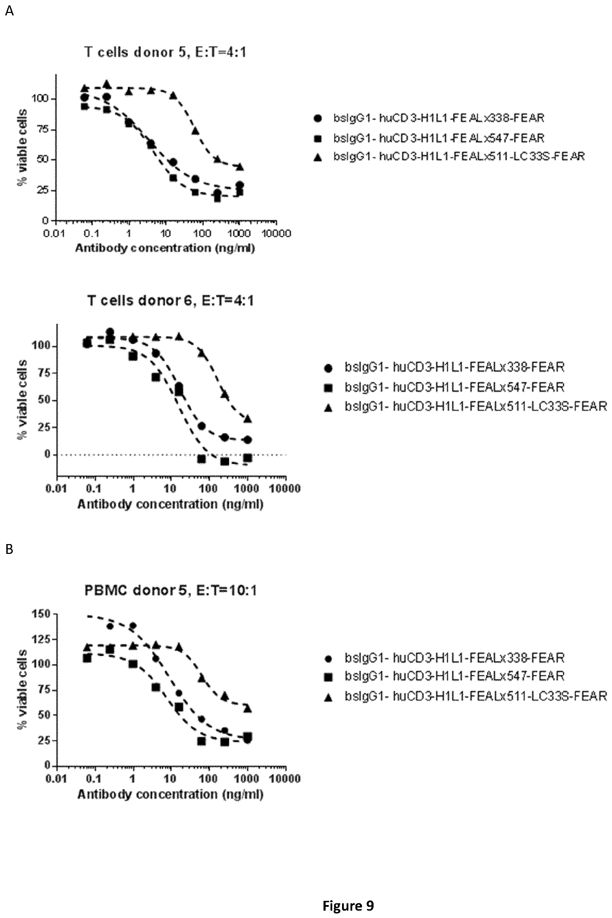

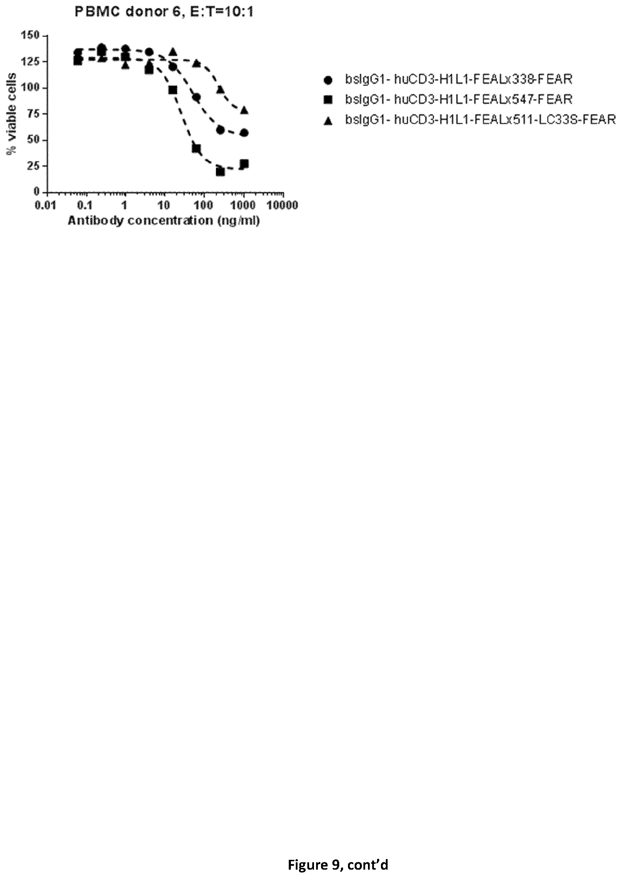

**Figure 9: Induction of cytotoxicity *in vitro* CD3xPD-L1 bispecific antibodies in PC-3 cells.** PC-3 cells were incubated bsIgG1-huCD3-H1L1-FEALx338-FEAR, bsIgG1-huCD3-H1L1-FEALx547-FEAR and bsIgG1-huCD3-H1L1-FEALx511-LC33S-FEAR. Purified T cells (A) or PBMCs (B) were used as effector cells. Data shown are % viable cells, for one representative experiment.

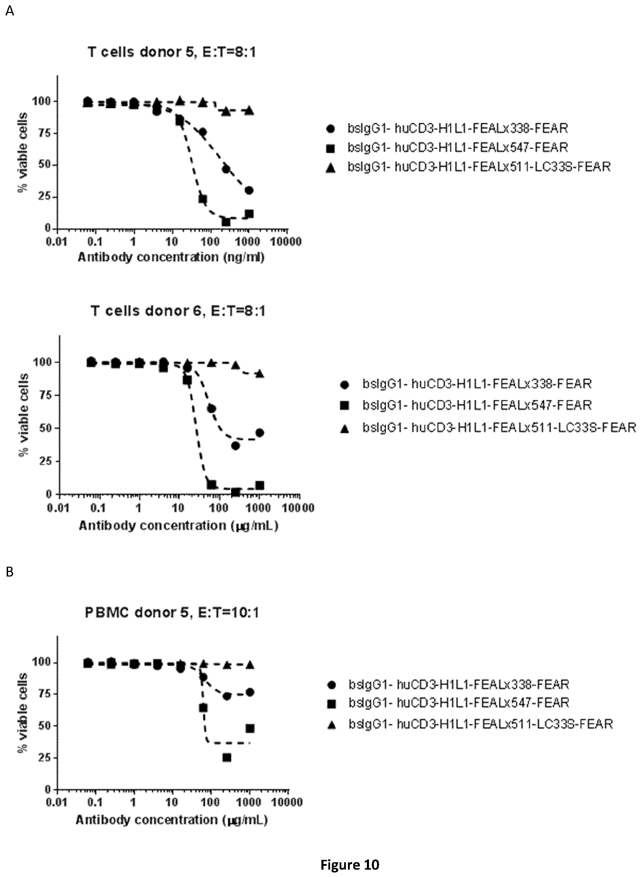

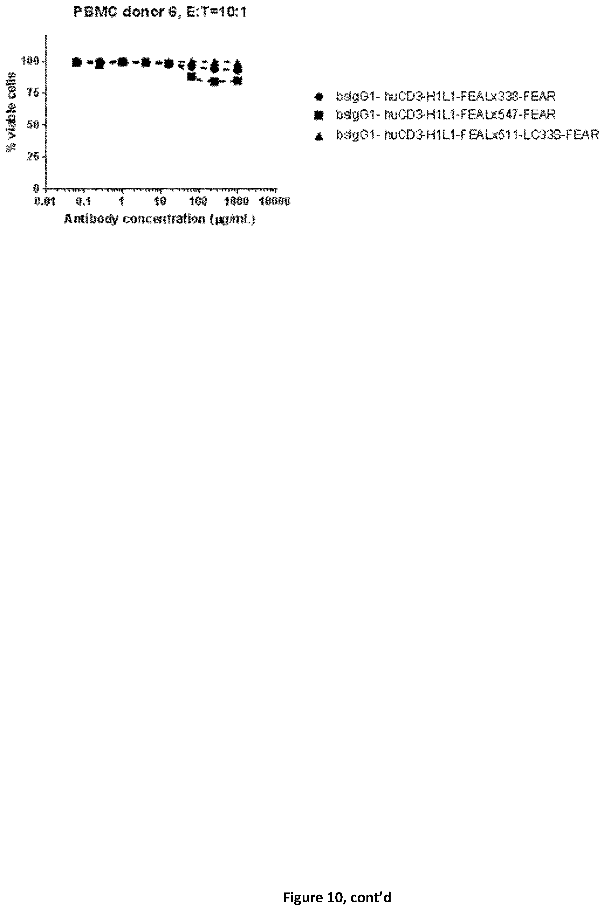

**Figure 10: Induction of cytotoxicity *in vitro* CD3xPD-L1 bispecific antibodies in HELA cells.** HELA cells were incubated bsIgG1-huCD3-H1L1-FEALx338-FEAR, bsIgG1-huCD3-H1L1-FEALx547-FEAR and bsIgG1-huCD3-H1L1-FEALx511-LC33S-FEAR. Purified T cells (A) or PBMCs (B) were used as effector cells. Data shown are % viable cells, for one representative experiment.

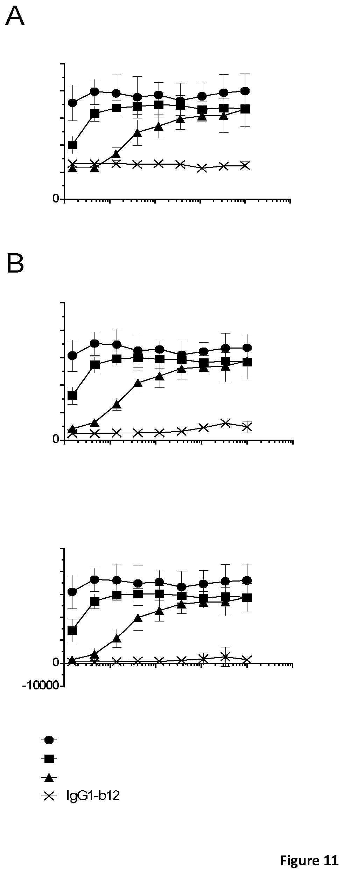

**Figure 11: T cell proliferation and activation by CD3xPD-L1 bispecific antibodies.**

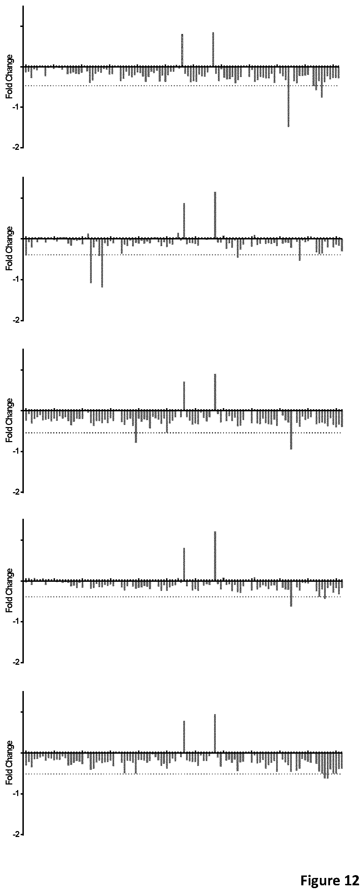

CD3xPD-L1 bispecific antibodies were tested in an *in vitro* assay to measure T cell activation and proliferation, using MDA-MB-231 cells as target cells and purified T cells as effector cells. (A) Total T cell count, (B) CD69$^{pos}$ T cell count, (C) CD25$^{pos}$ T cell count. Figure 12: Fold Change in binding of PD-L1 antibodies to PD-L1 variants with alanine mutations at positions 42 to 131. Fold change was defined as $Log_{10}$(Normalized gMFI$_{[ala\ mutant]}$/Normalized gMFI$_{[wt]}$). Residues where the Fold Change in binding was lower than mean Fold Change - 1.5 x SD (indicated by the dotted line) were considered 'loss of binding mutants'. Residues with a positive Fold Change in binding are loss of binding residues for the IgG1-625-FEAR-A488 control antibody (residues 75 and 86). Number above the x-axis refer to amino acid positions.

**Figure 12: Fold Change in binding of PD-L1 antibodies to PD-L1 variants with alanine mutations at positions 42 to 131.** Fold change was defined as $Log_{10}$(Normalized gMFI$_{[ala\ mutant]}$/Normalized gMFI$_{[wt]}$). Residues where the Fold Change in binding was lower than mean Fold Change - 1.5 x SD (indicated by the dotted line) were considered 'loss of binding mutants'. Residues with a positive Fold Change in binding are loss of binding residues for the IgG1-625-FEAR-A488 control antibody (residues 75 and 86). Number above the x-axis refer to amino acid positions.

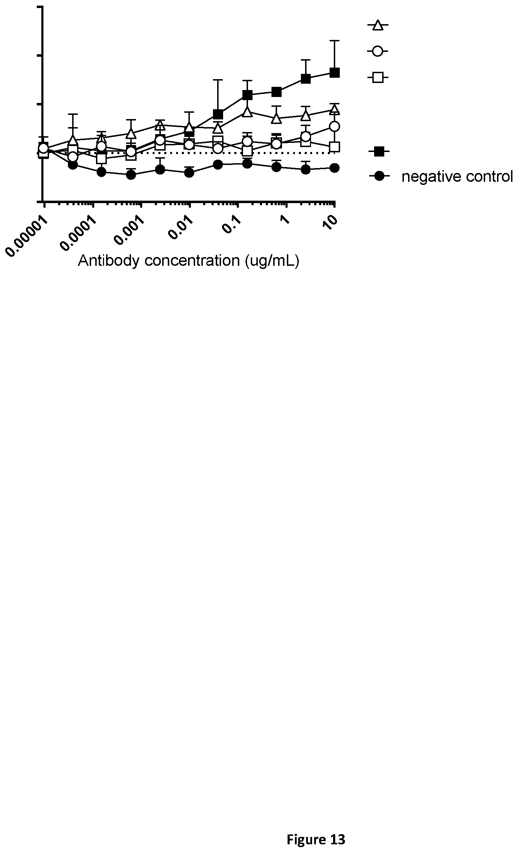

**Figure 13: Antibody-dependent cell-mediated cytotoxicity of MDA-MB-231 cells by PD-L1 antibodies as determined in a $^{51}$Cr release assay.**

ADCC of MDA-MB-231 cells was determined in an *in vitro* $^{51}$Cr-release assay with freshly isolated PBMC from a healthy human donor at an E:T ratio 100:1. For each data point, the mean and standard deviation of 3 replicate samples is presented. A representative example with PBMC of one donor is shown.

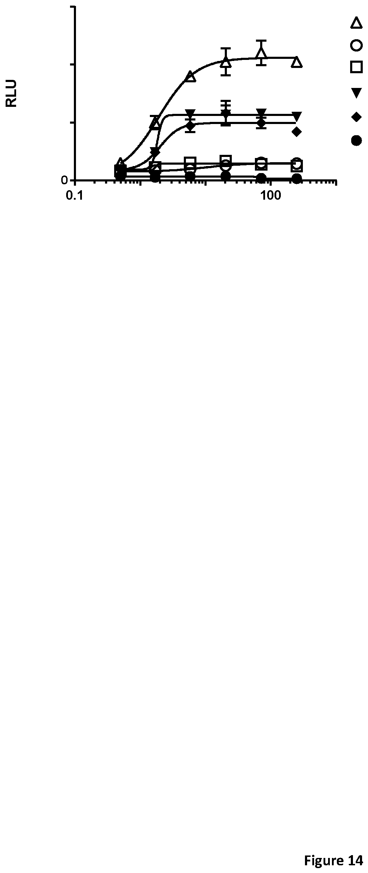

**Figure 14: Antibody-dependent cell-mediated cytotoxicity of MDA-MB-231 cells by PD-L1 antibodies as determined in a Luminescent ADCC Reporter BioAssay.**

ADCC of MDA-MB-231 cells by PD-L1 antibodies was quantified using Fc$\gamma$RIIIa-expressing Jurkat reporter cells that express luciferase upon Fc$\gamma$RIIIa binding. The production of luciferase is presented by relative luminescence units (RLU). For each data point, the mean and standard deviation of duplicates is presented.

## DETAILED DESCRIPTION OF THE INVENTION

Definitions

The term "immunoglobulin" refers to a class of structurally related glycoproteins consisting of two pairs of polypeptide chains, one pair of light (L) low molecular weight chains and one pair of heavy (H) chains, all four inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as VH or VH) and a heavy chain constant region (abbreviated herein as CH or CH). The heavy chain constant region typically is comprised of three domains, CH1, CH2, and CH3. The hinge region is the region between the CH1 and CH2 domains of the heavy chain and is highly flexible. Disulphide bonds in the hinge region are part of the interactions between two heavy chains in an IgG molecule. Each light chain typically is comprised of a light chain variable region (abbreviated herein as VL or VL) and a light chain constant region (abbreviated herein as CL or CL). The light chain constant region typically is comprised of one domain, CL. The VH and VL regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901-917 (1987)). Unless otherwise stated or contradicted by context, CDR sequences herein are identified according to IMGT rules using DomainGapAlign Version 4.9.2 (2016-09-26) (Lefranc MP., Nucleic Acids Research 1999;27:209-212 and Ehrenmann F., Kaas Q. and Lefranc M.-P. Nucleic Acids Res., 38, D301-307 (2010); see also internet http address http://www.imgt.org/). Unless otherwise stated or contradicted by context, reference to amino acid positions in the constant regions in the present invention is according to the EU-numbering (Edelman et al., Proc Natl Acad Sci USA. 1969 May;63(1):78-85; Kabat et al., Sequences of Proteins of Immunological Interest, Fifth Edition. 1991 NIH Publication No. 91-3242). For example, SEQ ID NO:93 herein sets forth amino acids positions 118-447 according to EU numbering, of the IgG1m(f) heavy chain constant region.

The term "amino acid corresponding to position..." as used herein refers to an amino acid position number in a human IgG1 heavy chain. Corresponding amino acid positions in other immunoglobulins may be found by alignment with human IgG1. Thus, an amino acid or segment in one sequence that "corresponds to" an amino acid or segment in another sequence is one that aligns with the other amino acid or segment using a standard sequence alignment program such as ALIGN, ClustalW or similar, typically at default settings and has at least 50%, at least 80%, at least 90%, or at least 95% identity to a human IgG1 heavy chain. It is considered well-known in the art how to align a sequence or segment in a sequence and thereby determine the corresponding position in a sequence to an amino acid position according to the present invention.

The term "antibody" (Ab) in the context of the present invention refers to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The variable regions of the heavy and light chains of the immunoglobulin molecule contain a binding domain that interacts with an antigen. The term "antibody-binding region", wherein used herein, refers to the region which interacts with the antigen and comprises both the VH and the VL regions. The term antibody when used herein comprises not only monospecific antibodies, but also multispecific antibodies which comprise multiple, such as two or more, e.g. three or more, different antigen-binding regions. The constant regions of the antibodies (Abs) may mediate the binding of the immunoglobulin to host tissues or factors, including various cells of the immune system (such as effector cells) and components of the complement system such as C1q, the first component in the classical pathway of complement activation. As indicated above, the term antibody herein, unless otherwise stated or clearly contradicted by context, includes fragments of an antibody that are antigen-binding fragments, *i.e.*, retain the ability to specifically bind to the antigen. It has been shown that the antigen-binding function of an antibody may be performed by fragments of a full-length antibody. Examples of antigen-binding fragments encompassed within the term "antibody" include (i) a Fab' or Fab fragment, a monovalent fragment consisting of the VL, VH, CL and CH1 domains, or a monovalent antibody as described in WO2007059782 (Genmab); (ii) F(ab')$_2$ fragments, bivalent fragments comprising two Fab fragments linked by a disulfide bridge at the hinge region; (iii) a Fd fragment consisting essentially of the VH and CH1 domains; (iv) a Fv fragment consisting essentially of the VL and VH domains of a single arm of an antibody, (v) a dAb fragment (Ward et al., Nature 341, 544-546 (1989)), which consists essentially of a VH domain and also called domain antibodies (Holt et al; Trends Biotechnol. 2003 Nov;21(11):484-90); (vi) camelid or nanobodies (Revets et al; Expert Opin Biol Ther. 2005 Jan;5(1):111-24) and (vii) an isolated complementarity determining region (CDR). Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise noted or clearly indicated by context. Although such fragments are generally included within the meaning of antibody, they collectively and each independently are unique features of the present invention, exhibiting different biological properties and utility. These and other useful antibody fragments in the context of the present invention, as well as bispecific formats of such fragments, are discussed further herein. It also should be understood that the term antibody, unless specified otherwise, also includes polyclonal antibodies, monoclonal antibodies (mAbs), antibody-like polypeptides, such as chimeric antibodies and humanized antibodies, and antibody fragments retaining the ability to specifically bind to the antigen (antigen-binding fragments) provided by any known technique, such as enzymatic cleavage, peptide synthesis, and recombinant techniques. An antibody as generated can possess any isotype. As used herein, the term "isotype" refers to the immunoglobulin class (for instance IgG1, IgG2, IgG3, IgG4, IgD, IgA, IgE, or IgM) that is encoded by heavy chain constant region genes. When a particular isotype, e.g. IgG1, is mentioned herein, the term is not limited to a specific isotype sequence, e.g. a particular IgG1 sequence, but is used to indicate that the antibody is closer in sequence to that isotype, e.g. IgG1, than to other isotypes. Thus, e.g. an IgG1 antibody of the invention may be a sequence variant of a naturally-occurring IgG1 antibody, including variations in the constant regions.

The term "monoclonal antibody" as used herein refers to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be generated by a hybridoma which includes a B cell obtained from a transgenic or transchromosomal non-human animal, such as a transgenic mouse, having a genome comprising a human heavy chain transgene and a light chain transgene, fused to an immortalized cell.

The term "bispecific antibody" or "bs" in the context of the present invention refers to an antibody having two different antigen-binding regions defined by different antibody sequences. In some embodiments, said different antigen-binding regions bind different epitopes on the same antigen. However, in preferred embodiments, said different antigen-binding regions bind different target antigens. A bispecific antibody can be of any format, including any of the bispecific antibody formats described herein below.

When used herein, the terms "half molecule", "Fab-arm" and "arm" refer to one heavy chain-light chain pair.

When a bispecific antibody is described to comprise a half-molecule antibody "derived from" a first antibody, and a half-molecule antibody "derived from" a second antibody, the term "derived from" indicates that the bispecific antibody was generated by recombining, by any known method, said half-molecules from each of said first and second antibodies into the resulting bispecific antibody. In this context, "recombining" is not intended to be limited by any particular method of recombining and thus includes all of the methods for producing bispecific antibodies described herein below, including for example recombining by half-molecule exchange, as well as recombining at nucleic acid level and/or through co-expression of two half-molecules in the same cells.

The term "monovalent antibody" means in the context of the present invention that an antibody molecule is capable of binding a single molecule of an antigen, and thus is not capable of crosslinking antigens or cells.

The term "full-length" when used in the context of an antibody indicates that the antibody is not a fragment, but contains all of the domains of the particular isotype normally found for that isotype in nature, e.g. the VH, CH1, CH2, CH3, hinge, VL and CL domains for an IgG1 antibody.

When used herein, unless contradicted by context, the term "Fc region" refers to an antibody region consisting of the two Fc sequences of the heavy chains of an immunoglobulin, wherein said Fc sequences comprise at least a hinge region, a CH2 domain, and a CH3 domain.

When used herein the term "heterodimeric interaction between the first and second CH3 regions" refers to the interaction between the first CH3 region and the second CH3 region in a first-CH3/second-CH3 heterodimeric protein.

When used herein the term "homodimeric interactions of the first and second CH3 regions" refers to the interaction between a first CH3 region and another first CH3 region in a first-CH3/first-CH3 homodimeric protein and the interaction between a second CH3 region and another second CH3 region in a second-CH3/second-CH3 homodimeric protein.

As used herein, the terms "capable of binding" or "binding" in the context of the binding of an antibody to a predetermined antigen or epitope typically is a binding with an affinity corresponding to a $K_D$ of about $10^{-6}$ M or less, such as about $10^{-7}$ M or less, such as about $10^{-8}$ M or less, such as about $10^{-9}$ M or less, about $10^{-10}$ M or less, or about $10^{-11}$ M or even less, when determined using Bio-Layer Interferometry (BLI), e.g. as described in Example 8, or, for instance, when determined using surface plasmon resonance (SPR) technology in a BIAcore 3000 instrument using the antigen as the ligand and the antibody as the analyte. The antibody binds to the predetermined antigen with an affinity corresponding to a $K_D$ that is at least ten-fold lower, such as at least 100-fold lower, for instance at least 1,000-fold lower, such as at least 10,000-fold lower, for instance at least 100,000-fold lower than its affinity for binding to a non-specific antigen (e.g., BSA, casein) other than the predetermined antigen or a closely-related antigen. The amount with which the affinity is lower is dependent on the $K_D$ of the antibody, so that when the $K_D$ of the antibody is very low (that is, the antibody is highly specific), then the degree to which the affinity for the antigen is lower than the affinity for a non-specific antigen may be at least 10,000-fold.

The term "$k_d$" (sec$^{-1}$), as used herein, refers to the dissociation rate constant of a particular antibody-antigen interaction. Said value is also referred to as the $k_{off}$ or $k_{dis}$ value.

The term "$K_D$" (M), as used herein, refers to the dissociation equilibrium constant of a particular antibody-antigen interaction. It is obtained by dividing kd by *ka*.

The term "$k_a$" (M$^{-1}$ $\times$ sec$^{-1}$), as used herein, refers to the association rate constant of a particular antibody-antigen interaction. Said value is also referred to as the $k_{on}$ value or on-rate.

In a preferred embodiment, the antibody of the invention is isolated. An "isolated antibody" as used herein, is intended to refer to an antibody which is substantially free of other antibodies having different antigenic specificities. In a preferred embodiment, an isolated bispecific antibody that specifically binds to PD-L1 and a second target, such as CD3, is substantially free of monospecific antibodies that specifically bind to PD-L1 or to the second target, e.g. CD3. In another preferred embodiment, the antibody, or a pharmaceutical composition comprising the antibody, is substantially free of naturally-arising antibodies that are not capable of binding to PD-L1. In a further preferred embodiment, the antibody of the invention possesses a structural change in its amino acid sequence, relative to the structure of a naturally-occurring anti-PD-L1 antibody, wherein said structural change causes said antibody to exhibit an altered functionality relative to the functionality exhibited by said naturally-occurring anti-PD-L1 antibody, said functionality being selected from the group consisting of: (i) PD-L1 binding affinity, (ii) ability to inhibit binding of PD-L1 to PD-1 and (iii) ability to induce Fc-mediated effector functions.

The term "PD-L1" when used herein, refers to the Programmed Death-Ligand 1 protein. PD-L1 is found in humans and other species, and thus, the term "PD-L1" is not limited to human PD-L1 unless contradicted by context.

Human, macaque and mouse PD-L1 sequences can be found through Genbank accession no. NP_054862.1, XP_005581836 and NP_068693, respectively.

The term "PD-L2" when used herein, refers to the human Programmed Death 1-Ligand 2 protein (Genbank accession no. NP_079515).

The term "PD-1" when used herein, refers to the human Programmed Death-1 protein, also known as CD279.

The term "CD3" as used herein, refers to the human Cluster of Differentiation 3 protein which is part of the T-cell co-receptor protein complex and is composed of four distinct chains. CD3 is also found in other species, and thus, the term "CD3" is not limited to human CD3 unless contradicted by context. In mammals, the complex contains a CD3γ (gamma) chain (human CD3γ chain UniProtKB/Swiss-Prot No P09693, or cynomolgus monkey CD3γ UniProtKB/Swiss-Prot No Q95LI7), a CD3δ (delta) chain (human CD3δ UniProtKB/Swiss-Prot No P04234, or cynomolgus monkey CD3δ UniProtKB/Swiss-Prot No Q95LI8), two CD3ε (epsilon) chains (human CD3ε UniProtKB/Swiss-Prot No P07766 (SEQ ID NO:95); cynomolgus CD3ε UniProtKB/Swiss-Prot No Q95LI5; or rhesus CD3ε UniProtKB/Swiss-Prot No G7NCB9), and a CD3ζ-chain (zeta) chain (human CD3ζ UniProtKB/Swiss-Prot No P20963, cynomolgus monkey CD3ζ UniProtKB/Swiss-Prot No Q09TK0). These chains associate with a molecule known as the T-cell receptor (TCR) and generate an activation signal in T lymphocytes. The TCR and CD3 molecules together comprise the TCR complex.

A "PD-L1 antibody" or "anti-PD-L1 antibody" is an antibody as described above, which binds specifically to the antigen PD-L1, in particular human PD-L1.

A "CD3 antibody" or "anti-CD3 antibody" is an antibody as described above, which binds specifically to the antigen CD3, in particular human CD3ε (epsilon).

A °CD3xPD-L1 antibody", °anti-CD3xPD-L1 antibody", "PD-L1xCD3 antibody" or "anti- PD-L1xCD3 antibody" is a bispecific antibody, which comprises two different antigen-binding regions, one of which binds specifically to the antigen PD-L1 and one of which binds specifically to CD3.

The present invention also provides antibodies comprising functional variants of the VL regions, VH regions, or one or more CDRs of the antibodies of the examples. A functional variant of a VL, VH, or CDR used in the context of an antibody still allows the antibody to retain at least a substantial proportion (at least about 50%, 60%, 70%, 80%, 90%, 95% or more) of the affinity and/or the specificity/selectivity of the "reference" or "parent" antibody and in some cases, such an antibody may be associated with greater affinity, selectivity and/or specificity than the parent antibody.

Such functional variants typically retain significant sequence identity to the parent antibody. The percent identity between two sequences is a function of the number of identical positions shared by the sequences (i.e., % homology = # of identical positions/total # of positions x 100), taking into account the number of gaps, and the length of each gap, which need to be introduced for optimal alignment of the two sequences. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4. In addition, the percent identity between two amino acid sequences may be determined using the Needleman and Wunsch, J. Mol. Biol. 48, 444-453 (1970) algorithm.

Exemplary variants include those which differ from VH and/or VL and/or CDR regions of the parent antibody sequences mainly by conservative substitutions; for instance, 10, such as 9, 8, 7, 6, 5, 4, 3, 2 or 1 of the substitutions in the variant are conservative amino acid residue replacements.

In the context of the present invention, conservative substitutions may be defined by substitutions within the classes of amino acids reflected in the following table:

**Amino acid residue classes for conservative substitutions**

| Acidic Residues | Asp (D) and Glu (E) |
|---|---|
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

In the context of the present invention, the following notations are, unless otherwise indicated, used to describe a mutation; i) substitution of an amino acid in a given position is written as e.g. K409R which means a substitution of a Lysine in position 409 with an Arginine; and ii) for specific variants the specific three or one letter codes are used, including the codes Xaa and X to indicate any amino acid residue. Thus, the substitution of Lysine with Arginine in position 409 is designated as: K409R, and the substitution of Lysine with any amino acid residue in position 409 is designated as K409X. In case of deletion of Lysine in position 409 it is indicated by K409*.

In the context of the present invention, "competition" refers to a significant reduction in the propensity for a particular molecule to bind a particular binding partner in the presence of another molecule that binds the binding partner. "Competition" can refer to both "blocking" or "displacement", i.e. a competing molecule can either be a blocking or a displacing molecule. "Displacing" refers to a condition wherein a second antibody can displace an antigen from an antigen-antibody complex (formed earlier) resulting in exchange of the antigen (Abdiche et al., 2017 Plos One 12(1): e0169535). Competition for binding to PD-L1 by two or more anti-PD-L1 antibodies may be determined by any suitable technique. In one embodiment, competition is determined as described in Example 9 herein.

Similarly, in the context of the present invention, "inhibition of PD-L1 binding to PD-1" refers to any detectably significant reduction in the binding of PD-L1 to PD-1 in the presence of an antibody capable of binding PD-L1. Typically, inhibition means an at least about 10% reduction, such as an at least about 15%, e.g. an at least about 20%, such as an at least 40% reduction in binding between PD-L1 and PD-1, caused by the presence of an anti-PD-L1 antibody. Inhibition of PD-L1 binding to PD-1 may be determined by any suitable technique. In one embodiment, inhibition is determined as described in Example 10 herein.

The term "epitope" means a protein determinant capable of specific binding to an antibody. Epitopes usually consist of surface groupings of molecules such as amino acids or sugar side chains and usually have specific three-dimensional structural characteristics, as well as specific charge characteristics. Conformational and nonconformational epitopes are distinguished in that the binding to the former but not the latter is lost in the presence of denaturing solvents. The epitope may comprise amino acid residues directly involved in the binding and other amino acid residues, which are not directly involved in the binding, such as amino acid residues which are effectively blocked or covered by the specifically antigen binding peptide (in other words, the amino acid residue is within the footprint of the specifically antigen binding peptide).

The term "chimeric antibody" as used herein, refers to an antibody wherein the variable region is derived from a non-human species (e.g. derived from rodents) and the constant region is derived from a different species, such as human. Chimeric monoclonal antibodies for therapeutic applications are developed to reduce antibody immunogenicity. The terms "variable region" or "variable domain" as used in the context of chimeric antibodies, refer to a region which comprises the CDRs and framework regions of both the heavy and light chains of the immunoglobulin. Chimeric antibodies may be generated by using standard DNA techniques as described in Sambrook et al., 1989, Molecular Cloning: A laboratory Manual, New York: Cold Spring Harbor Laboratory Press, Ch. 15. The chimeric antibody may be a genetically or an enzymatically engineered recombinant antibody. It is within the knowledge of the skilled person to generate a chimeric antibody, and thus, generation of the chimeric antibody according to the present invention may be performed by other methods than described herein.

The term "humanized antibody" as used herein, refers to a genetically engineered non-human antibody, which contains human antibody constant domains and non-human variable domains modified to contain a high level of sequence homology to human variable domains. This can be achieved by grafting of the six non-human antibody complementarity-determining regions (CDRs), which together form the antigen binding site, onto a homologous human acceptor framework region (FR) (see WO92/22653 and EP0629240). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and fully human constant regions. Optionally, additional amino acid modifications, which are not necessarily back-mutations, may be applied to obtain a humanized antibody with preferred characteristics, such as affinity and biochemical properties.

The term "human antibody" as used herein, refers to antibodies having variable and constant regions derived from human germline immunoglobulin sequences. Human antibodies may include amino acid residues not encoded by human germline immunoglobulin sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences. Human monoclonal antibodies of the invention can be produced by a variety of techniques, including conventional monoclonal antibody methodology, e.g., the standard somatic cell hybridization technique of Kohler and Milstein, Nature 256: 495 (1975). Although somatic cell hybridization procedures are preferred, in principle, other techniques for producing monoclonal antibody can be employed, e.g., viral or oncogenic transformation of B-lymphocytes or phage display techniques using libraries of human antibody genes. A suitable animal system for preparing hybridomas that secrete human monoclonal antibodies is the murine system. Hybridoma production in the mouse is a very well established procedure. Immunization protocols and techniques for isolation of immunized splenocytes for fusion are known in the art. Fusion partners (e.g., murine myeloma cells) and fusion procedures are also known. Human monoclonal antibodies can thus e.g. be generated using transgenic or transchromosomal mice or rats carrying parts of the human immune system rather than the mouse or rat system. Accordingly, in one embodiment, a human antibody is obtained from a transgenic animal, such as a mouse or a rat, carrying human germline immunoglobulin sequences instead of animal immunoglobulin sequences. In such embodiments, the antibody originates from human germline immunoglobulin sequences introduced in the animal, but the final antibody sequence is the result of said human germline immunoglobulin sequences being further modified by somatic hypermutations and affinity maturation by the endogeneous animal antibody machinery, see e.g. Mendez et al. 1997 Nat Genet. 15(2):146-56. The term "reducing conditions" or "reducing environment" refers to a condition or an environment in which a substrate, here a cysteine residue in the hinge region of an antibody, is more likely to become reduced than oxidized.

The term "recombinant host cell" (or simply "host cell"), as used herein, is intended to refer to a cell into which an expression vector has been introduced, e.g. an expression vector encoding an antibody of the invention. Recombinant host cells include, for example, transfectomas, such as CHO, CHO-S, HEK, HEK293, HEK-293F, Expi293F, PER.C6 or NS0 cells, and lymphocytic cells.

The term "treatment" refers to the administration of an effective amount of a therapeutically active antibody of the present invention with the purpose of easing, ameliorating, arresting or eradicating (curing) symptoms or disease states.

The term "effective amount" or "therapeutically effective amount" refers to an amount effective, at dosages and for periods of time necessary, to achieve a desired therapeutic result. A therapeutically effective amount of an antibody may vary according to factors such as the disease state, age, sex, and weight of the individual, and the ability of the antibody to elicit a desired response in the individual. A therapeutically effective amount is also one in which any toxic or detrimental effects of the antibody or antibody portion are outweighed by the therapeutically beneficial effects.

The term "anti-idiotypic antibody" refers to an antibody which recognizes unique determinants generally associated with the antigen-binding site of an antibody.

Further aspects and embodiments of the invention

As described above, in a first aspect, the invention relates to a bispecific antibody comprising an antigen-binding region capable of binding to human PD-L1 and an antigen-binding region capable of binding to human CD3ε (epsilon), wherein the antibody inhibits the binding of human PD-L1 to human PD-1. Such bispecific antibodies thus comprise two different antigen-binding regions, one which has a binding specificity for PD-L1 and one which has a binding specificity for CD3.

In one embodiment, said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO:11 and SEQ ID NO:21.

In a further embodiment, said antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively, or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO: 17, respectively, or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively.

In another embodiment, said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1, SEQ ID NO:8 and SEQ ID NO:18.

In another embodiment, wherein said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5, SEQ ID NO:15 and SEQ ID NO:22.

In a further embodiment, said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO: 1 and a VL sequence which has at least 90%, at least 95%, at least 97%, at least 99% or 100% amino acid sequence identity to the VL sequence set forth in: SEQ ID NO:5, or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15, or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22.

Thus, for example, said antigen-binding region capable of binding to human PD-L1 comprises:

a VH sequence which has at least 95% amino acid sequence identity to the VH sequence set forth in: SEQ ID NO:1 and a VL sequence which has at least 95% amino acid sequence identity to the VL sequence set forth in: SEQ ID NO:5, or

a VH sequence which has at least 97% amino acid sequence identity to the VH sequence set forth in: SEQ ID NO:1 and a VL sequence which has at least 97% amino acid sequence identity to the VL sequence set forth in: SEQ ID NO:5, or

a VH sequence which has at least 99% amino acid sequence identity to the VH sequence set forth in: SEQ ID NO:1 and a VL sequence which has at least 99% amino acid sequence identity to the VL sequence set forth in: SEQ ID NO:5, or

a VH sequence which has at least 95% amino acid sequence identity to the VH sequence set forth in: SEQ ID NO:8 and a VL sequence which has at least 95% amino acid sequence identity to the VL sequence set forth in: SEQ ID NO:15, or

a VH sequence which has at least 97% amino acid sequence identity to the VH sequence set forth in: SEQ ID NO:8 and a VL sequence which has at least 97% amino acid sequence identity to the VL sequence set forth in: SEQ ID NO:15, or

a VH sequence which has at least 99% amino acid sequence identity to the VH sequence set forth in: SEQ ID NO:8 and a VL sequence which has at least 99% amino acid sequence identity to the VL sequence set forth in: SEQ ID NO:15, or

a VH sequence which has at least 95% amino acid sequence identity to the VH sequence set forth in: SEQ ID NO:18 and a VL sequence which has at least 95% amino acid sequence identity to the VL sequence set forth in: SEQ ID NO:22, or

a VH sequence which has at least 97% amino acid sequence identity to the VH sequence set forth in: SEQ ID NO:18 and a VL sequence which has at least 97% amino acid sequence identity to the VL sequence set forth in: SEQ ID NO:22, or

a VH sequence which has at least 99% amino acid sequence identity to the VH sequence set forth in: SEQ ID NO:18 and a VL sequence which has at least 99% amino acid sequence identity to the VL sequence set forth in: SEQ ID NO:22.

In a further embodiment, said VH and VL sequences each comprise three CDR sequences, CDR1, CDR2 and CDR3, respectively, and four framework sequences, FR1, FR2, FR3 and FR4, respectively, and the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated. In the context of this embodiment, the % identity refers to the percentage identity obtained when the framework sequences are taken together as one consecutive sequence without the intermediate CDR sequences.

In a preferred embodiment of the antibody of the invention, said antigen-binding region capable of binding to human PD-L1 comprises:

(i) a VH sequence as set forth in SEQ ID NO: 1 and a VL sequence as set forth in SEQ ID NO:5, or

(ii) a VH sequence as set forth in SEQ ID NO:8 and a VL sequence as set forth in SEQ ID NO:15, or

(iii) a VH sequence as set forth in SEQ ID NO:18 and a VL sequence as set forth in SEQ ID NO:22.

Different antibodies capable of binding to the same antigen, such as PD-L1, may bind different regions of said antigen. In some cases, binding of one PD-L1 antibody to PD-L1 may still permit binding of a different PD-L1 antibody to PD-L1. In other cases, however, binding of one PD-L1 antibody to PD-L1 may compete with (block or displace) binding of a different PD-L1 antibody to PD-L1. Thus, competition experiments provide information on where on the target antigen an antibody binds, which may impact the functional effects of antibody binding.

In one embodiment, the bispecific antibody of the invention:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or

(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

In a further embodiment hereof, said antibody competes for binding to human PD-L1 with an antibody comprising a VH sequence as set forth in SEQ ID NO: 1 and a VL sequence as set forth in SEQ ID NO:5.

Antibodies which compete for target antigen binding may bind different epitopes on the antigen, wherein the epitopes are so close to each other that a first antibody binding to one epitope prevents binding of a second antibody to the other epitope. In other situations, however, two different antibodies may bind the same epitope on the antigen.

Thus, in one embodiment, the antibody of the invention:

(i) is capable of binding to the same epitope of human PD-L1 as an antibody comprising a VH sequence as set forth in SEQ ID NO: 1 and a VL sequence as set forth in SEQ ID NO:5, or

(ii) is capable of binding to the same epitope of human PD-L1 as an antibody comprising a VH sequence as set forth in SEQ ID NO:8 and a VL sequence as set forth in SEQ ID NO: 15, or

(iii) is capable of binding to the same epitope of human PD-L1 as an antibody comprising a VH sequence as set forth in SEQ ID NO:18 and a VL sequence as set forth in SEQ ID NO:22.

In a further embodiment, the binding of the bispecific antibody to human PD-L1 is not displaced by an antibody comprising a VH sequence as set forth in SEQ ID NO:53 and a VL sequence as set forth in SEQ ID NO:57.

In a further embodiment, the binding of the bispecific antibody to human PD-L1 is blocked by an antibody comprising a VH sequence as set forth in SEQ ID NO:18 and a VL sequence as set forth in SEQ ID NO:22. "Blocked" herein indicates that an antibody comprising a VH sequence as set forth in SEQ ID NO:18 and a VL sequence as set forth in SEQ ID NO:22 competes with the bispecific antibody, but does not displace it.

As described above, the above-mentioned bispecific antibodies of the first aspect of the invention comprise an antigen-binding region capable of binding to human CD3ε.

In one embodiment, the antigen-binding region capable of binding to human CD3ε comprises heavy chain variable (VH) region CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

The six CDR sequences as defined above are derived from a mouse antibody denoted SP34. Humanized versions of this antibody have been generated, and the humanized antibodies are denoted huCD3 herein and are further disclosed in WO2015001085 (Genmab).

In a preferred embodiment of the bispecific antibody of the invention, said bispecific antibody comprises:

(i) an antigen-binding region capable of binding to human PD-L1 comprising a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively,

or

(ii) an antigen-binding region capable of binding to human PD-L1 comprising a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively,

or

(iii) an antigen-binding region capable of binding to human PD-L1 comprising a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

In one embodiment, the bispecific antibody comprises an antigen-binding region capable of binding to human CD3ε which comprises a heavy chain variable region (VH), wherein said VH sequence has at least 90%, at least 95%, at least 97%, at least 99% or 100% amino acid sequence identity to the amino acid sequence as set forth in SEQ ID NO:25.

In another embodiment, the bispecific antibody comprises an antigen-binding region capable of binding to human CD3ε which comprises a light chain variable region (VL), wherein said VL sequence has at least 90%, at least 95%, at least 97%, at least 99% or 100% amino acid sequence identity to the amino acid sequence as set forth in SEQ ID NO:29.

In a preferred embodiment, the antigen-binding region capable of binding to human CD3ε comprises a VH sequence as set forth in SEQ ID NO:25 and a VL sequence as set forth in SEQ ID NO:29.

In one aspect, the bispecific antibodies according to the invention may be modified to reduce the affinity of the antibodies. This may be advantageous in some settings and lead to increased efficacy. In particular, low affinity of binding to human CD3ε may have an impact on the motility of T cells in circulation and at the tumor site, thus leading to better engagement of T cells with tumor cells, cf. Mølhøj et al., Molecular Immunology 44 (2007).

Accordingly, in a different embodiment of the bispecific antibody of the invention comprising an antigen-binding region capable of binding to human PD-L1 and an antigen-binding region capable of binding to human CD3ε, said bispecific antibody:

(i) has a lower affinity for human CD3ε binding as compared to an antibody having an antigen-binding region capable comprising a VH sequence as set forth in SEQ ID NO:25 and a VL sequence as set forth in SEQ ID NO:29, preferably wherein said affinity is at least 2-fold lower, e.g. at least 5-fold lower, such as at least 10-fold lower, e.g. at least 25-fold lower, such as at least 50-fold lower, and
(ii) is capable of mediating concentration-dependent cytotoxicity of MDA-MB-231 cells, PC-3 cells and/or HELA cells when using PBMCs or purified T cells as effector cells.

Similarly, in one embodiment, the antibody of the invention comprises an antigen-binding region capable of binding to human CD3ε, wherein said antigen-binding region capable of binding to human CD3ε:

(i) has a lower affinity for human CD3ε binding as compared to an antibody having an antigen-binding region capable comprising a VH sequence as set forth in SEQ ID NO:25 and a VL sequence as set forth in SEQ ID NO:29, preferably wherein said affinity is at least 2-fold lower, e.g. at least 5-fold lower, such as at least 10-fold lower, e.g. at least 25-fold lower, such as at least 50-fold lower, and
(ii) is capable of mediating concentration-dependent cytotoxicity of MDA-MB-231 cells, PC-3 cells and/or HELA cells when using PBMCs or purified T cells as effector cells.

Affinity for human CD3ε may e.g. be measured using octet binding affinity determination as described in Example 7 of WO2017009442.

The ability of an antibody to mediate cytotoxicity by PBMCs or purified T cells may e.g. be determined as described in Example 11 herein, i.e. MDA-MB-231, PC-3 cells or HELA cells are seeded and cultured in wells. Tumor cells, PBMCs and serial dilutions of antibody are added, and, following incubation, tumor cells are stained for viability.

Herein, huCD3-H1L1 refers to an anti-CD3 antibody having VH and VL sequences as set forth in SEQ ID NOs: 25 and 29. IgG1-huCD3-FEAL refers to a variant thereof comprising the substitutions L234F, L235E, D265A and F405L (see also elsewhere herein).

Examples of variants of IgG1-huCD3-FEAL having reduced affinity for human CD3ε have been described in WO2017009442 (Genmab). Example 7 (Table 6) of WO2017009442 discloses affinities of IgG1-huCD3-FEAL and seven variants thereof, measured using octet binding affinity determination:

| Antibody | <KD> (nM) | SDEV | SEM | CV |
|---|---|---|---|---|
| IgG1-huCD3-FEAL | 15 | 6 | 3 | 37 |
| IgG1-huCD3-Y114V-FEAL | 29 | 8 | 4 | 26 |
| IgG1-huCD3-T31P- FEAL | 42 | 9 | 4 | 21 |
| IgG1-huCD3-Y114M-FEAL | 42 | 14 | 8 | 33 |
| IgG1-huCD3-Y114R-FEAL | 46 | 10 | 6 | 22 |
| IgG1-huCD3-S110A-FEAL | 72 | 15 | 6 | 21 |
| IgG1-huCD3-T31M-FEAL | 99 | 23 | 13 | 23 |
| IgG1-huCD3-H101G-FEAL | 683 | 169 | 97 | 25 |

In one embodiment of the bispecific antibody of the invention, the antigen-binding region capable of binding to human CD3ε comprises:

(i) a heavy chain variable region (VH) comprising CDR1, CDR2, and CDR3 having the sequences as set forth in SEQ ID NOs: 99, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(ii) a heavy chain variable region (VH) comprising CDR1, CDR2, and CDR3 having the sequences as set forth in SEQ ID NOs: 100, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iii) a heavy chain variable region (VH) comprising CDR1, CDR2, and CDR3 having the sequences as set forth in SEQ ID NOs: 26, 27, and 101, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iv) a heavy chain variable region (VH) comprising CDR1, CDR2, and CDR3 having the sequences as set forth in SEQ ID NOs: 26, 27, and 102, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(v) a heavy chain variable region (VH) comprising CDR1, CDR2, and CDR3 having the sequences as set forth in SEQ ID NOs: 26, 27, and 103, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vi) a heavy chain variable region (VH) comprising CDR1, CDR2, and CDR3 having the sequences as set forth in SEQ ID NOs: 26, 27, and 104, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vii) a heavy chain variable region (VH) comprising CDR1, CDR2, and CDR3 having the sequences as set forth in SEQ ID NOs: 26, 27, and 105, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

In another embodiment, the antigen-binding region capable of binding to human CD3ε comprises:

(i) a VH sequence as set forth in SEQ ID NO:39 and a VL sequence as set forth in SEQ ID NO:29, or
(ii) a VH sequence as set forth in SEQ ID NO:40 and a VL sequence as set forth in SEQ ID NO:29, or
(iii) a VH sequence as set forth in SEQ ID NO:41 and a VL sequence as set forth in SEQ ID NO:29, or
(iv) a VH sequence as set forth in SEQ ID NO:42 and a VL sequence as set forth in SEQ ID NO:29, or
(v) a VH sequence as set forth in SEQ ID NO:43 and a VL sequence as set forth in SEQ ID NO:29, or
(vi) a VH sequence as set forth in SEQ ID NO:44 and a VL sequence as set forth in SEQ ID NO:29, or
(vii) a VH sequence as set forth in SEQ ID NO:45 and a VL sequence as set forth in SEQ ID NO:29.

In further preferred embodiments of the bispecific antibody of the invention, each of the antigen-binding regions comprises a heavy chain variable region (VH) and a light chain variable region (VL), and wherein said variable regions each comprise three CDR sequences, CDR1, CDR2 and CDR3, respectively, and four framework sequences, FR1, FR2, FR3 and FR4, respectively.

In further preferred embodiments of the bispecific antibody of the invention, the antibody comprises two heavy chain constant regions (CH), and two chain constant regions (CL).

In a preferred embodiment, the bispecific antibody comprises a first and second heavy chain, wherein each of said first and second heavy chains comprises at least a hinge region, a CH2 and a CH3 region, wherein in said first heavy chain at least one of the amino acids in a position corresponding to a position selected from the group consisting of T366, L368, K370, D399, F405, Y407, and K409 (according to EU numbering) has been substituted, and in said second heavy chain at least one of the amino acids in a position corresponding to a position selected from the group consisting of T366, L368, K370, D399, F405, Y407, and K409 (according to EU numbering) has been substituted, and wherein said first and said second heavy chains are not substituted in the same positions.

Most preferably, (i) the amino acid in the position corresponding to F405 (according to EU numbering) is L in said first heavy chain, and the amino acid in the position corresponding to K409 (according to EU numbering) is R in said second heavy chain, or (ii) the amino acid in the position corresponding to K409 (according to EU numbering) is R in said first heavy chain, and the amino acid in the position corresponding to F405 (according to EU numbering) is L in said second heavy chain.

In a further particularly preferred embodiment, the antibody is a CD3xPD-L1 bispecific antibody comprising a first and second heavy chain, wherein the positions corresponding to positions L234 and L235 in a human IgG1 heavy chain according to EU numbering of both the first heavy chain and the second heavy chain are F and E, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

In a further particularly preferred embodiment, the antibody is a CD3xPD-L1 bispecific antibody comprising a first and second heavy chain, wherein the positions corresponding to positions L234, L235, and D265 in a human IgG1 heavy chain according to EU numbering of both the first heavy chain and the second heavy chain are F, E, and A, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

Novel classes of PD-L1 antibodies

In a further aspect, the invention provides novel anti-PD-L1 antibodies that comprise an antigen-binding region capable of binding to human PD-L1. The antibodies of this aspect of the invention may be monospecific or multispecific, and, if multispecific, said multispecific antibodies may, or may not, comprise an antigen-binding region capable of binding to human CD3ε.

In one embodiment, the invention provides an antibody comprising an antigen-binding region capable of binding to human PD-L1, wherein said antigen-binding region capable of binding to human PD-L1 has the features as defined herein above.

In one embodiment, the invention provides an antibody comprising an antigen-binding region capable of binding to human PD-L1, wherein binding of the antibody to a mutant PD-L1 in which any one or more of the amino acid residues at positions corresponding to positions 113 (R113), 123 (Y123) and 125 (R125) in SEQ ID NO: 94 have been substituted with alanines, is reduced as compared to binding to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13.

The said antibody may bind to an epitope on PD-L1 (SEQ ID NO: 94), said epitope comprising the amino acid residues at position 113 (R113), the amino acid residue at position 123 (Y123) and/or the amino acid residue at position 125 (R125) of SEQ ID NO: 94.

In one embodiment, the invention provides an antibody comprising an antigen-binding region capable of binding to human PD-L1, wherein binding of the antibody to a mutant PD-L1 in which any one or more of the amino acid residues at positions corresponding to positions 19 (F19), 42 (F42), 45 (E45), 46 (K46), 94 (L94) and 116 (I116) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13.

The antibody may bind to an epitope on PD-L1 (SEQ ID NO: 94), said epitope comprising one or more amino acid residues selected from the group consisting of: the amino acid residues at position 45 (E45), the amino acid residue at position 46 (K46; and/or the amino acid residue at position 94 (L94) of SEQ ID NO: 94.

In one embodiment, the invention provides an antibody comprising an antigen-binding region capable of binding to human PD-L1, wherein binding of the antibody to a mutant PD-L1 in which any one or more of the amino acid residues at positions corresponding to positions 58 (E58) and 113 (R113) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13.

The antibody may bind to an epitope on PD-L1 (SEQ ID NO: 94), said epitope comprising the amino acid residue at position 58 (E58) and/or the amino acid residue at position 113 (R113) of SEQ ID NO: 94.

In further embodiments, the antibody according to the invention is capable of inducing dose-dependent lysis of epithelial cells of an adenocarcinoma, such MDA-MB-231 through antibody-dependent cell-mediated cytotoxicity (ADCC).

In further embodiments, the antibody according to the invention is capable of reducing the number of cells in a culture of said epithelial cells by at least 5%, such as at least 6%, 7%, 8%, 9% or at least 10% as a result of cell lysis.

ADCC may be determined *in vitro* in a $^{51}$Cr release assay, such as the assay disclosed in example 14. In particular, ADCC is determined *in vitro,* by incubating said epithelial cells with a composition comprising the antibody and effector cells, such as peripheral blood mononuclear cells (PBMCs), for 4 hours at 37°C, 5% $CO_2$, the amount of antibody in said composition being within the range of 0.1-1 $\mu$g/mL and the ratio of effector cells to epithelial cells being 100:1.

Lysis of epithelial cells may be determined *in vitro* in a luciferase reporter assay as a surrogate for ADCC, such as the luminescent ADCC reporter bioassay disclosed in example 14.

ADCC may in particular be determined *in vitro,* by

i) contacting a culture of said epithelial cells with a composition comprising the antibody and Jurkat human T-cells stably expressing FcγRIIIa (CD16) and firefly luciferase (effector cells), at an effector cell:epithelial cell ratio of 1:1.
ii) adjusting the culture of the epithelial cells and effector cells to room temperature for 15 minutes,
iii) incubating the culture of the epithelial cells and effector cells with a luciferase substrate, and
iv) determining luciferase production in said cell culture;

the amount of antibody in said composition being within the range of 0.5-250 ng/mL and the ratio of effector cells to epithelial cells being 1: 1.

ADCC of said epithelial cells may be determined in a luciferase reporter assay, such as a reporter assay defined in claim 23 or 24, then the ADCC observed after incubation of a culture of the epithelial cells with a test composition comprising said antibody is at least 1.5 times the ADCC observed after incubation of a culture of the epithelial cells with a composition comprising reference antibody; ADCC being determined as relative luminescence units (RLU), the concentration of antibody in said test composition and in said composition comprising a reference antibody being the same and within the range of 20 to 250 ng/ml, and the reference antibody being selected from:

a) an antibody comprising the VH sequence set forth in SEQ ID NO: 74 and the VL sequence set forth in SEQ ID NO: 78; and

b) an antibody comprising the VH sequence set forth in SEQ ID NO: 81 and the VL sequence set forth in SEQ ID NO: 85.

In one embodiment, there is provided an antibody comprising an antigen-binding region capable of binding to human PD-L1, wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO:11 and SEQ ID NO:21.

In a further embodiment, there is provided an antibody comprising an antigen-binding region capable of binding to human PD-L1, wherein said antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively, or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO: 17, respectively, or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24.

In a further embodiment, there is provided an antibody comprising an antigen-binding region capable of binding to human PD-L1, wherein said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1, SEQ ID NO:8 and SEQ ID NO:18.

In a further embodiment, there is provided an antibody comprising an antigen-binding region capable of binding to human PD-L1, wherein said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:52, SEQ ID NO:15 and SEQ ID NO:22.

In a further embodiment, there is provided an antibody comprising an antigen-binding region capable of binding to human PD-L1, wherein said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5, or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15, or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22.

In a further embodiment, there is provided an antibody comprising an antigen-binding region capable of binding to human PD-L1, wherein said antigen-binding region capable of binding to human PD-L1 comprises VH and VL sequences each comprising three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

In a further embodiment, there is provided an antibody comprising an antigen-binding region capable of binding to human PD-L1, wherein said antigen-binding region capable of binding to human PD-L1:

(i) a VH sequence as set forth in SEQ ID NO: 1 and a VL sequence as set forth in SEQ ID NO:5, or

(ii) a VH sequence as set forth in SEQ ID NO:8 and a VL sequence as set forth in SEQ ID NO:15, or

(iii) a VH sequence as set forth in SEQ ID NO:18 and a VL sequence as set forth in SEQ ID NO:22.

In a further embodiment, there is provided an antibody comprising an antigen-binding region capable of binding to human PD-L1, wherein said antibody:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or

(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

Preferably, said antibody competes for binding to human PD-L1 with an antibody comprising a VH sequence as set forth in SEQ ID NO: 1 and a VL sequence as set forth in SEQ ID NO:5.

In a further embodiment , there is provided an antibody comprising an antigen-binding region capable of binding to human PD-L1, wherein the binding of said antibody to human PD-L1 is not displaced by an antibody comprising a VH sequence as set forth in SEQ ID NO:53 and a VL sequence as set forth in SEQ ID NO:57.

In a further embodiment, there is provided an antibody comprising an antigen-binding region capable of binding to human PD-L1, wherein the binding of said antibody to human PD-L1 is not blocked by binding of an antibody comprising a VH sequence as set forth in SEQ ID NO: 106 and a VL sequence as set forth in SEQ ID NO: 110.

In a further embodiment , there is provided an antibody comprising an antigen-binding region capable of binding to human PD-L1, wherein the binding of said antibody to human PD-L1 is not displaced by an antibody comprising a VH sequence as set forth in SEQ ID NO:53 and a VL sequence as set forth in SEQ ID NO:57 and wherein said antibody inhibits the binding of human PD-L1 to human PD-1.

In a further embodiment , there is provided an antibody comprising an antigen-binding region capable of binding to human PD-L1, wherein the binding of said antibody to human PD-L1 is not displaced by an antibody comprising a VH sequence as set forth in SEQ ID NO:53 and a VL sequence as set forth in SEQ ID NO:57 and wherein said antibody competes for binding to human PD-L1 with an antibody comprising a VH sequence as set forth in SEQ ID NO:18 and a VL sequence as set forth in SEQ ID NO:22.

In a further embodiment , there is provided an antibody comprising an antigen-binding region capable of binding to human PD-L1, wherein the binding of said antibody to human PD-L1 is not displaced by an antibody comprising a VH sequence as set forth in SEQ ID NO:53 and a VL sequence as set forth in SEQ ID NO:57 and wherein the binding of said antibody to human PD-L1 is blocked by an antibody comprising a VH sequence as set forth in SEQ ID NO:18 and a VL sequence as set forth in SEQ ID NO:22. "Blocked" herein indicates that an antibody comprising a VH sequence as set forth in SEQ ID NO: 18 and a VL sequence as set forth in SEQ ID NO:22 competes with the bispecific antibody, but does not displace it.

In a further embodiment, there is provided an antibody comprising an antigen-binding region capable of binding to human PD-L1, wherein said antibody:

(i) is capable of binding to the same epitope of human PD-L1 as an antibody comprising a VH sequence as set forth in SEQ ID NO: 1 and a VL sequence as set forth in SEQ ID NO:5, or

(ii) is capable of binding to the same epitope of human PD-L1 as an antibody comprising a VH sequence as set forth in SEQ ID NO:8 and a VL sequence as set forth in SEQ ID NO: 15, or

(iii) is capable of binding to the same epitope of human PD-L1 as an antibody comprising a VH sequence as set forth in SEQ ID NO:18 and a VL sequence as set forth in SEQ ID NO:22.

In a further aspect, the invention relates to an antibody comprising an antigen-binding region capable of binding to human PD-L1, wherein said antibody comprises:

(i) a heavy chain variable region (VH) comprising CDR1, CDR2, and CDR3 sequences as set forth in SEQ ID NOs: 33, 34 and 35, respectively, and a light chain variable region (VL) comprising CDR1, CDR2, and CDR3 having the sequences as set forth in SEQ ID NO:37, the sequence KAS, and the sequence as set forth in SEQ ID NO:38, respectively, or

(ii) a heavy chain variable region (VH) comprising CDR1, CDR2, and CDR3 sequences as set forth in SEQ ID NOs: 47, 48 and 49, respectively, and a light chain variable region (VL) comprising CDR1, CDR2, and CDR3 having the sequences as set forth in SEQ ID NO:51, the sequence DVI, and the sequence as set forth in SEQ ID NO:52, respectively, or

(iii) a heavy chain variable region (VH) comprising CDR1, CDR2, and CDR3 sequences as set forth in SEQ ID NOs: 54, 55 and 56, respectively, and a light chain variable region (VL) comprising CDR1, CDR2, and CDR3 having the sequences as set forth in SEQ ID NO:58, the sequence RDS, and the sequence as set forth in SEQ ID NO:59, respectively, or

(iv) a heavy chain variable region (VH) comprising CDR1, CDR2, and CDR3 sequences as set forth in SEQ ID NOs: 61, 62 and 63, respectively, and a light chain variable region (VL) comprising CDR1, CDR2, and CDR3 having the sequences as set forth in SEQ ID NO:65, the sequence DDS, and the sequence as set forth in SEQ ID NO:66, respectively, or

(v) a heavy chain variable region (VH) comprising CDR1, CDR2, and CDR3 sequences as set forth in SEQ ID NOs: 107, 108 and 109, respectively, and a light chain variable region (VL) comprising CDR1, CDR2, and CDR3 having the sequences as set forth in SEQ ID NO:111, the sequence EDS, and the sequence as set forth in SEQ ID NO:113, respectively.

(vi) a heavy chain variable region (VH) comprising CDR1, CDR2, and CDR3 sequences as set forth in SEQ ID NOs: 68, 69 and 70, respectively, and a light chain variable region (VL) comprising CDR1, CDR2, and CDR3 having the sequences as set forth in SEQ ID NO:72, the sequence EDS, and the sequence as set forth in SEQ ID NO:73, respectively.

In a further embodiment, the antibody comprises:

(i) a VH sequence as set forth in SEQ ID NO:32 and a VL sequence as set forth in SEQ ID NO:36, or
(ii) a VH sequence as set forth in SEQ ID NO:46 and a VL sequence as set forth in SEQ ID NO:50, or
(iii) a VH sequence as set forth in SEQ ID NO:53 and a VL sequence as set forth in SEQ ID NO:57, or
(iv) a VH sequence as set forth in SEQ ID NO:60 and a VL sequence as set forth in SEQ ID NO:64, or
(v) a VH sequence as set forth in SEQ ID NO:106 and a VL sequence as set forth in SEQ ID NO:110, or
(v) a VH sequence as set forth in SEQ ID NO:67 and a VL sequence as set forth in SEQ ID NO:71.

In one embodiment, the antibody comprising an antigen-binding region capable of binding to human PD-L1 is monovalent.

In another embodiment, the antibody comprising an antigen-binding region capable of binding to human PD-L1 is a monospecific antibody comprising two or more identical antigen-binding regions.

In a further embodiment, the antibody comprising an antigen-binding region capable of binding to human PD-L1 is a bivalent antibody having two antigen-binding regions capable of binding to human PD-L1 and wherein said two antigen-binding regions have identical variable region sequences.

In a different embodiment, the antibody comprising an antigen-binding region capable of binding to human PD-L1 is a bivalent bispecific antibody, which, in addition to said (first) antigen-binding region capable of binding to human PD-L1, comprises a (second) antigen-binding region capable of binding to a second antigen, wherein said second antigen is human CD3ε.

In a different embodiment, the antibody comprising an antigen-binding region capable of binding to human PD-L1 is a bivalent bispecific antibody, which, in addition to said (first) antigen-binding region capable of binding to human PD-L1, comprises a (second) antigen-binding region capable of binding to a second antigen or to a different epitope of human PD-L1, wherein said second antigen is not human CD3ε.

In a further aspect, the invention relates to a multispecific antibody comprising a first antigen-binding region capable of binding to human PD-L1 and a second antigen-binding region capable of binding to a second antigen or to a different epitope of human PD-L1, wherein said second antigen optionally is not human CD3ε, and wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO: 11, SEQ ID NO:21, SEQ ID NO:35, SEQ ID NO:49, SEQ ID NO:56, SEQ ID NO:63 and SEQ ID NO:70. "Different epitope" herein means that the epitope to which the second antigen-binding region binds is different from the epitope to which the first antigen-binding region binds.

In one embodiment of said multispecific antibody, said antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively, or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively, or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively,

(iv) a heavy chain variable region (VH) comprising CDR1, CDR2, and CDR3 sequences as set forth in SEQ ID NOs: 33, 34 and 35, respectively, and a light chain variable region (VL) comprising CDR1, CDR2, and CDR3 having the sequences as set forth in SEQ ID NO:37, the sequence KAS, and the sequence as set forth in SEQ ID NO:38, respectively, or

(v) a heavy chain variable region (VH) comprising CDR1, CDR2, and CDR3 sequences as set forth in SEQ ID NOs: 47, 48 and 49, respectively, and a light chain variable region (VL) comprising CDR1, CDR2, and CDR3 having the sequences as set forth in SEQ ID NO:51, the sequence DVI, and the sequence as set forth in SEQ ID NO:52, respectively, or

(vi) a heavy chain variable region (VH) comprising CDR1, CDR2, and CDR3 sequences as set forth in SEQ ID NOs: 54, 55 and 56, respectively, and a light chain variable region (VL) comprising CDR1, CDR2, and CDR3 having the sequences as set forth in SEQ ID NO:58, the sequence RDS, and the sequence as set forth in SEQ ID NO:59, respectively, or

(vii) a heavy chain variable region (VH) comprising CDR1, CDR2, and CDR3 sequences as set forth in SEQ ID NOs: 61, 62 and 63, respectively, and a light chain variable region (VL) comprising CDR1, CDR2, and CDR3 having the sequences as set forth in SEQ ID NO:65, the sequence DDS, and the sequence as set forth in SEQ ID NO:66, respectively, or

(viii) a heavy chain variable region (VH) comprising CDR1, CDR2, and CDR3 sequences as set forth in SEQ ID NOs: 68, 69 and 70, respectively, and a light chain variable region (VL) comprising CDR1, CDR2, and CDR3 having the sequences as set forth in SEQ ID NO:72, the sequence EDS, and the sequence as set forth in SEQ ID NO:73, respectively.

In another embodiment of said multispecific antibody, said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1, SEQ ID NO:8 and SEQ ID NO:18.

In another embodiment of said multispecific antibody, said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5, SEQ ID NO:15 and SEQ ID NO:22.

In another embodiment of said multispecific antibody, said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5, or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15, or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22.

In another embodiment of said multispecific antibody, said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

In another embodiment of said multispecific antibody, said antigen-binding region capable of binding to human PD-L1 comprises:

(i) a VH sequence as set forth in SEQ ID NO: 1 and a VL sequence as set forth in SEQ ID NO:5, or
(ii) a VH sequence as set forth in SEQ ID NO:8 and a VL sequence as set forth in SEQ ID NO:15, or
(iii) a VH sequence as set forth in SEQ ID NO:18 and a VL sequence as set forth in SEQ ID NO:22, or
(iv) a VH sequence as set forth in SEQ ID NO:32 and a VL sequence as set forth in SEQ ID NO:36, or
(v) a VH sequence as set forth in SEQ ID NO:46 and a VL sequence as set forth in SEQ ID NO:50, or
(vi) a VH sequence as set forth in SEQ ID NO:53 and a VL sequence as set forth in SEQ ID NO:57, or
(v) a VH sequence as set forth in SEQ ID NO:106 and a VL sequence as set forth in SEQ ID NO: 110, or
(vii) a VH sequence as set forth in SEQ ID NO:60 and a VL sequence as set forth in SEQ ID NO:64, or
(viii) a VH sequence as set forth in SEQ ID NO:67 and a VL sequence as set forth in SEQ ID NO:71.

In a further aspect, the invention relates to a multispecific antibody comprising a first antigen-binding region capable of binding to human PD-L1 and a second antigen-binding region capable of binding to a second antigen or to a different epitope of human PD-L1, wherein said second antigen optionally is not human CD3ε, wherein said antibody:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or

(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

In one embodiment of said multispecific antibody, said antibody competes for binding to human PD-L1 with an antibody comprising a VH sequence as set forth in SEQ ID NO: 1 and a VL sequence as set forth in SEQ ID NO:5.

In a further aspect, the invention relates to a multispecific antibody comprising a first antigen-binding region capable of binding to human PD-L1 and a second antigen-binding region capable of binding to a second antigen or to a different epitope of human PD-L1, wherein the binding of said antibody to human PD-L1 is not displaced by an antibody comprising a VH sequence as set forth in SEQ ID NO:53 and a VL sequence as set forth in SEQ ID NO:57.

In one embodiment hereof, the antibody inhibits the binding of human PD-L1 to human PD-1.

In a further embodiment, the antibody competes for binding to human PD-L1 with an antibody comprising a VH sequence as set forth in SEQ ID NO:18 and a VL sequence as set forth in SEQ ID NO:22.

In a further embodiment, the binding of said antibody to human PD-L1 is blocked by an antibody comprising a VH sequence as set forth in SEQ ID NO:18 and a VL sequence as set forth in SEQ ID NO:22. "Blocked" herein indicates that an antibody comprising a VH sequence as set forth in SEQ ID NO:18 and a VL sequence as set forth in SEQ ID NO:22 competes with the bispecific antibody, but does not displace it.

In a further aspect, the invention relates to a multispecific antibody comprising a first antigen-binding region capable of binding to human PD-L1 and a second antigen-binding region capable of binding to a second antigen or to a different epitope of human PD-L1, wherein said second antigen optionally is not human CD3ε, and wherein said first antigen-binding region:

(i) is capable of binding to the same epitope of human PD-L1 as an antibody comprising a VH sequence as set forth in SEQ ID NO:1 and a VL sequence as set forth in SEQ ID NO:5, or

(ii) is capable of binding to the same epitope of human PD-L1 as an antibody comprising a VH sequence as set forth in SEQ ID NO:8 and a VL sequence as set forth in SEQ ID NO:15, or

(iii) is capable of binding to the same epitope of human PD-L1 as an antibody comprising a VH sequence as set forth in SEQ ID NO:18 and a VL sequence as set forth in SEQ ID NO:22.

In a further aspect, the invention relates to a bivalent bispecific antibody comprising a first antigen-binding region capable of binding to human PD-L1 and a second antigen-binding region capable of binding to a second antigen or to a different epitope of human PD-L1, wherein said second antigen optionally is not human CD3ε, and wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO:11 and SEQ ID NO:21.

In one embodiment of said bivalent bispecific antibody, said antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively, or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO: 17, respectively, or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively.

In another embodiment of said bivalent bispecific antibody, said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO: 1, SEQ ID NO:8 and SEQ ID NO: 18.

In another embodiment of said bivalent bispecific antibody, said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5, SEQ ID NO:15 and SEQ ID NO:22.

In another embodiment of said bivalent bispecific antibody, said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5, or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15, or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22.

In another embodiment of said bivalent bispecific antibody, said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

In another embodiment of said bivalent bispecific antibody, said antigen-binding region capable of binding to human PD-L1 comprises:

(i) a VH sequence as set forth in SEQ ID NO: 1 and a VL sequence as set forth in SEQ ID NO:5, or

(ii) a VH sequence as set forth in SEQ ID NO:8 and a VL sequence as set forth in SEQ ID NO:15, or

(iii) a VH sequence as set forth in SEQ ID NO:18 and a VL sequence as set forth in SEQ ID NO:22.

In a further aspect, the invention relates to a bivalent bispecific antibody comprising a first antigen-binding region capable of binding to human PD-L1 and a second antigen-binding region capable of binding to a second antigen or to a different epitope of human PD-L1, wherein said second antigen optionally is not human CD3ε, wherein said antibody:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or

(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

In one embodiment of said bivalent bispecific antibody, said antibody competes for binding to human PD-L1 with an antibody comprising a VH sequence as set forth in SEQ ID NO: 1 and a VL sequence as set forth in SEQ ID NO:5.

In a further aspect, the invention relates to a bivalent bispecific antibody comprising a first antigen-binding region capable of binding to human PD-L1 and a second antigen-binding region capable of binding to a second antigen or to a different epitope of human PD-L1, wherein the binding of said antibody to human PD-L1 is not displaced by an antibody comprising a VH sequence as set forth in SEQ ID NO:53 and a VL sequence as set forth in SEQ ID NO:57.

In one embodiment hereof, the antibody inhibits the binding of human PD-L1 to human PD-1.

In a further embodiment, the antibody competes for binding to human PD-L1 with an antibody comprising a VH sequence as set forth in SEQ ID NO:18 and a VL sequence as set forth in SEQ ID NO:22.

In a further embodiment, the binding of said antibody to human PD-L1 is blocked by an antibody comprising a VH sequence as set forth in SEQ ID NO:18 and a VL sequence as set forth in SEQ ID NO:22. "Blocked" herein indicates that an antibody comprising a VH sequence as set forth in SEQ ID NO:18 and a VL sequence as set forth in SEQ ID NO:22 competes with the bispecific antibody, but does not displace it.

In a further aspect, the invention relates to a bivalent bispecific antibody comprising a first antigen-binding region capable of binding to human PD-L1 and a second antigen-binding region capable of binding to a second antigen or to a different epitope of human PD-L1, wherein said second antigen optionally is not human CD3ε, and wherein said first antigen-binding region:

(i) is capable of binding to the same epitope of human PD-L1 as an antibody comprising a VH sequence as set forth in SEQ ID NO:1 and a VL sequence as set forth in SEQ ID NO:5, or

(ii) is capable of binding to the same epitope of human PD-L1 as an antibody comprising a VH sequence as set forth in SEQ ID NO:8 and a VL sequence as set forth in SEQ ID NO: 15, or

(iii) is capable of binding to the same epitope of human PD-L1 as an antibody comprising a VH sequence as set forth in SEQ ID NO:18 and a VL sequence as set forth in SEQ ID NO:22.

Further embodiments of the antibodies of the invention

In one embodiment, the antibody according to the invention binds human PD-L1 with a KD of about $10^{-8}$ M or less, such as about $10^{-9}$ M or less, e.g. about $10^{-10}$ M or less, when determined as described in Example 8 herein.

In a further embodiment, the antibody of the invention mediates concentration-dependent cytotoxicity of MDA-MB-231 cells, PC-3 cells and/or HELA cells when using purified T cells as effector cells, when assayed as described in Example 11 herein.

In a preferred embodiment, the antibody of the invention does not bind to human PD-L2.

Antibody formats

As described above, various formats of antibodies have been described in the art. The antibody of the invention can in principle be of any isotype. The choice of isotype typically will be guided by the desired Fc-mediated effector functions, such as ADCC induction, or the requirement for an antibody devoid of Fc-mediated effector function ("inert" antibody). Exemplary isotypes are IgG1, IgG2, IgG3, and IgG4. Either of the human light chain constant regions, kappa or lambda, may be used. The effector function of the antibodies of the present invention may be changed by isotype switching to, e.g., an IgG1, IgG2, IgG3, IgG4, IgD, IgA, IgE, or IgM antibody for various therapeutic uses. In one embodiment, both heavy chains of an antibody of the present invention are of the IgG1 isotype, for instance an IgG1,κ. Optionally, the heavy chain may be modified in the hinge and/or CH3 region as described elsewhere herein.

Preferably, each of the antigen-binding regions comprises a heavy chain variable region (VH) and a light chain variable region (VL), and wherein said variable regions each comprise three CDR sequences, CDR1, CDR2 and CDR3, respectively, and four framework sequences, FR1, FR2, FR3 and FR4, respectively. Furthermore, preferably, the antibody comprises two heavy chain constant regions (CH), and two light chain constant regions (CL).

In one embodiment, the antibody is a full-length antibody, such as a full-length IgG1 antibody. In another embodiment, the antibody is a full-length IgG4 antibody, preferably with a stabilized hinge region. Modifications that stabilize the IgG4 hinge region, such as the S228P mutation in the core hinge, have been described in the art, see e.g. Labrijn et al., 2009 Nat Biotechnol. 27(8):767-71.

In other embodiments, the antibody of the invention is an antibody fragment, such as a Fab' or Fab fragment, a monovalent fragment consisting of the VL, VH, CL and CH1 domains, a monovalent antibody as described in WO2007059782 (Genmab), a F(ab')$_2$ fragment, a Fd fragment, a Fv fragment, a dAb fragment, camelid or nanobodies, or an isolated complementarity determining region (CDR).

Antibodies of the invention are preferably human, humanized or chimeric. In embodiments wherein the antibody is a bispecific antibody, both half-molecules can be human, humanized or chimeric, or the half-molecules can differ in character with respect to sequence origin.

For example, in one embodiment, the bispecific antibody comprises two half-molecules each comprising an antigen-binding region, wherein

(i) the half-molecule(s) comprising the antigen-binding region capable of binding to human PD-L1 is/are chimeric, and/or

(ii) the half-molecule comprising the antigen-binding region capable of binding to human CD3ε (epsilon), if present, is chimeric.

For example, in another embodiment, the bispecific antibody comprises two half-molecules each comprising an antigen-binding region, wherein

(i) the half-molecule(s) comprising the antigen-binding region capable of binding to human PD-L1 is/are humanized, and/or

(ii) the half-molecule comprising the antigen-binding region capable of binding to human CD3ε (epsilon), if present, is humanized.

For example, in a further embodiment, the bispecific antibody comprises two half-molecules each comprising an antigen-binding region, wherein

(i) the half-molecule(s) comprising the antigen-binding region capable of binding to human PD-L1 is/are human, and/or

(ii) the half-molecule comprising the antigen-binding region capable of binding to human CD3ε (epsilon), if present, is human.

Thus, for example, in one embodiment, the antigen-binding region(s) capable of binding to human PD-L1 is/are humanized, and the antigen-binding region capable of binding to human CD3ε (epsilon), if present, is humanized.

In a different embodiment, the antigen-binding region(s) capable of binding to human PD-L1 is/are human, and the antigen-binding region capable of binding to human CD3ε (epsilon), if present, is human.

In a further embodiment, the antibody is bispecific antibody comprising an antigen-binding region capable of binding to human PD-L1 and an antigen-binding region capable of binding to human CD3ε (epsilon), wherein the half-molecule comprising the antigen-binding region capable of binding to human PD-L1 is human, humanized or chimeric, and the half-molecule comprising the antigen-binding region capable of binding to human CD3ε (epsilon) is humanized.

Preferably, the half-molecule comprising the antigen-binding region capable of binding to human PD-L1 is human and the half-molecule comprising the antigen-binding region capable of binding to human CD3ε (epsilon) is humanized.

Bispecific antibody formats

Many different formats and uses of bispecific antibodies are known in the art, and were reviewed by Kontermann; Drug Discov Today, 2015 Jul;20(7):838-47 and; MAbs, 2012 Mar-Apr;4(2):182-97.

A bispecific antibody according to the present invention is not limited to any particular bispecific format or method of producing it.

Examples of bispecific antibody molecules which may be used in the present invention comprise (i) a single antibody that has two arms comprising different antigen-binding regions; (ii) a single chain antibody that has specificity to two different epitopes, e.g., via two scFvs linked in tandem by an extra peptide linker; (iii) a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In: Antibody Engineering, Springer Berlin Heidelberg (2010)); (iv) a chemically-linked bispecific (Fab')2 fragment; (v) a Tandab, which is a fusion of two single chain diabodies resulting in a tetravalent bispecific antibody that has two binding sites for each of the target antigens; (vi) a flexibody, which is a combination of scFvs with a diabody resulting in a multivalent molecule; (vii) a so-called "dock and lock" molecule, based on the "dimerization and docking domain" in Protein Kinase A, which, when applied to Fabs, can yield a trivalent bispecific binding protein consisting of two identical Fab fragments linked to a different Fab fragment; (viii) a so-called Scorpion molecule, comprising, e.g., two scFvs fused to both termini of a human Fab-arm; and (ix) a diabody.

In one embodiment, the bispecific antibody of the present invention is a diabody, a cross-body, or a bispecific antibody obtained via a controlled Fab-arm exchange (such as described in WO2011131746 (Genmab)).

Examples of different classes of bispecific antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant-domains, Fc regions or parts thereof; (v) Fab fusion molecules, wherein different Fab-fragments are fused together, fused to heavy-chain constant-domains, Fc regions or parts thereof; and (vi) ScFv- and diabody-based and heavy chain antibodies (e.g., domain antibodies, nanobodies) wherein different single chain Fv molecules or different diabodies or different heavy-chain antibodies (e.g. domain antibodies, nanobodies) are fused to each other or to another protein or carrier molecule fused to heavy-chain constant-domains, Fc regions or parts thereof.

Examples of IgG-like molecules with complementary CH3 domain molecules include but are not limited to the Triomab/Quadroma molecules (Trion Pharma/Fresenius Biotech; Roche, WO2011069104), the so-called Knobs-into-Holes molecules (Genentech, WO9850431), CrossMAbs (Roche, WO2011117329) and the electrostatically-matched molecules (Amgen, EP1870459 and WO2009089004; Chugai, US201000155133; Oncomed, WO2010129304), the LUZ-Y molecules (Genentech, Wranik et al. J. Biol. Chem. 2012, 287(52): 43331-9, doi: 10.1074/jbc.M112.397869. Epub 2012 Nov 1), DIG-body and PIG-body molecules (Pharmabcine, WO2010134666, WO2014081202), the Strand Exchange Engineered Domain body (SEEDbody) molecules (EMD Serono, WO2007110205), the Biclonics molecules (Merus, WO2013157953), FcΔAdp molecules (Regeneron, WO201015792), bispecific IgG1 and IgG2 molecules (Pfizer/Rinat, WO11143545), Azymetric scaffold molecules (Zymeworks/Merck, WO2012058768), mAb-Fv molecules (Xencor, WO2011028952), bivalent bispecific antibodies (WO2009080254) and the DuoBody® molecules (Genmab, WO2011131746).

Examples of recombinant IgG-like dual targeting molecules include but are not limited to Dual Targeting (DT)-Ig molecules (WO2009058383), Two-in-one Antibody (Genentech; Bostrom, et al 2009. Science 323, 1610-1614.), Cross-linked Mabs (Karmanos Cancer Center), mAb2 (F-Star, WO2008003116), Zybody molecules (Zyngenia; LaFleur et al. MAbs. 2013 Mar-Apr;5(2):208-18), approaches with common light chain (Crucell/Merus, US7,262,028), κλBodies (NovImmune, WO2012023053) and CovX-body (CovX/Pfizer; Doppalapudi, V.R., et al 2007. Bioorg. Med. Chem. Lett. 17,501-506.).

Examples of IgG fusion molecules include but are not limited to Dual Variable Domain (DVD)-Ig molecules (Abbott, US7,612,181), Dual domain double head antibodies (Unilever; Sanofi Aventis, WO20100226923), IgG-like Bispecific molecules (ImClone/Eli Lilly, Lewis et al. Nat Biotechnol. 2014 Feb;32(2):191-8), Ts2Ab (MedImmune/AZ; Dimasi et al. J Mol Biol. 2009 Oct 30;393(3):672-92) and BsAb molecules (Zymogenetics, WO2010111625), HERCULES molecules (Biogen Idec, US007951918), scFv fusion molecules (Novartis), scFv fusion molecules (Changzhou Adam Biotech Inc, CN 102250246) and TvAb molecules (Roche, WO2012025525, WO2012025530).

Examples of Fc fusion molecules include but are not limited to ScFv/Fc Fusions (Pearce et al., Biochem Mol Biol Int. 1997 Sep;42(6):1179-88), SCORPION molecules (Emergent BioSolutions/Trubion, Blankenship JW, et al. AACR 100th Annual meeting 2009 (Abstract # 5465); Zymogenetics/BMS, WO2010111625), Dual Affinity Retargeting Technology (Fc-DART) molecules (MacroGenics, WO2008157379, WO2010080538) and Dual(ScFv)2-Fab molecules (National Research Center for Antibody Medicine - China).

Examples of Fab fusion bispecific antibodies include but are not limited to F(ab)2 molecules (Medarex/AMGEN; Deo et al J Immunol. 1998 Feb 15;160(4):1677-86.), Dual-Action or Bis-Fab molecules (Genentech, Bostrom, et al 2009. Science 323, 1610-1614.), Dock-and-Lock (DNL) molecules (ImmunoMedics, WO2003074569, WO2005004809), Bivalent Bispecific molecules (Biotecnol, Schoonjans, J Immunol. 2000 Dec 15;165(12):7050-7.) and Fab-Fv molecules (UCB-Celltech, WO 2009040562 A1).

Examples of ScFv-, diabody-based and domain antibodies include but are not limited to Bispecific T Cell Engager (BiTE) molecules (Micromet, WO2005061547), Tandem Diabody molecules (TandAb) (Affimed) Le Gall et al., Protein Eng Des Sel. 2004 Apr;17(4):357-66.), Dual Affinity Retargeting Technology (DART) molecules (MacroGenics, WO2008157379, WO2010080538), Single-chain Diabody molecules (Lawrence, FEBS Lett. 1998 Apr 3;425(3):479-84), TCR-like Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75.), dual targeting nanobodies (Ablynx, Hmila et al., FASEB J. 2010) and dual targeting heavy chain only domain antibodies.

In one aspect, the bispecific antibody of the invention comprises a first Fc sequence comprising a first CH3 region, and a second Fc sequence comprising a second CH3 region, wherein the sequences of the first and second CH3 regions are different and are such that the heterodimeric interaction between said first and second CH3 regions is stronger than each of the homodimeric interactions of said first and second CH3 regions. More details on these interactions and how they can be achieved are provided in WO2011131746 and WO2013060867 (Genmab), which are hereby incorporated by reference.

As described further herein, a stable bispecific antibody, such as a bispecific CD3xPD-L1 antibody, can be obtained at high yield using a particular method on the basis of one homodimeric starting PD-L1 antibody and one homodimeric starting antibody capable of binding a different PD-L1 epitope or different antigen (such as a homodimeric starting CD3 antibody) containing only a few, conservative, asymmetrical mutations in the CH3 regions. Asymmetrical mutations mean that the sequences of said first and second CH3 regions contain amino acid substitutions at non-identical positions.

In one embodiment of the bispecific antibody as defined in any of the embodiments disclosed herein, the first CH3 region has an amino acid substitution at a position selected from the group consisting of: 366, 368, 370, 399, 405, 407 and 409, and the second CH3 region has an amino acid substitution at a position selected from the group consisting of: 366, 368, 370, 399, 405, 407 and 409, and wherein the first and second CH3 regions are not substituted in the same positions.

In one embodiment of the bispecific antibody as defined in any of the embodiments disclosed herein, the first CH3 region has an amino acid substitution at position 366, and said second CH3 region has an amino acid substitution at a position selected from the group consisting of: 368, 370, 399, 405, 407 and 409. In one embodiment, the amino acid at position 366 is selected from Ala, Asp, Glu, His, Asn, Val, or Gln.

In one embodiment of the bispecific antibody as defined in any of the embodiments disclosed herein, the first CH3 region has an amino acid substitution at position 368, and said second CH3 region has an amino acid substitution at a position selected from the group consisting of: 366, 370, 399, 405, 407 and 409.

In one embodiment of the bispecific antibody as defined in any of the embodiments disclosed herein, the first CH3 region has an amino acid substitution at position 370, and said second CH3 region has an amino acid substitution at a position selected from the group consisting of: 366, 368, 399, 405, 407 and 409.

In one embodiment of the bispecific antibody as defined in any of the embodiments disclosed herein, the first CH3 region has an amino acid substitution at position 399, and said second CH3 region has an amino acid substitution at a position selected from the group consisting of: 366, 368, 370, 405, 407 and 409.

In one embodiment of the bispecific antibody as defined in any of the embodiments disclosed herein, the first CH3 region has an amino acid substitution at position 405, and said second CH3 region has an amino acid substitution at a position selected from the group consisting of: 366, 368, 370, 399, 407 and 409.

In one embodiment of the bispecific antibody as defined in any of the embodiments disclosed herein, the first CH3 region has an amino acid substitution at position 407, and said second CH3 region has an amino acid substitution at a position selected from the group consisting of: 366, 368, 370, 399, 405, and 409.

In one embodiment of the bispecific antibody as defined in any of the embodiments disclosed herein, the first CH3 region has an amino acid substitution at position 409, and said second CH3 region has an amino acid substitution at a position selected from the group consisting of: 366, 368, 370, 399, 405, and 407.

Accordingly, in one embodiment of the bispecific antibody as defined in any of the embodiments disclosed herein, the sequences of said first and second CH3 regions contain asymmetrical mutations, *i.e.* mutations at different positions in the two CH3 regions, e.g. a mutation at position 405 in one of the CH3 regions and a mutation at position 409 in the other CH3 region.

In one embodiment of the bispecific antibody as defined in any of the embodiments disclosed herein, the first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an amino-acid substitution at a position selected from the group consisting of: 366, 368, 370, 399, 405 and 407. In one such embodiment, said first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an amino acid other than Phe, e.g. Gly, Ala, Val, Ile, Ser, Thr, Lys, Arg, His, Asp, Asn, Glu, Gln, Pro, Trp, Tyr, Cys, Lys, or Leu, at position 405. In a further embodiment hereof, said first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an amino acid other than Phe, Arg or Gly, e.g. Leu, Ala, Val, Ile, Ser, Thr, Met, Lys, His, Asp, Asn, Glu, Gln, Pro, Trp, Tyr, or Cys, at position 405.

In another embodiment of the bispecific antibody as defined in any of the embodiments disclosed herein, said first CH3 region comprises a Phe at position 405 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region comprises an amino acid other than Phe, e.g. Gly, Ala, Val, Ile, Ser, Thr, Lys, Arg, His, Asp, Asn, Glu, Gln, Pro, Trp, Tyr, Leu, Met, or Cys, at position 405 and a Lys at position 409. In a further embodiment hereof, said first CH3 region comprises a Phe at position 405 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region comprises an amino acid other than Phe, Arg or Gly, e.g. Leu, Ala, Val, Ile, Ser, Thr, Met, Lys, His, Asp, Asn, Glu, Gln, Pro, Trp, Tyr, or Cys, at position 405 and a Lys at position 409.

In another embodiment of the bispecific antibody as defined in any of the embodiments disclosed herein, said first CH3 region comprises a Phe at position 405 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region comprises a Leu at position 405 and a Lys at position 409. In a further embodiment hereof, said first CH3 region comprises a Phe at position 405 and an Arg at position 409 and said second CH3 region comprises an amino acid other than Phe, Arg or Gly, e.g. Leu, Ala, Val, Ile, Ser, Thr, Lys, Met, His, Asp, Asn, Glu, Gln, Pro, Trp, Tyr, or Cys, at position 405 and a Lys at position 409. In another embodiment, said first CH3 region comprises Phe at position 405 and an Arg at position 409 and said second CH3 region comprises a Leu at position 405 and a Lys at position 409.

In a further embodiment of the bispecific antibody as defined in any of the embodiments disclosed herein, said first CH3 region comprises an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region comprises a Lys at position 409, a Thr at position 370 and a Leu at position 405. In a further embodiment, said first CH3 region comprises an Arg at position 409 and said second CH3 region comprises a Lys at position 409, a Thr at position 370 and a Leu at position 405.

In an even further embodiment of the bispecific antibody as defined in any of the embodiments disclosed herein, said first CH3 region comprises a Lys at position 370, a Phe at position 405 and an Arg at position 409 and said second CH3 region comprises a Lys at position 409, a Thr at position 370 and a Leu at position 405.

In another embodiment of the bispecific antibody as defined in any of the embodiments disclosed herein, said first CH3 region comprises an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region comprises a Lys at position 409 and: a) an Ile at position 350 and a Leu at position 405, or b) a Thr at position 370 and a Leu at position 405.

In another embodiment of the bispecific antibody as defined in any of the embodiments disclosed herein, said first CH3 region comprises an Arg at position 409 and said second CH3 region comprises a Lys at position 409 and: a) an Ile at position 350 and a Leu at position 405, or b) a Thr at position 370 and a Leu at position 405.

In another embodiment of the bispecific antibody as defined in any of the embodiments disclosed herein, said first CH3 region comprises a Thr at position 350, a Lys at position 370, a Phe at position 405 and an Arg at position 409 and said second CH3 region comprises a Lys at position 409 and: a) an Ile at position 350 and a Leu at position 405, or b) a Thr at position 370 and a Leu at position 405.

In another embodiment of the bispecific antibody as defined in any of the embodiments disclosed herein, said first CH3 region comprises a Thr at position 350, a Lys at position 370, a Phe at position 405 and an Arg at position 409 and said second CH3 region comprises an Ile at position 350, a Thr at position 370, a Leu at position 405 and a Lys at position 409.

In one embodiment of the bispecific antibody as defined in any of the embodiments disclosed herein, said first CH3 region has an amino acid other than Lys, Leu or Met at position 409 and said second CH3 region has an amino acid other than Phe at position 405, such as other than Phe, Arg or Gly at position 405; or said first CH3 region has an amino acid other than Lys, Leu or Met at position 409 and said second CH3 region has an amino acid other than Tyr, Asp, Glu, Phe, Lys, Gln, Arg, Ser or Thr at position 407.

In one embodiment, the bispecific antibody as defined in any of the embodiments disclosed herein comprises a first CH3 region having an amino acid other than Lys, Leu or Met at position 409 and a second CH3 region having an amino acid other than Tyr, Asp, Glu, Phe, Lys, Gln, Arg, Ser or Thr at position 407.

In one embodiment, the bispecific antibody as defined in any of the embodiments disclosed herein comprises a first CH3 region having a Tyr at position 407 and an amino acid other than Lys, Leu or Met at position 409 and a second CH3 region having an amino acid other than Tyr, Asp, Glu, Phe, Lys, Gln, Arg, Ser or Thr at position 407 and a Lys at position 409.

In one embodiment, the bispecific antibody as defined in any of the embodiments disclosed herein comprises a first CH3 region having a Tyr at position 407 and an Arg at position 409 and a second CH3 region having an amino acid other than Tyr, Asp, Glu, Phe, Lys, Gln, Arg, Ser or Thr at position 407 and a Lys at position 409.

In another embodiment, said first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an amino acid other than Tyr, Asp, Glu, Phe, Lys, Gln, Arg, Ser or Thr, e.g. Leu, Met, Gly, Ala, Val, Ile, His, Asn, Pro, Trp, or Cys, at position 407. In another embodiment, said first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an Ala, Gly, His, Ile, Leu, Met, Asn, Val or Trp at position 407.

In another embodiment of the bispecific antibody as defined in any of the embodiments disclosed herein, said first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has a Gly, Leu, Met, Asn or Trp at position 407.

In another embodiment of the bispecific antibody as defined in any of the embodiments disclosed herein, said first CH3 region has a Tyr at position 407 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an amino acid other than Tyr, Asp, Glu, Phe, Lys, Gln, Arg, Ser or Thr, e.g. Leu, Met, Gly, Ala, Val, Ile, His, Asn, Pro, Trp, or Cys, at position 407 and a Lys at position 409.

In another embodiment of the bispecific antibody as defined in any of the embodiments disclosed herein, said first CH3 region has a Tyr at position 407 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has an Ala, Gly, His, Ile, Leu, Met, Asn, Val or Trp at position 407 and a Lys at position 409.

In another embodiment of the bispecific antibody as defined in any of the embodiments disclosed herein, said first CH3 region has a Tyr at position 407 and an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409 and said second CH3 region has a Gly, Leu, Met, Asn or Trp at position 407 and a Lys at position 409.

In another embodiment of the bispecific antibody as defined in any of the embodiments disclosed herein, said first CH3 region has a Tyr at position 407 and an Arg at position 409 and said second CH3 region has an amino acid other than Tyr, Asp, Glu, Phe, Lys, Gln, Arg, Ser or Thr, e.g. Leu, Met, Gly, Ala, Val, Ile, His, Asn, Pro, Trp, or Cys, at position 407 and a Lys at position 409.

In another embodiment of the bispecific antibody as defined in any of the embodiments disclosed herein, said first CH3 region has a Tyr at position 407 and an Arg at position 409 and said second CH3 region has an Ala, Gly, His, Ile, Leu, Met, Asn, Val or Trp at position 407 and a Lys at position 409.

In another embodiment of the bispecific antibody as defined in any of the embodiments disclosed herein, said first CH3 region has a Tyr at position 407 and an Arg at position 409 and said second CH3 region has a Gly, Leu, Met, Asn or Trp at position 407 and a Lys at position 409.

In another embodiment of the bispecific antibody as defined in any of the embodiments disclosed herein, the first CH3 region has an amino acid other than Lys, Leu or Met, e.g. Gly, Ala, Val, Ile, Ser, Thr, Phe, Arg, His, Asp, Asn, Glu, Gln, Pro, Trp, Tyr, or Cys, at position 409, and the second CH3 region has

(i) an amino acid other than Phe, Leu and Met, e.g. Gly, Ala, Val, Ile, Ser, Thr, Lys, Arg, His, Asp, Asn, Glu, Gln, Pro, Trp, Tyr, or Cys, at position 368, or
(ii) a Trp at position 370, or
(iii) an amino acid other than Asp, Cys, Pro, Glu or Gln, e.g. Phe, Leu, Met, Gly, Ala, Val, Ile, Ser, Thr, Lys, Arg, His, Asn, Trp, Tyr, or Cys, at position 399 or
(iv) an amino acid other than Lys, Arg, Ser, Thr, or Trp, e.g. Phe, Leu, Met, Ala, Val, Gly, Ile, Asn, His, Asp, Glu, Gln, Pro, Tyr, or Cys, at position 366.

In one embodiment, the first CH3 region has an Arg, Ala, His or Gly at position 409, and the second CH3 region has

(i) a Lys, Gln, Ala, Asp, Glu, Gly, His, Ile, Asn, Arg, Ser, Thr, Val, or Trp at position 368, or
(ii) a Trp at position 370, or
(iii) an Ala, Gly, Ile, Leu, Met, Asn, Ser, Thr, Trp, Phe, His, Lys, Arg or Tyr at position 399, or
(iv) an Ala, Asp, Glu, His, Asn, Val, Gln, Phe, Gly, Ile, Leu, Met, or Tyr at position 366.

In one embodiment, the first CH3 region has an Arg at position 409, and the second CH3 region has

(i) an Asp, Glu, Gly, Asn, Arg, Ser, Thr, Val, or Trp at position 368, or
(ii) a Trp at position 370, or
(iii) a Phe, His, Lys, Arg or Tyr at position 399, or
(iv) an Ala, Asp, Glu, His, Asn, Val, Gln at position 366.

In a preferred embodiment, the bispecific antibody comprises a first and second heavy chain, wherein each of said first and second heavy chains comprises at least a hinge region, a CH2 and a CH3 region, wherein (i) the amino acid in the position corresponding to F405 (according to EU numbering) is L in said first heavy chain, and the amino acid in the position corresponding to K409 (according to EU numbering) is R in said second heavy chain, or (ii) the amino acid in the position corresponding to K409 (according to EU numbering) is R in said first heavy chain, and the amino acid in the position corresponding to F405 (according to EU numbering) is L in said second heavy chain.

In addition to the above-specified amino-acid substitutions, said first and second heavy chains may contain further amino-acid substitutions, deletion or insertions relative to wild-type heavy chain sequences.

In a further embodiment, said first and second Fab-arms (or heavy chain constant domains) comprise, except for the specified mutations, a CH3 sequence independently selected from the following: (IgG1m(a)) (SEQ ID NO:96), (IgG1m(f)) (SEQ ID NO:97), and (IgG1m(ax)) (SEQ ID NO:98).

In one embodiment, neither said first nor said second Fc sequence comprises a Cys-Pro-Ser-Cys sequence in the (core) hinge region.

In a further embodiment, both said first and said second Fc sequence comprise a Cys-Pro-Pro-Cys sequence in the (core) hinge region.

Methods of preparing bispecific antibodies

Traditional methods such as the hybrid hybridoma and chemical conjugation methods (Marvin and Zhu (2005) Acta Pharmacol Sin 26:649) can be used in the preparation of the bispecific antibodies of the invention. Co-expression in a host cell of two antibodies, consisting of different heavy and light chains, leads to a mixture of possible antibody products in addition to the desired bispecific antibody, which can then be isolated by, e.g., affinity chromatography or similar methods.

Strategies favoring the formation of a functional bispecific, product, upon co-expression of different antibody constructs can also be used, e.g., the method described by Lindhofer et al. (1995 J Immunol 155:219). Fusion of rat and mouse hybridomas producing different antibodies leads to a limited number of heterodimeric proteins because of preferential species-restricted heavy/light chain pairing. Another strategy to promote formation of heterodimers over homodimers is a "knob-into-hole" strategy in which a protuberance is introduced on a first heavy-chain polypeptide and a corresponding cavity in a second heavy-chain polypeptide, such that the protuberance can be positioned in the cavity at the interface of these two heavy chains so as to promote heterodimer formation and hinder homodimer formation. "Protuberances" are constructed by replacing small amino-acid side-chains from the interface of the first polypeptide with larger side chains. Compensatory "cavities" of identical or similar size to the protuberances are created in the interface of the second polypeptide by replacing large amino-acid side-chains with smaller ones (US patent 5,731,168). EP1870459 (Chugai) and WO2009089004 (Amgen) describe other strategies for favoring heterodimer formation upon co-expression of different antibody domains in a host cell. In these methods, one or more residues that make up the CH3-CH3 interface in both CH3 domains are replaced with a charged amino acid such that homodimer formation is electrostatically unfavorable and heterodimerization is electrostatically favorable. WO2007110205 (Merck) describe yet another strategy, wherein differences between IgA and IgG CH3 domains are exploited to promote heterodimerization.

Another *in vitro* method for producing bispecific antibodies has been described in WO2008119353 (Genmab), wherein a bispecific antibody is formed by "Fab-arm" or "half-molecule" exchange (swapping of a heavy chain and attached light chain) between two monospecific IgG4- or IgG4-like antibodies upon incubation under reducing conditions. The resulting product is a bispecific antibody having two Fab arms which may comprise different sequences.

A preferred method for preparing bispecific antibodies, such as bispecific CD3xPD-L1 antibodies, of the present invention includes the methods described in WO2011131746 and WO2013060867 (Genmab) comprising the following

**EP 4 527 854 A2**

steps:

a) providing a first antibody comprising an Fc region, said Fc region comprising a first CH3 region;

b) providing a second antibody comprising a second Fc region, said Fc region comprising a second CH3 region,

wherein the first antibody is a PD-L1 antibody according to the invention and the second antibody is antibody which is capable of binding to a different PD-L1 epitope or a different antigen, such as human CD3, or vice versa; and

wherein the sequences of said first and second CH3 regions are different and are such that the heterodimeric interaction between said first and second CH3 regions is stronger than each of the homodimeric interactions of said first and second CH3 regions;

c) incubating said first antibody together with said second antibody under reducing conditions; and

d) obtaining said bispecific antibody, e.g. a bispecific PD-L1xCD3 antibody.

Similarly, there is provided a method for producing an antibody according to the invention, comprising the steps of:

a) culturing a host cell producing a first antibody comprising an antigen-binding region capable of binding to human PD-L1 as defined herein and purifying said first antibody from the culture;

b) culturing a host cell producing a second antibody comprising an antigen-binding region capable of binding to a different PD-L1 epitope or a different antigen, e.g. a human CD3ε binding region as defined herein, purifying said second antibody from the culture;

c) incubating said first antibody together with said second antibody under reducing conditions sufficient to allow the cysteines in the hinge region to undergo disulfide-bond isomerization, and

d) obtaining said bispecific antibody.

In one embodiment, the said first antibody together with said second antibody are incubated under reducing conditions sufficient to allow the cysteines in the hinge region to undergo disulfide-bond isomerization, wherein the heterodimeric interaction between said first and second antibodies in the resulting heterodimeric antibody is such that no Fab-arm exchange occurs at 0.5 mM GSH after 24 hours at 37°C.

Without being limited to theory, in step c), the heavy-chain disulfide bonds in the hinge regions of the parent antibodies are reduced and the resulting cysteines are then able to form inter heavy-chain disulfide bond with cysteine residues of another parent antibody molecule (originally with a different specificity). In one embodiment of this method, the reducing conditions in step c) comprise the addition of a reducing agent, e.g. a reducing agent selected from the group consisting of: 2-mercaptoethylamine (2-MEA), dithiothreitol (DTT), dithioerythritol (DTE), glutathione, tris(2-carboxyethyl) phosphine (TCEP), L-cysteine and beta-mercapto-ethanol, preferably a reducing agent selected from the group consisting of: 2-mercaptoethylamine, dithiothreitol and tris(2-carboxyethyl)phosphine. In a further embodiment, step c) comprises restoring the conditions to become non-reducing or less reducing, for example by removal of a reducing agent, e.g. by desalting.

For this method, any of the antibodies, e.g. CD3 and PD-L1 antibodies described above may be used including first and second CD3 and PD-L1 antibodies, respectively, comprising a first and/or second Fc region. Examples of such first and second Fc regions, including combination of such first and second Fc regions may include any of those described above. In a particular embodiment, the first and second antibodies, e.g. CD3 and PD-L1 antibodies, respectively, may be chosen so as to obtain a bispecific antibody as described herein.

In one embodiment of this method, said first and/or second antibodies are full-length antibodies.

The Fc regions of the first and second antibodies may be of any isotype, including, but not limited to, IgG1, IgG2, IgG3 or IgG4. In one embodiment of this method, the Fc regions of both said first and said second antibodies are of the IgG1 isotype. In another embodiment, one of the Fc regions of said antibodies is of the IgG1 isotype and the other of the IgG4 isotype. In the latter embodiment, the resulting bispecific antibody comprises an Fc sequence of an IgG1 and an Fc sequence of IgG4 and may thus have interesting intermediate properties with respect to activation of effector functions.

In a further embodiment, one of the antibody starting proteins has been engineered to not bind Protein A, thus allowing to separate the heterodimeric protein from said homodimeric starting protein by passing the product over a protein A column.

As described above, the sequences of the first and second CH3 regions of the homodimeric starting antibodies are different and are such that the heterodimeric interaction between said first and second CH3 regions is stronger than each of the homodimeric interactions of said first and second CH3 regions. More details on these interactions and how they can be achieved are provided in WO2011131746 and WO2013060867 (Genmab), which are hereby incorporated by reference in their entirety.

In particular, a stable bispecific antibody, e.g. a bispecific CD3xPD-L1 antibody, can be obtained at high yield using the above method of the invention on the basis of two homodimeric starting antibodies which bind PD-L1 and a different antigen or a different epitope of PD-L1, e.g. CD3, respectively, and contain only a few, conservative, asymmetrical mutations in the CH3 regions. Asymmetrical mutations mean that the sequences of said first and second CH3 regions contain amino acid substitutions at non-identical positions.

The bispecific antibodies of the invention may also be obtained by co-expression of constructs encoding the first and second polypeptides in a single cell. Thus, in a further aspect, the invention relates to a method for producing a bispecific antibody, said method comprising the following steps:

a) providing a first nucleic-acid construct encoding a first polypeptide comprising a first Fc sequence and a first antigen-binding region of a first antibody heavy chain, said first Fc sequence comprising a first CH3 region,
b) providing a second nucleic-acid construct encoding a second polypeptide comprising a second Fc sequence and a second antigen-binding region of a second antibody heavy chain, said second Fc sequence comprising a second CH3 region,

wherein the sequences of said first and second CH3 regions are different and are such that the heterodimeric interaction between said first and second CH3 regions is stronger than each of the homodimeric interactions of said first and second CH3 regions, and wherein said first homodimeric protein has an amino acid other than Lys, Leu or Met at position 409 and said second homodimeric protein has an amino-acid substitution at a position selected from the group consisting of: 366, 368, 370, 399, 405 and 407,
optionally wherein said first and second nucleic acid constructs encode light chain sequences of said first and second antibodies

c) co-expressing said first and second nucleic-acid constructs in a host cell, and
d) obtaining said heterodimeric protein from the cell culture.

Materials and methods for the production of antibodies of the invention

In further aspects, the invention relates to materials and methods for the recombinant production of antibodies according to the invention. Suitable expression vectors, including promoters, enhancers, etc., and suitable host cells for the production of antibodies are well-known in the art.

Thus, in one aspect, there is provided a nucleic acid construct comprising:

(i) a nucleic acid sequence encoding a heavy chain sequence of an antibody comprising an antigen-binding region capable of binding to human PD-L1 as defined herein, and/or

(ii) a nucleic acid sequence encoding a light chain sequence of an antibody comprising an antigen-binding region capable of binding to human PD-L1 as defined herein.

In one embodiment, the nucleic acid construct further comprises:

(i) a nucleic acid sequence encoding a heavy chain sequence of an antibody comprising an antigen-binding region capable of binding to a different PD-L1 epitope or a different antigen, e.g. human CD3ε as defined herein, and

(ii) a nucleic acid sequence encoding a light chain sequence of an antibody comprising an antigen-binding region capable of binding to a different PD-L1 epitope or a different antigen, e.g. human CD3ε as defined herein.

In an even further aspect, the invention relates to an expression vector comprising nucleic acid constructs as defined herein above.

An expression vector in the context of the present invention may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment, an antibody-encoding mucleic acid, e.g. a PD-L1 or a CD3 antibody-encoding nucleic acid, is comprised in a naked DNA or RNA vector, including, for example, a linear expression element (as described in for instance Sykes and Johnston, Nat Biotech 17, 355-59 (1997)), a compacted nucleic acid vector (as described in for instance US 6,077, 835 and/or WO 00/70087), a plasmid vector such as pBR322, pUC 19/18, or pUC 118/119, a "midge" minimally-sized nucleic acid vector (as described in for instance Schakowski et al., Mol Ther 3, 793-800 (2001)), or as a precipitated nucleic acid vector construct, such as a Ca3(P04)2-precipitated construct (as described in for instance WO200046147, Benvenisty and Reshef, PNAS USA 83, 9551-55 (1986), Wigler et al., Cell 14, 725 (1978), and Coraro and Pearson, Somatic Cell Genetics 7, 603 (1981)). Such nucleic acid vectors and the usage thereof are well known in the art (see for instance US 5,589,466 and US 5,973,972).

In one embodiment, the vector is suitable for expression of the antibody, e.g. the PD-L1 antibody and/or the CD3 antibody in a bacterial cell. Examples of such vectors include expression vectors such as BlueScript (Stratagene), pIN vectors (Van Heeke & Schuster, J Biol Chem 264, 5503-5509 (1989), pET vectors (Novagen, Madison WI) and the like).

An expression vector may also or alternatively be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in: F. Ausubel et al., ed. Current Protocols in Molecular Biology, Greene Publishing and Wiley InterScience New York (1987), and Grant et al., Methods in Enzymol 153, 516-544 (1987)).

An expression vector may also or alternatively be a vector suitable for expression in mammalian cells, e.g. a vector comprising glutamine synthetase as a selectable marker, such as the vectors described in Bebbington (1992) Biotechnology (NY) 10:169-175.

A nucleic acid and/or vector may also comprise a nucleic acid sequence encoding a secretion/localization sequence, which can target a polypeptide, such as a nascent polypeptide chain, to the periplasmic space or into cell culture media. Such sequences are known in the art, and include secretion leader or signal peptides.

The expression vector may comprise or be associated with any suitable promoter, enhancer, and other expression-facilitating elements. Examples of such elements include strong expression promoters (e. g., human CMV IE promoter/enhancer as well as RSV, SV40, SL3-3, MMTV, and HIV LTR promoters), effective poly (A) termination sequences, an origin of replication for plasmid product in E. coli, an antibiotic resistance gene as selectable marker, and/or a convenient cloning site (e.g., a polylinker). Nucleic acids may also comprise an inducible promoter as opposed to a constitutive promoter such as CMV IE.

In one embodiment, the antibody-encoding expression vector, e.g. the PD-L1 and/or CD3 antibody-encoding expression vector may be positioned in and/or delivered to the host cell or host animal via a viral vector.

In an even further aspect, the invention relates to a host cell comprising one or more of the nucleic-acid constructs or the expression vector specified herein above.

Thus, the present invention also relates to a recombinant eukaryotic or prokaryotic host cell which produces an antibody of the present invention, such as a transfectoma.

Examples of host cells include yeast, bacterial, plant and mammalian cells, such as CHO, CHO-S, HEK, HEK293, HEK-293F, Expi293F, PER.C6 or NS0 cells or lymphocytic cells. A preferred host cell is a CHO-K1 cell.

For example, in one embodiment, the host cell may comprise a first and second nucleic acid construct stably integrated into the cellular genome. In another embodiment, the present invention provides a cell comprising a non-integrated nucleic acid, such as a plasmid, cosmid, phagemid, or linear expression element, which comprises a first and second nucleic acid construct as specified above.

In a further aspect, the invention relates to a hybridoma which produces a PD-L1 antibody as defined herein.

Fc regions

In some embodiments, the antibody according to the present invention comprises, in addition to the antigen-binding regions, an Fc region consisting of the Fc sequences of the two heavy chains.

The first and second Fc sequences may each be of any isotype, including, but not limited to, IgG1, IgG2, IgG3 and IgG4, and may comprise one or more mutations or modifications. In one embodiment, each of the first and second Fc sequences is of the IgG4 isotype or derived therefrom, optionally with one or more mutations or modifications. In another embodiment, each of the first and second Fc sequences is of the IgG1 isotype or derived therefrom, optionally with one or more mutations or modifications. In another embodiment, one of the Fc sequences is of the IgG1 isotype and the other of the IgG4 isotype, or is derived from such respective isotypes, optionally with one or more mutations or modifications.

In one embodiment, one or both Fc sequences are effector-function-deficient. For example, the Fc sequence(s) may be of an IgG4 isotype, or a non-IgG4 type, e.g. IgG1, IgG2 or IgG3, which has been mutated such that the ability to mediate effector functions, such as ADCC, has been reduced or even eliminated. Such mutations have e.g. been described in Dall'Acqua WF et al., J Immunol. 177(2):1129-1138 (2006) and Hezareh M, J Virol.; 75(24):12161-12168 (2001). In another embodiment, one or both Fc sequences comprise an IgG1 wildtype sequence.

Antibodies according to the present invention may comprise modifications in the Fc region. When an antibody comprises such modifications, it may become an inert, or non-activating, antibody. The term "inertness", "inert" or "non-activating" as used herein, refers to an Fc region which is at least not able to bind any Fcγ receptors, induce Fc-mediated cross-linking of FcRs, or induce FcR-mediated cross-linking of target antigens via two Fc regions of individual antibodies, or is not able to bind C1q. The inertness of an Fc region of an antibody, e.g. a humanized or chimeric CD3 antibody, is advantageously tested using the antibody in a monospecific format.

Several variants can be constructed to make the Fc region of an antibody inactive for interactions with Fcγ (gamma) receptors and C1q for therapeutic antibody development. Examples of such variants are described herein.

Thus, in one embodiment of the antibody of the invention, said antibody comprises a first and a second heavy chain, wherein one or both heavy chains are modified so that the antibody induces Fc-mediated effector function to a lesser extent relative to an antibody which is identical, except for comprising non-modified first and second heavy chains. Said Fc-mediated effector function may be measured by determining Fc-mediated CD69 expression, by binding to Fcγ receptors, by binding to C1q, or by induction of Fc-mediated cross-linking of FcRs.

In one such embodiment, the heavy chain constant sequences have been modified so that said antibody reduces Fc-mediated CD69 expression by at least 50%, at least 60%, at least 70%, at least 80%, at least 90%, at least 99% or 100% when compared to a wild-type (unmodified) antibody, wherein said Fc-mediated CD69 expression is determined in a PBMC-based functional assay, e.g. as described in Example 3 of WO2015001085.

In another such embodiment, the heavy and light chain constant sequences have been modified so that binding of C1q to said antibody is reduced compared to an unmodified antibody by at least 70%, at least 80%, at least 90%, at least 95%, at least 97%, or 100%, wherein C1q binding is determined by ELISA.

In another embodiment, the antibody comprises an Fc region which has been modified so that said antibody mediates reduced Fc-mediated T-cell proliferation compared to an unmodified antibody by at least 50%, at least 60%, at least 70%, at least 80%, at least 90%, at least 99% or 100%, wherein said T-cell proliferation is measured in a peripheral blood mononuclear cell (PBMC)-based functional assay.

Thus, amino acids in the Fc region that play a dominant role in the interactions with C1q and the Fcγ receptors may be modified.

Examples of amino acid positions that may be modified, e.g. in an IgG1 isotype antibody, include positions L234, L235 and P331. Combinations thereof, such as L234F/L235E/P331S, can cause a profound decrease in binding to human CD64, CD32, CD16 and C1q.

Hence, in one embodiment, the amino acid in at least one position corresponding to L234, L235 and P331, may be A, A and S, respectively (Xu et al., 2000, Cell Immunol. 200(1):16-26; Oganesyan et al., 2008, Acta Cryst. (D64):700-4). Also, L234F and L235E amino acid substitutions can result in Fc regions with abrogated interactions with Fcγ receptors and C1q (Canfield et al., 1991, J. Exp. Med. (173):1483-91; Duncan et al., 1988, Nature (332):738-40). Hence, in one embodiment, the amino acids in the positions corresponding to L234 and L235, may be F and E, respectively. A D265A amino acid substitution can decrease binding to all Fcγ receptors and prevent ADCC (Shields et al., 2001, J. Biol. Chem. (276):6591-604). Hence, in one embodiment, the amino acid in the position corresponding to D265 may be A. Binding to C1q can be abrogated by mutating positions D270, K322, P329, and P331. Mutating these positions to either D270A or K322A or P329A or P331A can make the antibody deficient in CDC activity Idusogie EE, et al., 2000, J Immunol. 164: 4178-84). Hence, in one embodiment, the amino acids in at least one position corresponding to D270, K322, P329 and P331, may be A, A, A, and A, respectively.

An alternative approach to minimize the interaction of the Fc region with Fcγ receptors and C1q is by removal of the glycosylation site of an antibody. Mutating position N297 to e.g. Q, A, or E removes a glycosylation site which is critical for IgG-Fc gamma Receptor interactions. Hence, in one embodiment, the amino acid in a position corresponding to N297, may be G, Q, A or E Leabman et al., 2013, MAbs; 5(6):896-903). Another alternative approach to minimize interaction of the Fc region with Fcγ receptors may be obtained by the following mutations; P238A, A327Q, P329A or E233P/L234V/L235A/G236del (Shields et al., 2001, J. Biol. Chem. (276):6591-604).

Alternatively, human IgG2 and IgG4 subclasses are considered naturally compromised in their interactions with C1q and Fc gamma Receptors although interactions with Fcγ receptors were reported (Parren et al., 1992, J. Clin Invest. 90: 1537-1546; Bruhns et al., 2009, Blood 113: 3716-3725). Mutations abrogating these residual interactions can be made in both isotypes, resulting in reduction of unwanted side-effects associated with FcR binding. For IgG2, these include L234A and G237A, and for IgG4, L235E. Hence, in one embodiment, the amino acid in a position corresponding to L234 and G237 in a human IgG2 heavy chain, may be A and A, respectively. In one embodiment, the amino acid in a position corresponding to L235 in a human IgG4 heavy chain, may be E.

Other approaches to further minimize the interaction with Fcγ receptors and C1q in IgG2 antibodies include those described in WO2011066501 and Lightle, S., et al., 2010, Protein Science (19):753-62.

The hinge region of the antibody can also be of importance with respect to interactions with Fcγ receptors and complement (Brekke et al., 2006, J Immunol 177:1129-1138; Dall'Acqua WF, et al., 2006, J Immunol 177:1129-1138). Accordingly, mutations in or deletion of the hinge region can influence effector functions of an antibody.

Thus, in one embodiment, the antibody comprises a first and a second immunoglobulin heavy chain, wherein in at least one of said first and second immunoglobulin heavy chains one or more amino acids in the positions corresponding to positions L234, L235, D265, N297, and P331 in a human IgG1 heavy chain, are not L, L, D, N, and P, respectively.

In one embodiment, in both the first and second heavy chains one or more amino acids in the position corresponding to positions L234, L235, D265, N297, and P331 in a human IgG1 heavy chain, are not L, L, D, N, and P, respectively.

In one embodiment, in both said first and second heavy chains the amino acid in the position corresponding to position D265 in a human IgG1 heavy chain, is not D.

Thus, in one embodiment, in both said first and second heavy chains the amino acid in the position corresponding to position D265 in a human IgG1 heavy chain are selected from the group consisting of: A and E.

In further embodiment, in at least one of said first and second heavy chains the amino acids in the positions corresponding to positions L234 and L235 in a human IgG1 heavy chain, are not L and L, respectively.

In a particular embodiment, in at least one of said first and second heavy chains the amino acids in the positions corresponding to positions L234 and L235 in a human IgG1 heavy chain, are F and E, respectively.

In one embodiment, in both said first and second heavy chains the amino acids in the positions corresponding to positions L234 and L235 in a human IgG1 heavy chain, are F and E, respectively.

In a particular embodiment, in at least one of said first and second heavy chains the amino acids in the positions corresponding to positions L234, L235, and D265 in a human IgG1 heavy chain, are F, E, and A, respectively.

In a particularly preferred embodiment, in both said first and second heavy chains the amino acids in the positions corresponding to positions L234, L235, and D265 in a human IgG1 heavy chain, are F, E, and A, respectively.

In a further particularly preferred embodiment, the antibody is a bispecific antibody comprising a first and second heavy chain, wherein the positions corresponding to positions L234 and L235 in a human IgG1 heavy chain according to EU numbering of both the first heavy chain and the second heavy chain are F and E, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

In a further particularly preferred embodiment, the antibody is a bispecific antibody comprising a first and second heavy chain, wherein the positions corresponding to positions L234, L235, and D265 in a human IgG1 heavy chain according to EU numbering of both the first heavy chain and the second heavy chain are F, E, and A, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

Antibody variants having the combination of three amino acid substitutions L234F, L235E and D265A and in addition the K409R or the F405L mutation are herein termed with the suffix "FEAR" or "FEAL", respectively.

Herein, huCD3-H1L1 refers to the humanized SP34 anti-CD3 antibody having VH and VL sequences as set forth in SEQ ID NOs: 25 and 29.

In a preferred embodiment, the bispecific antibody of the invention comprises:

(i) a half-molecule antibody derived from IgG1-huCD3-H1L1-FEAL, and a half-molecule antibody derived from IgG1-PDL1-338-FEAR, IgG1-PDL1-511-FEAR or IgG1-PDL1-547-FEAR, or
(ii) a half-molecule antibody derived from IgG1-huCD3-H1L1-FEAR, and a half-molecule antibody derived from and a half-molecule antibody derived from IgG1-PDL1-338-FEAL, IgG1-PDL1-511-FEAL or IgG1-PDL1-547-FEAL.

In a further embodiment, the first heavy chain or half-molecule comprises the sequence set forth in SEQ ID NO:90 and the second heavy chain comprises the sequence set forth in SEQ ID NO:89.

In a further embodiment of the invention, one or both antibodies forming part of the bispecific antibody have been engineered to reduce or increase the binding to the neonatal Fc receptor (FcRn) in order to manipulate the serum half-life of the bispecific antibody. Techniques for increasing or reducing the serum half-life are well-known in the art. See for example Dall'Acqua et al. 2006, J. Biol. Chem., 281:23514-24; Hinton et al. 2006, J. Immunol., 176:346-56; and Zalevsky et al. 2010 Nat. Biotechnol., 28:157-9.

Conjugates

In a further aspect, the present invention provides antibodies that are linked or conjugated to one or more therapeutic moieties, such as a cytokine, an immune-suppressant, an immune-stimulatory molecule and/or a radioisotope. Such conjugates are referred to herein as "immunoconjugates" or "drug conjugates". Immunoconjugates which include one or more cytotoxins are referred to as "immunotoxins".

In one embodiment, the first and/or second Fc sequence is conjugated to a drug or a prodrug or contains an acceptor group for the same. Such acceptor group may e.g. be an unnatural amino acid.

Compositions

In a further aspect, the invention relates to a pharmaceutical composition comprising an antibody according to any one of the embodiments disclosed herein and a pharmaceutically-acceptable carrier.

The pharmaceutical composition of the present invention may contain one antibody of the present invention or a combination of different antibodies of the present invention.

The pharmaceutical compositions may be formulated in accordance with conventional techniques such as those disclosed in Remington: The Science and Practice of Pharmacy, 19th Edition, Gennaro, Ed., Mack Publishing Co., Easton, PA, 1995. A pharmaceutical composition of the present invention may e.g. include diluents, fillers, salts, buffers, detergents (e. g., a nonionic detergent, such as Tween-20 or Tween-80), stabilizers (e. g., sugars or protein-free amino acids), preservatives, tissue fixatives, solubilizers, and/or other materials suitable for inclusion in a pharmaceutical composition.

Pharmaceutically acceptable carriers include any and all suitable solvents, dispersion media, coatings, antibacterial and antifungal agents, isotonicity agents, antioxidants and absorption delaying agents, and the like that are physiologically compatible with an antibody of the present invention. Examples of suitable aqueous and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, carboxymethyl cellulose colloidal solutions, tragacanth gum and injectable organic esters, such as ethyl oleate, and/or various buffers. Pharmaceutically acceptable carriers include sterile aqueous solutions or dispersions and sterile powders for the extemporaneous preparation of sterile injectable solutions or dispersion. Proper fluidity may be maintained, for example, using coating materials, such as lecithin, by the maintenance of the required particle size in the case of dispersions, and using surfactants.

Pharmaceutical compositions of the present invention may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole, butylated hydroxytoluene, lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Pharmaceutical compositions of the present invention may also comprise isotonicity agents, such as sugars, polyalcohols, such as mannitol, sorbitol, glycerol or sodium chloride in the compositions.

The pharmaceutical compositions of the present invention may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The antibodies of the present invention may be prepared with carriers that will protect the antibody against rapid release, such as a controlled release formulation, including implants, transdermal patches, and microencapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, polyorthoesters, and polylactic acid alone or with a wax, or other materials well known in the art. Methods for the preparation of such formulations are generally known to those skilled in the art.

Sterile injectable solutions may be prepared by incorporating the active compound in the required amount in an appropriate solvent with one or a combination of ingredients e.g. as enumerated above, as required, followed by sterilization microfiltration. Generally, dispersions are prepared by incorporating the active compound into a sterile vehicle that contains a basic dispersion medium and the required other ingredients e.g. from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

The actual dosage levels of the active ingredients in the pharmaceutical compositions may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The pharmaceutical composition may be administered by any suitable route and mode. In one embodiment, a pharmaceutical composition of the present invention is administered parenterally. "Administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In one embodiment, that pharmaceutical composition is administered by intravenous or subcutaneous injection or infusion.

Uses

In one aspect, the invention relates to the antibody according to any one of the embodiments disclosed herein, or the pharmaceutical composition as disclosed herein, for use as a medicament.

In a further aspect, the invention relates to the antibody according to any one of the embodiments disclosed herein, or the pharmaceutical composition as disclosed herein for use in the treatment of a disease, such as cancer.

In a further aspect, the invention relates to a method of treatment of a disease comprising administering an effective amount of antibody according to any one of the embodiments disclosed herein, or the pharmaceutical composition as disclosed herein to a subject in need thereof.

In particular, the bispecific antibodies according to the invention may be useful in therapeutic settings in which specific targeting and T cell-mediated killing of cells that express PD-L1 is desired, and they may be more efficient compared to a regular anti-PD-L1 antibody in certain such indications and settings.

The antibodies of the invention also have additional utility in therapy and diagnosis of a variety of PD-L1-related diseases. For example, the antibodies can be used to elicit *in vivo* or *in vitro* one or more of the following biological activities: to inhibit the growth of and/or differentiation of a cell expressing PD-L1; to kill a cell expressing PD-L1; to mediate phagocytosis or ADCC of a cell expressing PD-L1 in the presence of human effector cells; to mediate CDC of a cell expressing PD-L1 in the presence of complement; to mediate apoptosis of a cell expressing PD-L1; and/or to induce translocation into lipid rafts upon binding PD-L1.

In one aspect, the invention relates to the antibody according to any one of the embodiments disclosed herein, or the pharmaceutical composition as disclosed herein for use in the treatment of cancer.

In a further aspect, the invention relates to the antibody according to any one of the embodiments disclosed herein, or the pharmaceutical composition as disclosed herein for use in the treatment of cancer disease characterized by the presence of solid tumors.

In a further aspect, the invention relates to the antibody according to any one of the embodiments disclosed herein, or the pharmaceutical composition as disclosed herein for use in the treatment of cancer disease selected from the group consisting of: melanoma, ovarian cancer, lung cancer, colorectal cancer, head and neck cancer, gastric cancer, breast cancer, renal cancer, bladder cancer, esophageal cancer, pancreatic cancer, hepatic cancer, thymoma and thymic carcinoma, brain cancer, glioma, adrenocortical carcinoma, thyroid cancer, other skin cancers, sarcoma, multiple myeloma, leukemia, lymphoma, myelodysplastic syndromes, ovarian cancer, endometriosis cancer, prostate cancer, penile cancer, Hodgkins lymphoma, non-Hodgkins lymphoma, Merkel cell carcinoma and mesothelioma.

In a further aspect, the invention relates to the use of an antibody according to any one of the embodiments disclosed herein for the manufacture of a medicament, such as a medicament for the treatment of cancer, e.g. a cancer disease characterized by the presence of solid tumors or a cancer disease selected from the group consisting of: melanoma, ovarian cancer, lung cancer, colon cancer and head and neck cancer.

The present invention also relates to a method for inhibiting growth and/or proliferation of one or more tumor cells expressing PD-L1, comprising administration, to an individual in need thereof, of an antibody of the present invention.

The present invention alto relates to a method for treating cancer, comprising

a) selecting a subject suffering from a cancer comprising tumor cells expressing PD-L1, and
b) administering to the subject the antibody of the present invention or a pharmaceutical composition of the present invention.

Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage.

The efficient dosages and the dosage regimens for the antibodies depend on the disease or condition to be treated and may be determined by the persons skilled in the art. An exemplary, non-limiting range for a therapeutically effective amount of a compound of the present invention is about 0.001-10 mg/kg, such as about 0.001-5 mg/kg, for example about 0.001-2 mg/kg, such as about 0.001-1 mg/kg, for instance about 0.001, about 0.01, about 0.1, about 1 or about 10 mg/kg. Another exemplary, non-limiting range for a therapeutically effective amount of an antibody of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3, about 5, or about 8 mg/kg.

A physician or veterinarian having ordinary skill in the art may readily determine and prescribe the effective amount of the pharmaceutical composition required. For example, the physician or veterinarian could start doses of the antibody employed in the pharmaceutical composition at levels lower than that required to achieve the desired therapeutic effect and gradually increase the dosage until the desired effect is achieved. In general, a suitable daily dose of an antibody of the present invention will be that amount of the compound which is the lowest dose effective to produce a therapeutic effect. Administration may e.g. be parenteral, such as intravenous, intramuscular or subcutaneous. In one embodiment, the antibodies may be administered by infusion in a weekly dosage of calculated by mg/m$^2$. Such dosages can, for example, be based on the mg/kg dosages provided above according to the following: dose (mg/kg) $\times$ 70: 1.8. Such administration may be repeated, e.g., 1 to 8 times, such as 3 to 5 times. The administration may be performed by continuous infusion over a period of from 2 to 24 hours, such as from 2 to 12 hours. In one embodiment, the antibodies may be administered by slow continuous infusion over a long period, such as more than 24 hours, to reduce toxic side effects.

In one embodiment, the antibodies may be administered in a weekly dosage of calculated as a fixed dose for up to 8 times, such as from 4 to 6 times when given once a week. Such regimen may be repeated one or more times as necessary, for example, after 6 months or 12 months. Such fixed dosages can, for example, be based on the mg/kg dosages provided above, with a body weight estimate of 70 kg. The dosage may be determined or adjusted by measuring the amount of antibody of the present invention in the blood upon administration by for instance taking out a biological sample and using anti-idiotypic antibodies which target the PD-L1 antigen antigen-binding region of the antibodies of the present invention.

In one embodiment, the antibodies may be administered as maintenance therapy, such as, e.g., once a week for a period of 6 months or more.

An antibody may also be administered prophylactically to reduce the risk of developing cancer, delay the onset of the occurrence of an event in cancer progression, and/or reduce the risk of recurrence when a cancer is in remission.

The antibodies of the invention may also be administered in combination therapy, *i.e.*, combined with other therapeutic agents relevant for the disease or condition to be treated. Accordingly, in one embodiment, the antibody-containing medicament is for combination with one or more further therapeutic agents, such as a cytotoxic, chemotherapeutic or anti-angiogenic agent.

In a further aspect, the invention relates to an anti-idiotypic antibody which binds to the PD-L1-binding region as defined in any one of the embodiments disclosed herein.

Additional items of the present disclosure:

1. An antibody comprising an antigen-binding region capable of binding to human PD-L1, wherein the antibody inhibits the binding of human PD-L1 to human PD-1, and

(i) competes for binding to human PD-L1 with an antibody comprising a VH sequence as set forth in SEQ ID NO:8 and a VL sequence as set forth in SEQ ID NO: 15 **[511],** but does not compete for binding to human PD-L1 with an antibody comprising a VH sequence as set forth in SEQ ID NO:18 and a VL sequence as set forth in SEQ ID NO:22 **[547],** or

(ii) competes for binding to human PD-L1 with an antibody comprising a VH sequence as set forth in SEQ ID NO:18 and a VL sequence as set forth in SEQ ID NO:22 **[547],** but does not compete for binding to human PD-L1 with an antibody comprising a VH sequence as set forth in SEQ ID NO:8 and a VL sequence as set forth in SEQ ID NO: 15 **[511].**

2. The antibody according to item 1, wherein said antibody competes for binding to human PD-L1 with an antibody comprising a VH sequence as set forth in SEQ ID NO:1 and a VL sequence as set forth in SEQ ID NO:5 **[338].**

3. The antibody according to any of the preceding items, wherein the binding of said antibody to human PD-L1 is not displaced by an antibody comprising a VH sequence as set forth in SEQ ID NO:53 and a VL sequence as set forth in SEQ ID NO:57**[476].**

4. The antibody according to any of the preceding items, wherein the binding of said antibody to human PD-L1 is not blocked by binding of an antibody comprising a VH sequence as set forth in SEQ ID NO: 106 and a VL sequence as set forth in SEQ ID NO: 110 **[625].**

5. The antibody according to any of the preceding items, wherein the binding of said antibody to human PD-L1 is blocked by an antibody comprising a VH sequence as set forth in SEQ ID NO:18 and a VL sequence as set forth in SEQ ID NO:22 **[547].**

6. The antibody according to item 1, wherein said antibody:

(i) is capable of binding to the same epitope of human PD-L1 as an antibody comprising a VH sequence as set forth in SEQ ID NO:1 and a VL sequence as set forth in SEQ ID NO:5 **[338],** or

(ii) is capable of binding to the same epitope of human PD-L1 as an antibody comprising a VH sequence as set forth in SEQ ID NO:8 and a VL sequence as set forth in SEQ ID NO:15 **[511],** or

(iii) is capable of binding to the same epitope of human PD-L1 as an antibody comprising a VH sequence as set forth in SEQ ID NO:18 and a VL sequence as set forth in SEQ ID NO:22 **[547].**

7. The antibody according to any one of items 1-4 and 6, wherein binding of the antibody to a mutant PD-L1 in which any one or more of the amino acid residues at positions corresponding to positions 113 (R113), 123 (Y123) and 125 (R125) in SEQ ID NO: 94 have been substituted with alanines, is reduced as compared to binding to wild type PD-L1 having the amino acid sequence set forth in SEQ ID NO: 94; reduced binding being determined as fold change in binding of said antibody being less than mean fold change in binding over all alanine mutants - 1.5xSD , wherein SD is the standard deviation of all calculated fold changes for the antibody to the mutant PDL1 and fold change in binding is calculated as set forth in Example 13 **[338].**

8. The antibody according to any of items 1-2 and 6, wherein said antibody binds to an epitope on PD-L1 (SEQ ID NO: 94), said epitope comprising the amino acid residues at position 113 (R113), the amino acid residue at position 123 (Y123) and/or the amino acid residue at position 125 (R125) of SEQ ID NO: 94.

9. The antibody according to any one of items 1, 3, 4 and 6, wherein binding of the antibody to a mutant PD-L1 in which any one or more of the amino acid residues at positions corresponding to positions 19 (F19), 42 (F42), 45 (E45), 46 (K46), 94 (L94) and 116 (I116) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13 **[511].**

10. The antibody according to any of items 1, 3, 4 and 6, wherein said antibody binds to an epitope on PD-L1 (SEQ ID NO: 94), said epitope comprising one or more amino acid residues selected from the group consisting of: the amino acid residues at position 45 (E45), the amino acid residue at position 46 (K46; and/or the amino acid residue at position 94 (L94) of SEQ ID NO: 94.

11. The antibody according to any one of items 1, 5 and 6, wherein binding of the antibody to a mutant PD-L1 in which any one or more of the amino acid residues at positions corresponding to positions 58 (E58) and 113 (R113) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13 **[547].**

12. The antibody according to any of items 1, 5 and 6, wherein said antibody binds to an epitope on PD-L1 (SEQ ID NO: 94), said epitope comprising the amino acid residue at position 58 (E58) and/or the amino acid residue at position 113 (R113) of SEQ ID NO: 94.

13. The antibody according to any one of the preceding items, wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO:11 and SEQ ID NO:21.

14. The antibody according to any one of the preceding items, wherein said antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively **[338],** or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively **[511],** or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively **[547].**

15. The antibody according to any one of the preceding items, wherein said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1, SEQ ID NO:8 and SEQ ID NO:18.

16. The antibody according to any one of the preceding items, wherein said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5, SEQ ID NO:15 and SEQ ID NO:22.

17. The antibody according to any one of the preceding items, wherein said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5 **[338],** or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15 **[511],** or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22 **[547].**

18. The antibody according to item 17, wherein said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

19. The antibody according to any one of the preceding items, wherein said antibody is capable of inducing dose-dependent lysis of epithelial cells of an adenocarcinoma, such MDA-MB-231 through antibody-dependent cell-mediated cytotoxicity (ADCC).

20. The antibody according to item 19, wherein said antibody is capable of reducing the number of cells in a culture of said epithelial cells by at least 5%, such as at least 6%, 7%, 8%, 9% or at least 10% as a result of cell lysis.

21. The antibody according to item 19 or 20, wherein ADCC is determined *in vitro* in a $^{51}$Cr release assay, such as the assay disclosed in example 14.

22. The antibody according to any one of items 19 to 20, wherein ADCC is determined *in vitro*, by incubating said epithelial cells with a composition comprising the antibody and effector cells, such as peripheral blood mononuclear cells (PBMCs), for 4 hours at 37°C, 5% $CO_2$, the amount of antibody in said composition being within the range of 0.1-1 μg/mL and the ratio of effector cells to epithelial cells being 100:1.

23. The antibody according to item 19 or 20, wherein said lysis of epithelial cells is determined *in vitro* in a luciferase reporter assay as a surrogate for ADCC, such as the luminescent ADCC reporter bioassay disclosed in example 14.

24. The antibody according to item 23, wherein ADCC is determined *in vitro,* by

i) contacting a culture of said epithelial cells with a composition comprising the antibody and Jurkat human T-cells stably expressing FcγRIIIa (CD16) and firefly luciferase (effector cells), at an effector cell:epithelial cell ratio of 1:1.
ii) adjusting the culture of the epithelial cells and effector cells to room temperature for 15 minutes,
iii) incubating the culture of the epithelial cells and effector cells with a luciferase substrate, and
iv) determining luciferase production in said cell culture;

the amount of antibody in said composition being within the range of 0.5-250 ng/mL and the ratio of effector cells to epithelial cells being 1:1.

25. The antibody according to any one of items 19, 20, 23 and 24, wherein when ADCC of said epithelial cells is determined in a luciferase reporter assay, such as a reporter assay defined in item 23 or 24, then the ADCC observed after incubation of a culture of the epithelial cells with a test composition comprising said antibody is at least 1.5 times the ADCC observed after incubation of a culture of the epithelial cells with a composition comprising reference antibody; ADCC being determined as relative luminescence units (RLU), the concentration of antibody in said test composition and in said composition comprising a reference antibody being the same and within the range of 20 to 250 ng/ml, and the reference antibody being selected from:

a) an antibody comprising the VH sequence set forth in SEQ ID NO: 74 and the VL sequence set forth in SEQ ID NO: 78; and
b) an antibody comprising the VH sequence set forth in SEQ ID NO: 81 and the VL sequence set forth in SEQ ID NO: 85.

26. The antibody according to any one of the preceding items, wherein said antigen-binding region capable of binding to human PD-L1 comprises:

(i) a VH sequence as set forth in SEQ ID NO:1 and a VL sequence as set forth in SEQ ID NO:5 **[338],** or

(ii) a VH sequence as set forth in SEQ ID NO:8 and a VL sequence as set forth in SEQ ID NO:15 **[511],** or

(iii) a VH sequence as set forth in SEQ ID NO:18 and a VL sequence as set forth in SEQ ID NO:22 **[547].**

27. An antibody comprising an antigen-binding region capable of binding to human PD-L1, wherein said antibody comprises:

(i) a heavy chain variable region (VH) comprising CDR1, CDR2, and CDR3 sequences as set forth in SEQ ID NOs: 33, 34 and 35, respectively, and a light chain variable region (VL) comprising CDR1, CDR2, and CDR3 having the sequences as set forth in SEQ ID NO:37, the sequence KAS, and the sequence as set forth in SEQ ID NO:38, respectively **[321],** or

(ii) a heavy chain variable region (VH) comprising CDR1, CDR2, and CDR3 sequences as set forth in SEQ ID NOs: 47, 48 and 49, respectively, and a light chain variable region (VL) comprising CDR1, CDR2, and CDR3 having the sequences as set forth in SEQ ID NO:51, the sequence DVI, and the sequence as set forth in SEQ ID NO:52, respectively **[421],** or

(iii) a heavy chain variable region (VH) comprising CDR1, CDR2, and CDR3 sequences as set forth in SEQ ID NOs: 54, 55 and 56, respectively, and a light chain variable region (VL) comprising CDR1, CDR2, and CDR3 having the sequences as set forth in SEQ ID NO:58, the sequence RDS, and the sequence as set forth in SEQ ID NO:59, respectively **[476],** or

(iv) a heavy chain variable region (VH) comprising CDR1, CDR2, and CDR3 sequences as set forth in SEQ ID NOs: 61, 62 and 63, respectively, and a light chain variable region (VL) comprising CDR1, CDR2, and CDR3 having the sequences as set forth in SEQ ID NO:65, the sequence DDS, and the sequence as set forth in SEQ ID NO:66, respectively **[516],** or

(v) a heavy chain variable region (VH) comprising CDR1, CDR2, and CDR3 sequences as set forth in SEQ ID NOs: 107, 108 and 109, respectively, and a light chain variable region (VL) comprising CDR1, CDR2, and CDR3 having the sequences as set forth in SEQ ID NO:111, the sequence EDS, and the sequence as set forth in SEQ ID NO:113, respectively **[625],** or

(vi) a heavy chain variable region (VH) comprising CDR1, CDR2, and CDR3 sequences as set forth in SEQ ID NOs: 68, 69 and 70, respectively, and a light chain variable region (VL) comprising CDR1, CDR2, and CDR3 having the sequences as set forth in SEQ ID NO:72, the sequence EDS, and the sequence as set forth in SEQ ID NO:73, respectively **[632].**

28. The antibody according to item 27, wherein said antibody comprises:

(i) a VH sequence as set forth in SEQ ID NO:32 and a VL sequence as set forth in SEQ ID NO:36 **[321],** or
(ii) a VH sequence as set forth in SEQ ID NO:46 and a VL sequence as set forth in SEQ ID NO:50 **[421],** or
(iii) a VH sequence as set forth in SEQ ID NO:53 and a VL sequence as set forth in SEQ ID NO:57 **[476],** or
(iv) a VH sequence as set forth in SEQ ID NO:60 and a VL sequence as set forth in SEQ ID NO:64 **[516],** or
(v) a VH sequence as set forth in SEQ ID NO:106 and a VL sequence as set forth in SEQ ID NO: 110 **[625],** or
(vi) a VH sequence as set forth in SEQ ID NO:67 and a VL sequence as set forth in SEQ ID NO:71**[632]**.

29. The antibody according to any one of the preceding items, wherein said antibody is monovalent.

30. The antibody according to any one of the preceding items, wherein said antibody is a bivalent antibody having two antigen-binding regions capable of binding to human PD-L1 and wherein said two antigen-binding regions have identical variable region sequences.

31. The antibody according to any one of the preceding items, wherein said antibody is a bivalent bispecific antibody, which, in addition to said (first) antigen-binding region capable of binding to human PD-L1, comprises a (second) antigen-binding region capable of binding to a second antigen or to a different epitope of human PD-L1, wherein said second antigen is not human CD3ε.

32. A bispecific antibody comprising an antigen-binding region capable of binding to human PD-L1 and an antigen-binding region capable of binding to human CD3ε (epsilon), wherein the antigen-binding region capable of binding to human PD-L1 has the features set forth in any one of the preceding items.

33. The bispecific antibody according to item 32, wherein the antigen-binding region capable of binding to human CD3ε comprises (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

34. The bispecific antibody according to item 32 or 33, comprising:

(i) an antigen-binding region capable of binding to human PD-L1 comprising a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7 **[338],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(ii) an antigen-binding region capable of binding to human PD-L1 comprising a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17 **[338],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iii) an antigen-binding region capable of binding to human PD-L1 comprising a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 sequences having the sequences as set forth in SEQ ID NO:23, the sequence DDN, and the sequence as set forth in SEQ ID NO:24 **[547],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

35. The bispecific antibody according to any one items 32 to 34, wherein the antigen-binding region capable of binding to human CD3ε comprises a VH sequence as set forth in SEQ ID NO:25 and a VL sequence as set forth in SEQ ID NO:29.

36. The bispecific antibody according to item 32, wherein said bispecific antibody:

(i) has a lower affinity for human CD3ε binding as compared to an antibody having an antigen-binding region capable comprising a VH sequence as set forth in SEQ ID NO:25 and a VL sequence as set forth in SEQ ID NO:29, preferably wherein said affinity is at least 2-fold lower, e.g. at least 5-fold lower, such as at least 10-fold lower, e.g. at least 25-fold lower, such as at least 50-fold lower, and
(ii) is capable of mediating concentration-dependent cytotoxicity of MDA-MB-231 cells, PC-3 cells and/or HELA cells when using PBMCs or purified T cells as effector cells, e.g. when assayed as described in Example 11 herein.

37. The bispecific antibody according to item 36, wherein the antigen-binding region capable of binding to human CD3ε comprises:

(i) a heavy chain variable region (VH) comprising CDR1, CDR2, and CDR3 having the sequences as set forth in SEQ ID NOs: 99, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(ii) a heavy chain variable region (VH) comprising CDR1, CDR2, and CDR3 having the sequences as set forth in SEQ ID NOs: 100, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iii) a heavy chain variable region (VH) comprising CDR1, CDR2, and CDR3 having the sequences as set forth in SEQ ID NOs: 26, 27, and 101, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iv) a heavy chain variable region (VH) comprising CDR1, CDR2, and CDR3 having the sequences as set forth in SEQ ID NOs: 26, 27, and 102, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(v) a heavy chain variable region (VH) comprising CDR1, CDR2, and CDR3 having the sequences as set forth in SEQ ID NOs: 26, 27, and 103, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vi) a heavy chain variable region (VH) comprising CDR1, CDR2, and CDR3 having the sequences as set forth in SEQ ID NOs: 26, 27, and 104, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vii) a heavy chain variable region (VH) comprising CDR1, CDR2, and CDR3 having the sequences as set forth in SEQ ID NOs: 26, 27, and 105, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

38. The bispecific antibody according to item 36 or 37, wherein the antigen-binding region capable of binding to human CD3ε comprises:

(i) a VH sequence as set forth in SEQ ID NO:39 and a VL sequence as set forth in SEQ ID NO:29, or
(ii) a VH sequence as set forth in SEQ ID NO:40 and a VL sequence as set forth in SEQ ID NO:29, or
(iii) a VH sequence as set forth in SEQ ID NO:41 and a VL sequence as set forth in SEQ ID NO:29, or
(iv) a VH sequence as set forth in SEQ ID NO:42 and a VL sequence as set forth in SEQ ID NO:29, or
(v) a VH sequence as set forth in SEQ ID NO:43 and a VL sequence as set forth in SEQ ID NO:29, or
(vi) a VH sequence as set forth in SEQ ID NO:44 and a VL sequence as set forth in SEQ ID NO:29, or
(vii) a VH sequence as set forth in SEQ ID NO:45 and a VL sequence as set forth in SEQ ID NO:29.

39. A multispecific antibody comprising a first antigen-binding region capable of binding to human PD-L1 and a second antigen-binding region capable of binding to a second antigen or to a different epitope of human PD-L1, wherein said antigen-binding region capable of binding to human PD-L1 has the features set forth in any one of items 1-31.

40. The multispecific antibody according to item 39, wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4 **[338]**, SEQ ID NO:11 **[511]** and SEQ ID NO:21 **[547]**.

41. The multispecific antibody according to item 40, wherein said first antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively **[338]**, or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively **[511]**, or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively **[547]**.

42. The multispecific antibody according to item 40 or 41, wherein said first antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1 **[338]**, SEQ ID NO:8 **[511]** and SEQ ID NO:18 **[547]**.

43. The multispecific antibody according to any one of items 40 to 42, wherein said first antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5 **[338],** SEQ ID NO:15 **[511]**and SEQ ID NO:22 **[547].**

44. The multispecific antibody according to any one of items 40 to 43, wherein said first antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5 **[338],** or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15 **[511],** or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22 **[547].**

45. The multispecific antibody according to any one of items 40 to 44, wherein said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

46. The multispecific antibody according to any one of items 40 to 45, wherein said first antigen-binding region capable of binding to human PD-L1 comprises:

(i) a VH sequence as set forth in SEQ ID NO: 1 and a VL sequence as set forth in SEQ ID NO:5 **[338],** or

(ii) a VH sequence as set forth in SEQ ID NO:8 and a VL sequence as set forth in SEQ ID NO: 15 **[511],** or

(iii) a VH sequence as set forth in SEQ ID NO:18 and a VL sequence as set forth in SEQ ID NO:22 **[547].**

47. A multispecific antibody comprising a first antigen-binding region capable of binding to human PD-L1 and a second antigen-binding region capable of binding to a second antigen or to a different epitope of human PD-L1, wherein said antibody:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or

(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

48. The multispecific antibody according to item 47, wherein said antibody competes for binding to human PD-L1 with an antibody comprising a VH sequence as set forth in SEQ ID NO: 1 and a VL sequence as set forth in SEQ ID NO:5.

49. A multispecific antibody comprising a first antigen-binding region capable of binding to human PD-L1 and a second antigen-binding region capable of binding to a second antigen or to a different epitope of human PD-L1, wherein the binding of said antibody to human PD-L1 is not displaced by an antibody comprising a VH sequence as set forth in SEQ ID NO:53 and a VL sequence as set forth in SEQ ID NO:57.

50. The multispecific antibody according to item 49, wherein the antibody inhibits the binding of human PD-L1 to human PD-1.

51. The multispecific antibody according to item 49 or 50, wherein the antibody competes for binding to human PD-L1 with an antibody comprising a VH sequence as set forth in SEQ ID NO:18 and a VL sequence as set forth in SEQ ID NO:22.

52. The mulitspecific antibody according to any of items 49 to 51, wherein the binding of said antibody to human PD-L1 is blocked by an antibody comprising a VH sequence as set forth in SEQ ID NO:18 and a VL sequence as set forth in SEQ ID NO:22.

53. A multispecific antibody comprising a first antigen-binding region capable of binding to human PD-L1 and a second antigen-binding region capable of binding to a second antigen or to a different epitope of human PD-L1, wherein said first antigen-binding region:

(i) is capable of binding to the same epitope of human PD-L1 as an antibody comprising a VH sequence as set forth in SEQ ID NO:1 and a VL sequence as set forth in SEQ ID NO:5 **[338],** or

(ii) is capable of binding to the same epitope of human PD-L1 as an antibody comprising a VH sequence as set forth in SEQ ID NO:8 and a VL sequence as set forth in SEQ ID NO:15 **[511],** or

(iii) is capable of binding to the same epitope of human PD-L1 as an antibody comprising a VH sequence as set forth in SEQ ID NO:18 and a VL sequence as set forth in SEQ ID NO:22 **[547].**

54. The multispecific antibody according to any one of items 40 to 53, wherein the antibody is bispecific.

55. The multispecific antibody according to item 54, wherein the antbody is bivalent.

56. The multispecific antibody according to any one of items 40 to 55, wherein the antibody is capable of binding a second antigen and said second antigen is not human CD3ε.

57. The antibody according to any one of the preceding items, wherein the antibody is a full-length antibody.

58. The antibody according to item 57, wherein the antibody is a full-length IgG1 antibody.

59. The antibody according to any one of the preceding items, wherein the antibody is an antibody fragment.

60. The antibody according to any one of items 32 to 59, wherein the antibody comprises two half-molecules each comprising an antigen-binding region, wherein

(i) the half-molecule(s) comprising the antigen-binding region capable of binding to human PD-L1 is/are chimeric, and/or

(ii) the half-molecule comprising the antigen-binding region capable of binding to human CD3ε (epsilon), if present, is chimeric.

61. The antibody according to any one of the preceding items, wherein

(i) the antigen-binding region(s) capable of binding to human PD-L1 is/are humanized, and/or

(ii) the antigen-binding region capable of binding to human CD3ε (epsilon), if present, is humanized.

62. The antibody according to any one of the preceding items, wherein

(i) the antigen-binding region(s) capable of binding to human PD-L1 is/are human, and/or

(ii) the antigen-binding region capable of binding to human CD3ε (epsilon), if present, is human.

63. The antibody according to any one of the preceding items, wherein each of the antigen-binding regions comprises a heavy chain variable region (VH) and a light chain variable region (VL), and wherein said variable regions each comprise three CDR sequences, CDR1, CDR2 and CDR3, respectively, and four framework sequences, FR1, FR2, FR3 and FR4, respectively.

64. The antibody according to item 63, wherein the antibody comprises two heavy chain constant regions (CH), and two chain constant regions (CL).

65. The antibody according to any one of the preceding items, wherein the antibody comprises a first and second heavy chain, wherein each of said first and second heavy chains comprises at least a hinge region, a CH2 and a CH3 region, wherein in said first heavy chain at least one of the amino acids in a position corresponding to a position selected from the group consisting of T366, L368, K370, D399, F405, Y407, and K409 (according to EU numbering) has been substituted, and in said second heavy chain at least one of the amino acids in a position corresponding to a position selected from the group consisting of T366, L368, K370, D399, F405, Y407, and K409 (according to EU numbering) has been substituted, and wherein said first and said second heavy chains are not substituted in the same positions.

66. The antibody according to item 65, wherein (i) the amino acid in the position corresponding to F405 (according to EU numbering) is L in said first heavy chain, and the amino acid in the position corresponding to K409 (according to EU numbering) is R in said second heavy chain, or (ii) the amino acid in the position corresponding to K409 (according to EU numbering) is R in said first heavy chain, and the amino acid in the position corresponding to F405 (according to EU numbering) is L in said second heavy chain.

67. The antibody according to any one of the preceding items, wherein said antibody comprises a first and a second heavy chain and wherein one or both heavy chains are modified so that the antibody induces Fc-mediated effector function to a lesser extent relative to an antibody which is identical, except for comprising non-modified first and second heavy chains.

68. The antibody according to item 67, wherein said Fc-mediated effector function is measured by determining Fc-mediated CD69 expression, by binding to Fcγ receptors, by binding to C1q, or by induction of Fc-mediated cross-linking of FcRs.

69. The antibody according to item 67 or 68, wherein the heavy and light chain constant sequences have been modified so that said antibody reduces Fc-mediated CD69 expression by at least 50%, at least 60%, at least 70%, at least 80%, at least 90%, at least 99% or 100% when compared to a wild-type antibody wherein said Fc-mediated CD69 expression is determined in a PBMC-based functional assay.

70. The antibody according to any one of the preceding items, wherein said antibody comprises a first and a second heavy chain, wherein in at least one of said first and second heavy chains one or more amino acids in the positions corresponding to positions L234, L235, D265, N297, and P331 in a human IgG1 heavy chain according to EU numbering, are not L, L, D, N, and P, respectively.

71. The antibody according to item 70, wherein the positions corresponding to positions L234 and L235 in a human IgG1 heavy chain according to EU numbering are F and E, respectively, in said first and second heavy chains.

72. The antibody according to item 71, wherein the antibody is a bispecific antibody comprising a first and second heavy chain and wherein the positions corresponding to positions L234 and L235 in a human IgG1 heavy chain according to EU numbering of both the first heavy chain and the second heavy chain are F and E, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

73. The antibody according to item 70, wherein the positions corresponding to positions L234, L235, and D265 in a human IgG1 heavy chain according to EU numbering are F, E, and A, respectively, in said first and second heavy chains.

74. The antibody according to item 73, wherein the antibody is a bispecific antibody comprising a first and second heavy chain and wherein the positions corresponding to positions L234, L235, and D265 in a human IgG1 heavy chain according to EU numbering of both the first heavy chain and the second heavy chain are F, E, and A, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

75. The antibody according to any one of the preceding items, wherein the antibody does not bind to human PD-L2.

76. The antibody according to any one of the preceding items, wherein the antibody binds human PD-L1 with a $K_D$ of about $10^{-8}$ M or less, such as about $10^{-9}$ M or less, e.g. about $10^{-10}$ M or less, when determined as described in Example 8 herein.

77. The antibody according to any one of the preceding items, wherein the antibody mediates concentration-dependent cytotoxicity of MDA-MB-231 cells, PC-3 cells and/or HELA cells when using purified T cells as effector cells, when assayed as described in Example 11 herein.

78. A nucleic acid construct comprising:

(i) a nucleic acid sequence encoding a heavy chain sequence of an antibody comprising an antigen-binding region capable of binding to human PD-L1 as defined in any one of items 1 to 31, and/or

(ii) a nucleic acid sequence encoding a light chain sequence of an antibody comprising an antigen-binding region capable of binding to human PD-L1 as defined in any one of items 1 to 31.

79. The nucleic acid construct according to item 73, further comprising

(i) a nucleic acid sequence encoding a heavy chain sequence of an antibody comprising an antigen-binding region capable of binding to human CD3ε as defined in any one of items 33 to 38, and

(ii) a nucleic acid sequence encoding a light chain sequence of an antibody comprising an antigen-binding region capable of binding to human CD3ε as defined in any one of items 33 to 38.

80. An expression vector comprising a nucleic acid construct as defined in item 78 or 79.

81. A host cell comprising a nucleic acid construct as defined in item 78 or 79 or an expression vector as defined in item 80.

82. The host cell according to item 81, wherein said host cell is a mammalian cell, such as a Chinese hamster ovary cell.

83. A pharmaceutical composition comprising an antibody according to any one of items 1 to 77 and a pharmaceutically-acceptable carrier.

84. The antibody according to any one of items 1 to 77 or the pharmaceutical composition according to item 83 for use as a medicament.

85. The antibody according to any one of items 1 to 80 or the pharmaceutical composition according to item 70 for use in the treatment of cancer.

86. The antibody according to any one of items 1 to 77 or the pharmaceutical composition according to item 70 for use in the treatment of a cancer disease characterised by the presence of solid tumors.

87. The antibody according to any one of items 1 to 78 or the pharmaceutical composition according to item 70 for use in the treatment of a cancer disease selected from the group consisting of: melanoma, ovarian cancer, lung cancer, colon cancer and head and neck cancer.

88. A method of treatment of a disease comprising administering an antibody according to any one of items 1 to 75 or the pharmaceutical composition according to item 83 to a subject in need thereof.

89. Use of an antibody according to any one of items 1 to 77 for the manufacture of a medicament, such as a medicament for the treatment of cancer, e.g. a cancer disease characterized by the presence of solid tumors or a cancer disease selected from the group consisting of: melanoma, ovarian cancer, lung cancer, colon cancer and head and neck cancer.

90. The method or use according to any of items 83 to 89 wherein the method or use comprises combination with one or more further therapeutic agent, such as a chemotherapeutic agent.

91. A method for producing an antibody according to any one of items 1 to 77, comprising the steps of:

a) culturing a host cell producing a first antibody comprising an antigen-binding region capable of binding to human PD-L1 as defined in any one of items 1 to 13 and purifying said first antibody from the culture;
b) culturing a host cell producing a second antibody comprising an antigen-binding region capable of binding to a different epitope of PD-L1 or a different antigen, e.g. a human CD3ε-binding region as defined in any one of items 14 to 19, and purifying said second antibody from the culture;
c) incubating said first antibody together with said second antibody under reducing conditions sufficient to allow the cysteines in the hinge region to undergo disulfide-bond isomerization, and
d) obtaining said bispecific antibody.

92. An anti-idiotypic antibody which binds to the antigen-binding region capable of binding to human PD-L1 as defined in any one of items 1 to 77.

The present invention is further illustrated by the following examples, which should not be construed as limiting the scope of the invention.

**EXAMPLES**

**Example 1: Generation of PD-L1 antibodies**

Immunization of OmniRat animals and hybridoma generation

Immunization and hybridoma generation was performed at Aldevron GmbH (Freiburg, Germany). A cDNA encoding amino acid 19-238 of human PD-L1 was cloned into Aldevron proprietary expression plasmids. Groups of OmniRat animals (transgenic rats expressing a diversified repertoire of antibodies with fully human idiotypes; Ligand Pharmaceuticals Inc., San Diego, USA) were immunized by intradermal application of DNA-coated gold-particles using a hand-held device for particle-bombardment ("gene gun"). Cell surface expression on transiently transfected HEK cells was confirmed with an anti-PD-L1 antibody based on MPDL3280A from Genentech. Serum samples were collected after a series of immunizations and tested in flow cytometry on HEK cells transiently transfected with the aforementioned expression plasmids. Antibody-producing cells were isolated and fused with mouse myeloma cells (Ag8) according to standard procedures. Hybridomas producing antibodies specific for PD-L1 were identified by screening in the same assay as described above. Cell pellets of positive hybridomas cells were prepared using an RNA protection agent (RNAlater, ThermoFisher Scientific, cat. no. AM7020) and further processed for sequencing of the variable domains of the antibodies.

Sequence analysis of the PD-L1 antibody variable domains and cloning in expression vectors

Total RNA was prepared from 0.2 to $5\times10^6$ hybridoma cells and 5'-RACE-Complementary DNA (cDNA) was prepared from total RNA, using the SMART RACE cDNA Amplification kit (Clontech), according to the manufacturer's instructions. VH and VL coding regions were amplified by PCR and cloned directly, in frame, in pOMTG1f-FEAR-LIC (human IgG1) and pEFC33D-Kappa (human Kappa) or pOMTL-LIC (human Lambda) expression vectors, by ligation independent cloning (Aslanidis, C. and P.J. de Jong, Nucleic Acids Res 1990;18(20): 6069-74). In these plasmids, antibody sequences are expressed using a CMV promoter. For each antibody, 8 VL clones and 8 VH clones were sequenced. CDR sequences were defined according to IMGT definitions [Lefranc MP. et al., Nucleic Acids Research, 27, 209-212, 1999; Brochet X. Nucl. Acids Res. 36, W503-508 (2008)]. Clones with a correct Open Reading Frame (ORF) were selected for further study and expression. LEE PCR products of all combinations of heavy chains and light chains that were found per hybridoma culture were transiently co-expressed in Expi293F cells using ExpiFectamine. Per hybridoma, the HC/LC pair that showed the best binding in a homogeneous dose-response screen was selected as lead candidate.

Three PD-L1 antibodies, numbered 338, 511 and 547, respectively were selected for further experimentation. Their variable region sequences are shown in the Sequence Listing herein.

For antibody IgG1-PDL1-511-FEAR a variant with point mutation in the variable domains was generated in order to remove a cysteine residue which potentially could generate undesired disulphide bridges: IgG1-PDL1-511-FEAR-LC33S. This mutant was generated by gene synthesis (Geneart).

LEE PCR

Linear expression elements (LEE's) were produced by amplifying the fragment containing the CMV promoter, HC or LC encoding regions and the poly A signal containing elements from the expression plasmids. For this the regions were amplified using Accuprime Taq DNA polymerase (Life Technologies) and the primers CMVPf(BsaI)2 and TkpA(BsaI) r, performing 35 cycles of 45 seconds at 94 °C, 30 seconds at 55 °C and 2 (LC) or 3 (HC) minutes at 68 °C, using 50x diluted plasmid miniprep material, as a DNA template.

Transient expression of LEE fragments in Expi293F cells

For LEE expression of Abs, 1.11 $\mu$l of the HC LEE PCR reaction mixture and 1.11 $\mu$l of the LC PCR reaction mixture were mixed and transfected in Expi293F cells in a total volume of 125 $\mu$l using ExpiFectamin 293 as transfection reagent, according to the instructions of the manufacturer (Thermo Fisher Scientific, USA), using 96 well plates as vessel.

Expression of antibodies

Antibodies were expressed as IgG1,Kappa (for 338) or IgG1,Lambda (for 511 and 547). Plasmid DNA mixtures encoding both heavy and light chains of antibodies were transiently expressed using the Expi293F expression platform (Thermo Fisher Scientific, USA) essentially as described by the manufacturer.

Homogeneous binding assay

Antibodies were tested for binding in a homogeneous dose-response screen using CHO cells transfected with PDL1, PDL1mm or PDL1Mf (see also Example 2). Non-transfected CHO cells were used as negative control.

Cells ($2.5 \times 10^5$ cells/ml) were mixed with goat anti-human IgG Alexa 647, Fc$\gamma$ fragment specific (0.2 $\mu$g/ml; Jackson ImmunoResearch Laboratories, 109-605-098). Serial dilutions of test and control antibodies (range 0.001 to 3 $\mu$g/mL in 2-fold dilution steps) were prepared and 2 $\mu$l antibody dilution was added to 5 $\mu$l of the cell/conjugate mixture in 1536 well plates (Greiner, 789866). Plates were incubated at room temperature for 9 hours, and after which fluorescence intensity was determined using an ImageXpress Velos Laser Scanning Cytometer (Molecular Devices).

Purification of antibodies

Culture supernatant was filtered over 0.2 $\mu$m dead-end filters, loaded on 5 mL MabSelect SuRe columns (GE Healthcare) and eluted with 0.1 M sodium citrate-NaOH, pH 3. The eluate was immediately neutralized with 2M Tris-HCl, pH 9 and dialyzed overnight to 8.7 mM $Na_2HPO_4$, 1.8 mM $NaH_2PO_4$ 140.3 mM NaCl, pH 7.4 (B.Braun or GE Healthcare). Alternatively, subsequent to purification, the eluate was loaded on a HiPrep Desalting column and the antibody was exchanged into 8.7 mM $Na_2HPO_4$, 1.8 mM $NaH_2PO_4$ 140.3 mM NaCl, pH 7.4 (B.Braun or GE Healthcare) buffer. After dialysis or exchange of buffer, samples were sterile filtered over 0.2 $\mu$m dead-end filters. Purity was determined by CE-SDS using a LabChip GXII (Caliper Life Sciences, MA) and IgG concentration was measured using Nanodrop ND-1000 spectrophotometer (Isogen Life Science, Maarssen, The Netherlands). Purified antibodies were stored at 4°C.

**Example 2: Generation of screenings material**

Expression constructs for PD-L1

The following codon-optimized constructs for expression of full-length PD-L1 were generated: human (Homo sapiens) PD-L1 (Genbank accession no. NP_054862), cynomolgus monkey (Macaca fascicularis) PD-L1 (Genbank accession no. XP_005581836), mouse (Mus musculus) PD-L1 (Genbank accession no. NP_068693).

In addition, the following codon-optimized construct for the PD-L1 ECD was generated: the extracellular domain (ECD) of human PD-L1 (aa 1-238) with a C-terminal His-tag and C-tag (PDLoneECDHisCtag).

The constructs contained suitable restriction sites for cloning and an optimal Kozak (GCCGCCACC) sequence [Kozak et al. (1999) Gene 234: 187-208]. The constructs were cloned in the mammalian expression vector pMA (Geneart).

## Expression construct for PD-L2

Similarly, following codon-optimized construct for expression of full-length human PD-L2 was generated: human (Homo sapiens) PD-L2 (Genbank accession no. NP_079515)

## Expression in CHO-S cells

CHO-S cells were transiently transfected with the pMA vector containing coding sequence for the full human PD-L1, the full cynomolgus monkey and the full mouse respectively.

## Purification of His-tagged PD-L1

PDLoneECDHisCtag was expressed in HEK-293F cells. The His-tag enables purification with immobilized metal affinity chromatography. In this process, a chelator fixed onto the chromatographic resin is charged with $Co^{2+}$ cations. His-tagged protein containing supernatants were incubated with the resin in batch mode (i.e. solution). The His-tagged protein binds strongly to the resin beads, while other proteins present in the culture supernatant do not bind or bind weakly compared to the His-tagged proteins. After incubation, the beads are retrieved from the supernatant and packed into a column. The column is washed to remove weakly bound proteins. The strongly bound His-tagged proteins are then eluted with a buffer containing imidazole, which competes with the binding of His to $Co^{2+}$. The eluent is removed from the protein by buffer exchange on a desalting column.

## Example 3: Humanized CD3 antibody for the generation of CD3xPDL1 bispecific antibodies

The generation of humanized antibody IgG1-huCD3-H1L1 is described in Example 1 of WO2015001085. Antibody huCD3-H1L1-FEAL is a variant hereof having the following substitutions: L234F, L235E, D265A and F405L, as described herein above.

## Example 4: Generation of bispecific antibodies by 2-MEA-induced Fab-arm exchange

Bispecific IgG1 antibodies were generated by Fab-arm-exchange under controlled reducing conditions. The basis for this method is the use of complementary CH3 domains, which promote the formation of heterodimers under specific assay conditions as described in WO2011/131746. The F405L and K409R (EU numbering) mutations were introduced into the relevant antibodies to create antibody pairs with complementary CH3 domains.

To generate bispecific antibodies, the two parental complementary antibodies, each antibody at a final concentration of 0.5 mg/mL, were incubated with 75 mM 2-mercaptoethylamine-HCl (2-MEA) in a total volume of 100 $\mu$L TE at 31°C for 5 hours. The reduction reaction was stopped by removing the reducing agent 2-MEA using spin columns (Microcon centrifugal filters, 30k, Millipore) according to the manufacturer's protocol.

The following antibodies were used in the examples:

## CD3 antibodies

IgG1-huCD3-H1L1-FEAL (having the VH and VL sequences set forth in SEQ ID NO:25 and SEQ ID NO:29, respectively)
bsIgG1-huCD3-HILI-FEALxb12-FEAR is a bispecific antibody using as the second arm the antibody b12 which is a gp120 specific antibody (Barbas, CF. J Mol Biol. 1993 Apr 5;230(3):812-23).

## PDL1 antibodies and CD3xPDLlbispecific antibodies

IgG1-338-FEAR (having the VH and VL sequences set forth in SEQ ID NO:1 and SEQ ID NO:5, respectively)

IgG1-338-F405L

bsIgG1- huCD3-H1L1-FEALx338-FEAR

bsIgG1-b12-FEALx338-FEAR

IgG1-511-LC33S-FEAR (having the VH and VL sequences set forth in SEQ ID NO:8 and SEQ ID NO:15, respectively)

IgG1-511-F405L-LC33S

bsIgG1- huCD3-H1L1-FEALx511-LC33S-FEAR

bsIgG1-b12-FEALx511-LC33S-FEAR

IgG1-547-FEAR (having the VH and VL sequences set forth in SEQ ID NO:18 and SEQ ID NO:22, respectively)

IgG1-547-F405L

bsIgG1- huCD3-H1L1-FEALx547-FEAR

bsIgG1-b12-FEALx547-FEAR

IgG1-321-FEAR (having the VH and VL sequences set forth in SEQ ID NO:32 and SEQ ID NO:36, respectively)

IgG1-421-LC91S-FEAR (having the VH and VL sequences set forth in SEQ ID NO:46 and SEQ ID NO:50, respectively)

IgG1-476-N101Q-LC33S-FEAR (having the VH and VL sequences set forth in SEQ ID NO:53 and SEQ ID NO:57, respectively)

IgG1-625-FEAR (having the VH and VL sequences set forth in SEQ ID NO:106and SEQ ID NO:110, respectively)

IgG1-632-FEAR (having the VH and VL sequences set forth in SEQ ID NO:67 and SEQ ID NO:71, respectively)

IgG1-516-FEAR (having the VH and VL sequences set forth in SEQ ID NO:60 and SEQ ID NO:64, respectively)

IgG1-MPDL3280A-FEAR (based on PDL1 antibody MPDL3280A from Genentech; having the VH and VL sequences set forth in SEQ ID NO:74 and SEQ ID NO:78, respectively) IgG1-MPDL3280A-K409R

IgG1-MEDI4736-FEAR (based on PDL1 antibody MEDI4736 from MedImmune; having the VH and VL sequences set forth in SEQ ID NO:81 and SEQ ID NO:85, respectively) IgG1-MEDI4736-F405L

**Example 5: Binding of PD-L1 antibodies or CD3xPD-L1 or b12xPD-L1 bispecific antibodies to tumor cells**

Binding of PD-L1 antibodies and CD3xPD-L1 and b12xPD-L1 bispecific antibodies to the human tumor cell lines SK-MES-1 (lung squamous cell carcinoma; ATCC; Cat. no. HTB-58) MDA-MB-231 (breast adenocarcinoma; ATCC; Cat. no. HTB-26), PC-3 (prostate adenocarcinoma; ATCC; Cat. no. CRL-1435) and HELA (cervix adenocarcinoma; ATCC; Cat. no. CCL-2) was analyzed by flow cytometry.

Cells ($3\text{-}5 \times 10^4$ cells/well) were incubated in polystyrene 96-well round-bottom plates (Greiner bio-one, cat. no. 650101) with serial dilutions of antibodies (range 0.0001 to 10 $\mu$g/mL in 5-fold dilution steps) in 50 $\mu$L PBS/0.1% BSA/0.02% azide (staining buffer) at 4°C for 30 min.

After washing twice in staining buffer, cells were incubated in 50 $\mu$L secondary antibody at 4°C for 30 min. As a secondary antibody, R-Phycoerythrin (PE)-conjugated goat-anti-human IgG F(ab')$_2$ (Cat. no. 109-116-098, Jackson ImmunoResearch Laboratories, Inc., West Grove, PA) diluted 1:500 in staining buffer, was used for all experiments. Next, cells were washed twice in staining buffer, re-suspended in 20 $\mu$L staining buffer and analyzed on an iQue screener (Intellicyt Corporation, USA). Binding curves were analyzed using non-linear regression (sigmoidal dose-response with variable slope) using GraphPad Prism V75.04 software (GraphPad Software, San Diego, CA, USA).

Quantitative flow cytometry (QIFIKIT®, Dako; cat. no K0078) was performed as described (Poncelet and Carayon, 1985, J. Immunol. Meth. 85: 65-74), to quantify target expression on the plasma membrane of MDA-MB-231, PC-3 and HELA cells, and to determine the number of bound PDL1 molecules. It was determined that the cells lines have the following PD-L1 antigenic density (ABC, antibody binding capacity):

- SK-MES-1: appr. 30,000 ABC/cell
- MDA-MB-231: appr. 21,000 ABC/cell
- PC-3: appr. 6,000 ABC/cell
- HELA cells: appr. 2,000 ABC/cell.

Binding to MDA-MB-231 cells

Figure 1 shows that bsIgG1-huCD3-H1L1-FEALx338-FEAR (A), bsIgG1-b12-FEALx338-FEAR (D), bsIgG1-huCD3-H1L1-FEALx547-FEAR (B) and bsIgG1-b12-FEALx547-FEAR (E) showed dose-dependent binding to MDA-MB-231 cells, with higher maximum binding than monospecific, bivalent PD-L1 antibodies IgG1-338-FEAR and IgG1-547-FEAR. Maximum binding of bsIgG1-huCD3-H1L1-FEALx511-LC33S-FEAR (C) and bsIgG1-b12-FEALx511-LC33S-FEAR (F) was lower than bivalent, monospecific PD-L1 antibody IgG1-511-LC33S-FEAR.

Binding to PC-3 cells

Figure 2 shows that bsIgG1-huCD3-H1L1-FEALx338-FEAR (A), bsIgG1-b12-FEALx338-FEAR (D), bsIgG1-huCD3-H1L1-FEALx547-FEAR (B) and bsIgG1-b12-FEALx547-FEAR (E) showed dose-dependent binding to PC3 cells.. Maximum binding of bsIgG1-huCD3-H1L1-FEALx511-LC33S-FEAR (C) and bsIgG1-b12-FEALx511-LC33S-FEAR (F) was lower than bivalent, monospecific PD-L1 antibody IgG1-511-LC33S-FEAR.

Binding to HELA cells

Figure 3 shows that bsIgG1-huCD3-H1L1-FEALx338-FEAR (A) and bsIgG1-huCD3-H1L1-FEALx547-FEAR (B) showed dose-dependent binding to HELA cells. Maximum binding of monospecific, bivalent PD-L1 antibodies IgG1-338-FEAR and IgG1-547-FEAR could not be determined in the concentration range used. (C) bsIgG1-huCD3-H1L1-FEALx511-LC33S-FEAR and IgG1-511-LC33S-FEAR did not bind to HELA cells.

Binding to SK-MES-1 cells

Figure 4 shows that bsIgG1-b12-FEALx338-FEAR (A), and bsIgG1-b12-FEALx547-FEAR (B) showed dose-dependent binding to SK-MES-1 cells, with higher maximum binding than monospecific, bivalent PD-L1 antibodies IgG1-338-FEAR and IgG1-547-FEAR. Maximum binding of bsIgG1-b12-FEALx511-LC33S-FEAR (C) was lower than bivalent, monospecific PD-L1 antibody IgG1-511-LC33S-FEAR .

**Example 6: Binding to human PD-L2**

To show specific binding to PD-L1 and not to human PD-L2, binding of bsIgG1-huCD3-H1L1-FEALx338-FEAR and IgG1-338-FEAR, bsIgG1-huCD3-H1L1-FEALx547-FEAR and IgG1-547-FEAR and of bsIgG1-huCD3-H1L1-FEALx511-LC33S-FEAR and IgG1-511-LC33S-FEAR to CHO cells expressing human PD-L2 was determined by flow cytometry using a method as described above. A PE-conjugated PD-L2 specific antibody (Mylteni, clone MIH18; cat. no. 130-098-651) was used as positive control. None of the antibodies tested bound to CHO-PD-L2 cells.

**Example 7: Binding of PD-L1 antibodies or CD3xPD-L1 or b12xPD-L1 bispecific antibodies to cynomolgus PD-L1**

Binding to CHO cells expressing cynomolgus PD-L1 was determined by flow cytometry using a method as described above. Figure 5 shows that bsIgG1-huCD3-H1L1-FEALx338-FEAR (A), bsIgG1-huCD3-H1L1-FEALx547-FEAR (B), bsIgG1-huCD3-H1L1-FEALx511-LC33S-FEAR (C), bsIgG1-b12-FEALx338-FEAR (D), bsIgG1-b12-FEALx547-FEAR (E), and bsIgG1-b12-FEALx511-LC33S-FEAR (F), showed dose-dependent binding to CHO cells expressing cynomolgus PD-L1, with higher maximum binding than monospecific, bivalent PD-L1 antibodies IgG1-338-FEAR, IgG1-547-FEAR and IgG1-511-LC33S-FEAR.

**Example 8: Human and cynomolgus PD-L1 affinity determination using Bio-layer interferometry**

In a first set of experiments, affinities for recombinant-expressed human PD-L1 protein were determined using Bio-layer interferometry (BLI) on an Octet HTX instrument (ForteBio). Anti-Human IgG Fc Capture (AHC) biosensors (ForteBio) were loaded for 900 s with antibodies (1 $\mu$g/ml). After a baseline (100 s), the association (1000 s) and dissociation (2000 s) of PDLoneECDHisCtag in Sample Diluent (ForteBio) was determined, using a concentration range of 2.67 $\mu$g/ml-0.14 $\mu$g/ml (100 nM-1.56 nM) with 2-fold dilution steps. The experiment was carried out while shaking at 1000 rpm at 30°C. Data was analyzed with Data Analysis Software v9.0.0.12 (ForteBio) using the 1:1 model and a global full fit with 1000 s association time and 200 or 1000 s dissociation time. Data traces were corrected by subtraction of a buffer reference, the Y-axis was aligned to the last 10 s of the baseline, and interstep correction as well as Savitzky-Golay filtering was applied. Data traces with a response < 0.05 nm were excluded from analysis. As default, the fit using 1000 s dissociation time was used. A dissociation time of 200 s was used for IgG1-511-FEAR-LC33S, based on the $R^2$ value and visual inspection of the fit. Table 1 shows the results.

Table 1: Binding affinities of monospecific bivalent PD-L1 antibodies for human PD-L1 as determined by Bio-layer interferometry.

| Antibody | On-rate $k_a$ (1/Ms) | Off-rate $k_d$ (1/s) | $K_D$ (M) |
|---|---|---|---|
| IgG1-338-FEAR | 7.3E+05 | 6.4E-05 | 8.8E-11 |

(continued)

| Antibody | On-rate $k_a$ (1/Ms) | Off-rate $k_d$ (1/s) | $K_D$ (M) |
|---|---|---|---|
| IgG1-547-FEAR | 1.2E+06 | 3.6E-05 | 2.9E-11 |
| IgG1-511-LC33S-FEAR | 1.2E+06 | 4.5E-03 | 3.8E-09 |

In a second set of experiments (n=3), the affinities of the antibodies of PD-L1 antibodies to human and cynomolgus PD-L1, determined using BLI, were compared. The experimental set-up was as described above, with the exception of

- Loading time of AHC biosensors with antibodies was 600 s;
- Baseline was 300 s;
- In addition to PDLoneECD-HisCtag (human PD-L1, theoretical molecular weight 29 kDa), cynomolgus PD-L1/B7-H1 protein from (Acro Biosystems, cat. No. PD1-C52H4-100, theoretical molecular weight 27.1 kDa) was used as antigen;
- Concentration range of the antigen was 0.156 -10 nM (in first experiment) or 0.39 - 25 nM (in second and third experiment).

Table 2 shows the results (average of 3 experiments). Binding affinities of IgG1-338-FEAR, IgG1-547-FEAR and IgG1-511-LC33S-FEAR for human PD-L1 were in the same range as shown in Table 1, with deviation likely due to variations in assay conditions. Binding affinities of these antibodies for cynomolgus PD-L1 were very similar to those for human PD-L1. Binding affinities of IgG1-MEDI4738-FEAR and IgG1-MPDL3280A-FEAR were also determined. IgG1-MEDI4738-FEAR showed similar binding affinity for human and cynomolgus PD-L1. IgG1-MEDI4738-FEAR showed a large difference in binding affinity, with on average a 19.7 lower affinity (higher $K_D$) for cynomolgus PD-L1 than for human PD-L1.

Table 2: Binding affinities (average of 3 independent experiments) of monospecific bivalent PD-L1 antibodies for human and cynomolgus PD-L1 as determined by Bio-layer interferometry.

| Anti body | PD-L1 species | On-rate $k_a$ (1/Ms) | Off-rate $k_d$ (1/s) | $K_D$ (M) | Fold difference of $K_D$ |
|---|---|---|---|---|---|
| IgG1-338-FEAR | Human | 8.0E+05 | 2.5E-04 | 3.1E-10 | 2.3 |
| | Cynomolgus | 3.9E+05 | 2.8E-04 | 7.3E-10 | |
| IgG1-547-FEAR | Human | 9.7E+05 | 1.1E-04 | 1.2E-10 | 2.3 |
| | Cynomolgus | 4.4E+05 | 1.2E-04 | 2.7E-10 | |
| IgG1-511-LC33S-FEAR | Human | 9.6+05 | 4.8E-03 | 5. 1E-09 | 2.3 |
| | Cynomolgus | 4.5+E05 | 5.2E-03 | 1.2E-08 | |
| IgG1-MEDI4738-FEAR | Human | 8.8E+05 | 3.3E-04 | 3.7E-10 | 2.3 |
| | Cynomolgus | 4.3E+05 | 3.8E-04 | 8.6E-10 | |
| IgG1-MPDL3280A-FEAR | Human | 7.2E+05 | 3.3E-04 | 4.6E-10 | 19.7 |
| | Cynomolgus | 3.6E+05 | 3.0E-03 | 9.0E-09 | |

**Example 9: PD-L1 classical sandwich cross-block assay**

Antibody cross-block testing was performed using biolayer interferometry (BLI) on an Octet HTX instrument (ForteBio). Antibodies (20 μg/ml in 10 mM sodium acetate buffer pH 6.0 (ForteBio)) were immobilized on Amine-Reactive 2nd Generation (AR2G) biosensors (ForteBio) according to the instructions of the manufacturer. After a baseline (50 s) in Sample Diluent (ForteBio), biosensors containing immobilized antibodies were loaded for 500 s with PDLoneECDHisCtag (100 nM or 2.7 μg/ml), after which the association response of a second antibody (10 μg/ml) was followed for 500 s. Biosensors were regenerated by using 3 times 5 s alternating exposures to 10 mM glycine pH 2.5 and Sample Diluent. The experiment was repeated with a new set of second antibodies starting from the baseline step. Each biosensor was used 6 times. The experiment was performed at 30°C using a shaker speed of 1000 rpm. Data was analyzed using Data Analysis Software v9.0.0.12 (ForteBio). The Y-axis was aligned to the association step and Savitzky-Golay filtering was applied.

The average buffer response was subtracted from the association response of the second antibody in order to correct for the dissociation of PDLoneECDHisCtag from the immobilized antibody. The corrected association responses were plotted in a matrix format. Responses ≥ 0.1 nm were considered non-blocking antibody pairs (results indicated as plain numbers in the table in Figure 6), responses below 0.1 were considered to be blocking antibody pairs (results indicated as bold numbers in the table in Figure 6). Some antibody pairs showed displacing behavior (indicated by asterisk (*) in the table in Figure 6). Representative graphs are shown in figure (A) for displacing, (B), blocking and (C) non-blocking antibody pairs.

Cross-block experiments were performed for antibodies IgG1-338-FEAR, IgG1-547-FEAR, IgG1-511-LC33S-FEAR, IgG1-321-FEAR, IgG1-421-LC91S-FEAR, IgG1-476-N101Q-LC33S-FEAR, IgG1-632-FEAR, IgG1-516-FEAR, IgG1-MPDL3280A-FEAR and IgG1-MEDI4736-FEAR. The results are summarized in figure 6.

The data show that antibody IgG1-511-LC33S-FEAR defines a unique cross-block group, because it blocks IgG1-321-FEAR, IgG1-338-FEAR, IgG1-476-N101Q-LC33S-FEAR, IgG1-632-FEAR, IgG1-MPDL3280A-FEAR and IgG1-MEDI4736-FEAR, but it does not block IgG1-547-FEAR, IgG1-421-LC91S-FEAR or IgG1-516-FEAR binding to human PDL1.

Furthermore, antibody IgG1-547-FEAR defines a unique cross-block group, because it blocks IgG1-321-FEAR, IgG1-338-FEAR, IgG1-421-LC91S-FEAR, IgG1-MPDL3280A-FEAR and IgG1-MEDI4736-FEAR, but it does not block IgG1-511-LC33S-FEAR, IgG1-476-N101Q-LC33S-FEAR, IgG1-632-FEAR, IgG1-516-FEAR from binding to human PD1.

Moreover, antibody IgG1-476-N101Q-LC33S-FEAR showed displacing behavior in combination with IgG1-321-FEAR, IgG1-338-FEAR, IgG1-MPDL3280A-FEAR and IgG1-MEDI4736-FEAR, indicating that antibodies IgG1-321-FEAR, IgG1-338-FEAR, IgG1-MEDI4736-FEAR and IgG1-MPDL3280A-FEAR bind differently to human PD-L1 in comparison to IgG1-421-LC19S-FEAR, IgG1-547-FEAR, IgG1-LC33S-FEAR, IgG1-632-FEAR and IgG1-516-FEAR.

## Example 10: Effect of PD-L1 antibodies on the PD-1/PD-L1 interaction

The effect of bivalent and monovalent PD-L1 antibodies on the interaction of PD-1 and PD-L1 was determined in a PD-1/PD-L1 blockade bioassay as developed by Promega (Madison, USA). This is a bioluminescent cell-based assay consisting of two genetically engineered cell lines: PD-1 effector cells, which are Jurkat T cells expressing human PD-1 and a luciferase reporter driven by an NFAT response element (NFAT-RE), and PD-L1 aAPC/CHO-K1 cells, which are CHO-K1 cells expressing human PD-L1 and an engineered cell surface protein designed to activate cognate TCRs in an antigen-independent manner. When the two cell types are co-cultured, the PD-1/PD-L1 interaction inhibits TCR signaling and NFAT-RE-mediated luminescence. Addition of an antibody that blocks the PD-1/PD-L1 interaction releases the inhibitory signal and results in TCR activation and NFAT-RE-mediated luminescence.

PD-L1 aAPC/CHO-K1 cells (Promega, cat. no. J109A) were thawed according to the manufacturer's protocol, resuspended Ham's F12 medium (Promega, cat. no. J123A) containing 10% Fetal Bovine Serum (FBS; Promega, cat. no. J121A), and plated in 96 well flat bottom culture plates (CulturPlate-96, Perkin Elmer, cat. no. 6005680). Plates were incubated for 16 hours at 37°C, 5% $CO_2$. Supernatant was removed and serial dilutions of antibodies (final concentration ranging from 5 to 0.001 $\mu$g/mL; 4-fold dilutions in RPMI 1640 [Lonza, cat. no. BE12-115F] containing 1% Fetal Bovine Serum [FBS; Promega, cat. no. J121A]) were added. PD-1 effector cells (Promega, cat. no. J115A; thawed according to the manufacturer's protocol and resuspended in RPMI/1% FBS) were added. Plates were incubated for 6h at 37°C, 5% $CO_2$. After equilibration to room temperature, 40 $\mu$l Bio-Glo reagent (Bio-Glo luciferase assay substrate [Promega cat. no. G720B] reconstituted in Bio-Glo luciferase assay buffer [Promega, cat. no. G7198] according to the manufacturer's protocol) was added to each well. Plates were incubated at room temperature for 5-10 minutes and luminescence was measured using an EnVision Multilabel Reader (PerkinElmer). The effect on PD1-PD-L1 interaction, relative to control (without antibody added), was calculated as follows:

Fold induction = RLU (induced-background)/RLU (no antibody control-background), RLU is relative light units

Figure 7 shows that bivalent, monospecific antibodies IgG1-338-FEAR, IgG1-547-FEAR and IgG1-511-LC33S-FEAR efficiently blocked the interaction between PD1 and PD-L1 in a dose-dependent manner. Monovalent antibodies bsIgG1-b12-FEALx338-FEAR and bsIgG1-b12-FEALx547-FEAR also efficiently blocked PD1-PD-L1 interaction. bsIgG1-b12-FEALx511-LC33S-FEAR also blocked this interaction, albeit less efficiently.

## Example 11: *In vitro* cytotoxicity of CD3xPD-L1 bispecific antibodies

CD3xPD-L1 bispecific antibodies were tested in an *in vitro* cytotoxicity assay using tumor cell lines as target cells and purified T cells or peripheral blood mononuclear cells (PBMCs) as effector cells. T cells from donor buffy coats (Sanquin, Amsterdam, The Netherlands) were isolated using the RosetteSep human T cell enrichment cocktail (Cat: 15021C.1, Stemcell Technologies, France) according to manufacturer's instructions. PBMCs were isolated from 40 mL of buffy coat (Sanquin) using a Ficoll gradient (Lonza; lymphocyte separation medium, cat. no. 17-829E) according to the manufacturer's instructions.

MDA-MB-231 cells (16,000 cells/well), PC-3 cells (16,000 cells/well) or HELA cells (10,000 cells/well) were seeded into flat bottom 96 well plates (cat: 655180, Greiner-bio-one, The Netherlands) and cultured overnight at 37°C. T cells were added to tumor cells at an E:T ratio = 4:1 for MDA-MB-231 or PC-3 cells, and E:T = 8:1 for HELA cells. PBMC were added to tumor cells at an E:T ratio = 10:1. Serial dilutions of antibodies were added (final concentration ranging from 1000 to 0.06 ng/mL; 4-fold dilutions) and plates were incubated for 48 hours at 37°C. Next, supernatants were discarded and adhered cells were washed twice with PBS. 150 μL of 10% alamar blue (cat: DAL1100, Life Technologies, The Netherlands) solution, prepared in RPMI-1640 (cat: BE12-115F, Lonza, Switzerland) medium containing 10% donor bovine serum with iron (cat: 10371-029, Life Technologies, The Netherlands), was added to wells and incubated for 5h at 37°C. The absorbance was measured with Envision multilabel plate reader (PerkinElmer, US). Staurosporine (cat: S6942, Sigma-Aldrich, US) treated cells were set as 0% viability and untreated cells were set as 100% viability. The 'percentage viable cells' was calculated as follows:

% viable cells = (absorbance sample - absorbance staurosporine treated target cells)/(absorbance untreated target cells - absorbance staurosporine treated target cells) × 100.

Cytotoxicity of CD3xPD-L1 bispecific antibodies in MDA-MB-231 cells

Figure 8 shows that bsIgG1-huCD3-H1L1-FEALx338-FEAR, bsIgG1-huCD3-H1L1-FEALx547-FEAR and bsIgG1-huCD3-H1L1-FEALx511-LC33S-FEAR induced concentration-dependent cytotoxicity in the MDA-MB-231 cells, expressing relatively high levels of PD-L1, both when using purified T cells (A) and PBMCs (B) as effector cells.

Cytotoxicity of CD3xPD-L1 bispecific antibodies in PC-3 cells

Figure 9 shows that bsIgG1-huCD3-H1L1-FEALx338-FEAR, bsIgG1-huCD3-H1L1-FEALx547-FEAR and bsIgG1-huCD3-H1L1-FEALx511-LC33S-FEAR induced concentration-dependent cytotoxicity in PC-3 cells, both when using purified T cells (A) and PBMCs (B) as effector cells. bsIgG1-huCD3-H1L1-FEALx511-LC33S-FEAR was least efficient in inducing cytotoxicity in PC-3 cells, expressing moderate levels of PD-L1.

Cytotoxicity of CD3xPD-L1 bispecific antibodies in HELA cells

Figure 10 shows that bsIgG1-huCD3-H1L1-FEALx547-FEAR was capable of inducing cytotoxicity in HELA cells, expressing low levels of PD-L1, when using T cell as effector cells, and for one donor also when using PBMCs. bsIgG1-huCD3-H1L1-FEALx338-FEAR was less capable of inducing cytotoxicity in HELA cells, and bsIgG1-huCD3-H1L1-FEALx511-LC33S-FEAR did not induce cytotoxicity in HELA cells.

**Example 12: T cell activation and proliferation by CD3xPD-L1 bispecific antibodies**

CD3xPD-L1 bispecific antibodies were tested in an *in vitro* assay to measure T cell activation and proliferation, using MDA-MB-231 cells as target cells and purified T cells as effector cells. IgG1-b12 (with an Fc region capable of interacting with Fcγ receptors and C1q) was used as negative control. PBMCs were isolated from 40 mL of buffy coat (Sanquin) using a Ficoll gradient (Lonza; lymphocyte separation medium, cat. no. 17-829E) according to the manufacturer's instructions. From the purified PBMCs, T cells were isolated using the RosetteSep human T cell enrichment cocktail (Stemcell Technologies, France; cat. no. 15021C.1) according to the manufacturer's instructions. MDA-MB-231 cells were labeled with 0.07 μM CellTrace CFSE (ThermoFisher Scientific, cat. no. C34554) according to manufacturer's instruction and seeded (5,000 cells/well) into flat bottom 96 well plates (Greiner-bio-one, The Netherlands, cat. no. 655180) and adhered to the wells for 4 hours at 37°C. T cells were added to tumor cells at an E:T ratio = 8:1, so 40,000 cells/well. Serial dilutions of antibodies were added (final concentration ranging from 10000 to 1.5 ng/mL; 3-fold dilutions) and plates were incubated for 4 days at 37°C.

Next, supernatants (containing non-adherent cells) were transferred to a 96 well U-bottom plate (Greiner-bio-one), remaining cells were harvested through trypsin-EDTA (Lonza) treatment and combined with the cell supernatant in the 96-well U-bottom plate. Cells were washed with PBS (B.Braun) and stained 30 minutes at 4°C with a cocktail of antibodies: 1:200 anti-huCD4-pacific blue (Biolegend, cat. no. 300521), 1:50 anti-huCD8-FITC (BD, cat. no. 345772), 1:100 anti-huCD25-PE-Cy7 (eBiosciences, cat. no. 25-0259-42) and anti-huCD69-PE (BD, cat. no. 555531). Cells were washed once with ice-cold FACS-buffer and re-suspended in 80 μL FACS-buffer supplemented with 1:6000 diluted topro-3-iodine (ThermoFisher Scientific, cat. no. T3605).

T cell proliferation was determined by counting the total number of CD4$^{pos}$ and CD8$^{pos}$ T cells in a fixed volume of 50 μL on a flow cytometer. T cell activation was measured by counting the number of CD69$^{pos}$ (early T cell activation marker) and CD25$^{pos}$ (late T cell activation) cells in a fixed volume of 50 μL on a flow cytometer. Figure 11 shows that all CD3xPD-L1 bispecific antibodies induced T cell proliferation (indicated by increase in total number of T cells,). However, differences in the amount of activated and total T cells were seen between the different CD3xPD-L1 bispecific antibodies with bsIgG1-huCD3-H1L1-FEALx511-LC33S-FEAR being less effective, and bsIgG1-huCD3-H1L1-FEALx547-FEAR being most effective.

**Example 13. Determination of the contribution of PD-L1 amino acid residues in antibody binding using alanine scanning.**

Library design

A PD-L1 (Uniprot Q9NZQ7) single residue alanine library was synthesized (Geneart) in which all amino acid residues in the extracellular domain of human PD-L1 were individually mutated to alanines except for positions already containing alanines or cysteines. Cysteines were not mutated to minimize the chance of structural disruption of the antigen. The library was cloned in the pMAC expression vector containing a CMV/TK-polyA expression cassette, an Amp resistance gene and a pBR322 replication origin.

Library production and screening

The wild type PD-L1 and alanine mutants were expressed individually in FreeStyle HEK293 cells according to the manufacturer's instructions (Thermo Scientific). One day post transfection the cells were harvested. Approximately 100,000 cells were incubated with 20 μL Alexa488 conjugated antibody of interest in FACS buffer (Table 3). Cells were incubated for 1 hour at room temperature. Subsequently, 150 μL FACS buffer was added and cells were washed by centrifugation. Cells were suspended in 20 μL fresh FACS buffer (PBS [without $Ca^{++}$, $Mg^{++}$ and Phenol Red]/1% BSA fraction V/0.02% $NaN_3$) and stored at 4°C until analysis by flow cytometry using an iQue screener.
The entire experiment was performed 4 times.

Table 3: Antibodies used in determination of the contribution of PD-L1 amino acid residues in antibody binding using alanine scanning.

| Antibody | Stock concentration | Conjugate |
|---|---|---|
| BsG1-b12-FEALx547-FEAR-A488 | 3 μg/mL | Alexa488 |
| BsG1-b12-FEALx338-FEAR-A488 | | |
| IgG1-511-FEAR-LC33S-A488 | | |
| BsG1-b12-FEALx MPDL3280A-FEAR-A488 | | |
| IgG1 -MEDI4736-FEAR | | |
| IgG1-625-FEAR-A488 (used as control) | | |

Data analysis

For every sample, the average antibody binding per cell was determined as the geometric mean of the fluorescence intensity (gMFI) for the ungated cell population. The gMFI is influenced by the affinity of the antibody for the PD-L1 mutant and the expression level of the PD-L1 mutant per cell. Since specific alanine mutations can impact the surface expression level of the mutant PD-L1, and to correct for expression differences for each PD-L1 mutant in general, data were normalized against the binding intensity of a non-cross blocking PD-L1 specific control antibody (IgG1-625-FEAR-A488; comprising the heavy chain variable region (VH)set forth in SEQ ID NO: 106 and the light chain variable region (VL) set forth in SEQ ID NO: 110), using the following equation:

$$Normalized\ gMFI_{aa\ position} = \frac{gMFI_{Test\ Ab}}{gMFI_{Control\ Ab}}$$

In which 'aa position' refers to either a particular ala mutant of PD-L1 or wild type (wt) PD-L1.
To express loss or gain of binding of the antibodies on a linear Fold Change scale, the following calculation was used:

$$Fold\ Change = Log_{10}\left(\frac{Normalized\ gMFI_{ala\ mutant}}{Normalized\ gMFI_{wt}}\right)$$

Gain of binding in most cases will be caused by loss of binding of the reference antibody to specific ala mutants.

Upon these calculations, amino acid positions for which, upon replacing the amino acid with alanine, there is no loss or gain of binding by a particular antibody will give as result '0', gain of binding will result in '>0' and loss of binding will result in '<0'. To correct for sample variation, only PD-L1 amino acid residues where the Fold Change in binding was lower than the mean Fold Change - 1.5 $\times$ SD (indicated by the dotted line in Figure 12), where SD is the standard deviation of calculated fold changes from four independent experiments for a particular test antibody, were considered 'loss of binding mutants'.

In case the gMFI of the control antibody for a particular PD-L1 mutant was lower than the mean gMFI - 2.5 x SD of the mean gMFI$_{Control\ Ab}$, data were excluded from analysis (as for those PD-L1 mutants it was assumed expression levels were not sufficient).

Figure 12 shows the Fold Change in binding of the PD-L1 antibodies to PD-L1 variants with ala mutations at positions 42 to 131 (according to SEQ ID No 94). The results indicate that

- binding of antibody 338 is at least dependent on aa R113, Y123 and R125 of human PD-L1,
- binding of antibody 511 is at least dependent on aa F19, F42, E45, K46, L94 and I116 of human PD-L1; amino acids E45, K46 and L94 being directly involved in binding of the antibody and F19,F42 and I116 being indirectly involved in binding of the antibody due to their buried sidechains,
- binding of antibody 547 is at least dependent on aa E58 and R113 of human PD-L1,
- binding of antibody MEDI4736 is at least dependent on aa R113 and R125 of human PD-L1,
- and binding of antibody MPDL3280A is at least dependent on aa R125 and I126 of human PD-L1, where I126 due to its buried side chain may be indirectly involved in binding of the antibody.

**Example 14: Antibody-dependent cell-mediated cytotoxicity (ADCC)**

ADCC determined in a $^{51}$Cr release assay

MDA-MB-231 cells (ATCC, cat No.HTB-26) were harvested (to obtain $7 \times 10^6$ cells), washed (twice in PBS, 1500 rpm, 5 min) and collected in 2 mL RPMI 1640 medium supplemented with 10% cosmic calf serum (CCS) (HyClone, Logan, UT, USA)), to which 200 $\mu$Ci $^{51}$Cr (Chromium-51; Amersham Biosciences Europe GmbH, Roosendaal, The Netherlands) was added. The mixture was incubated for 1 hour at 37°C while shaking. After washing (twice in PBS, 1500 rpm, 5 min), the cells were re-suspended in RPMI 1640 medium/10% CCS and counted by trypan blue exclusion. Cells were adjusted to a concentration of $1 \times 10^5$ cells/mL.

Meanwhile, peripheral blood mononuclear cells (PBMCs) were isolated from fresh buffy coat (Sanquin, Amsterdam, The Netherlands) using standard Ficoll density centrifugation according to the manufacturer's instructions (lymphocyte separation medium; Lonza, Verviers, France). After resuspension of cells in RPMI 1640 medium/10% CCS, cells were counted by trypan blue exclusion and adjusted to $1 \times 10^7$ cells/mL.

50 $\mu$L of $^{51}$Cr-labeled targets cells were transferred to microtiter wells, and 50 $\mu$L of 30 $\mu$g/mL PD-L1 antibody was added (diluted in RPMI/10% CCS). As positive control, an antibody against an unrelated target expressed on MDA-MB-231 cells was used. Cells were incubated 15 min at RT, and 50 $\mu$L effector cells (PBMCs) were added, resulting in an effector to target ratio of 100:1. To determine the maximum amount of cell lysis, 100 $\mu$L 5% Triton-X100 was added instead of effector cells. 100 $\mu$L RPMI 1640/10% CCS was added instead of effector cells and antibody to determine the amount of spontaneous lysis. In addition, to determine the level of antibody independent cell lysis, 50 $\mu$L effector cells and 50 $\mu$L medium (instead of antibody) were added. The samples were incubated 4 hr at 37°C, 5% $CO_2$. To determine the amount of target cell lysis, the samples were centrifuged (1200 rpm, 3 min) and 75 $\mu$L of supernatant was transferred to micronic tubes, after which the released $^{51}$Cr was counted using a gamma counter. The percentage of antibody-mediated lysis was calculated as follows:

$$\frac{(counts\ per\ minute\ [cpm]\ sample - cpm\ antibody\ independent\ lysis)}{(cpm\ maximal\ lysis - cpm\ spontaneous\ lysis)}\ X\ 100\%$$

Figure 13 shows that IgG1-547-F405L induced ~10% dose-dependent lysis of MDA-MB-231 cells through ADCC. The positive control antibody induced only 20% maximum lysis, indicating that total lysis in this experiment was rather low. IgG1-511-F405L-LC33S and IgG1-338-F405L did not induce lysis of MDA-MB-231.

ADCC determined in a Luminescent ADCC Reporter BioAssay

The ability of PD-L1 antibodies to induce FcγRIIIa (CD16) crosslinking, as a surrogate for ADCC, was also determined using a Luminescent ADCC Reporter BioAssay (Promega, Cat # G7018) on MDA-MB-231 cells, according to the manufacturer's recommendations. As effector cells, the kit contains Jurkat human T cells that are engineered to stably express high affinity FcγRIIIa (V158) and a nuclear factor of activated T cells (NFAT)-response element driving expression of firefly luciferase. Briefly, MDA-MB-231 cells (12,500 cells/well) were seeded in Culture OptiPlates (Perkin Elmer) in ADCC Assay Buffer [RPMI-1640 medium [(Lonza, Cat # BE12-115F) supplemented with 3.5% Low IgG Serum] and incubated for 6 hours at 37°C/5%CO$_2$ in a total volume of 75 μL containing antibody concentration series (0.5-250 ng/mL final concentrations in 3.5-fold dilutions) and thawed ADCC Bioassay Effector Cells. After adjusting the plates for 15 minutes to room temperature (RT), 75 μL Bio Glo Assay Luciferase Reagent was added and plates were incubated for 5 minutes at RT. Luciferase production was quantified by luminescence readout on an EnVision Multilabel Reader (Perkin Elmer). Background levels were determined from wells to which only target cells and antibody (no effector cells) was added. As negative control, wells containing only target and effector cells (no antibody) were used.

Figure 14 shows that IgG1-547-F405L was highly effective in inducing ADCC as determined in the reporter assay. Also IgG1-MEDI4736-F405L and IgG1-MPDL3280A-K409R induced ADCC, but not to the same extent as IgG1-547-F405L. IgG1-511-F405L-LC33S and IgG1-338-F405L did not induce ADCC.

SEQUENCE LISTING

| SEQ ID NO | LABEL | SEQUENCE |
|---|---|---|
| 1 | VH-338 | EVQVVESGGGLVQPGGSLRLSCAASGFTFSRFWMSWVRQAPGKGL EWVANIKQDGGEKYYVDSVKGRFTISRDNAKNSLYLQMNSLRGEDT AVYYCARDDNWNGHFDYWGQGTLVTVSS |
| 2 | VH-338-CDR1 | GFTFSRFW |
| 3 | VH-338-CDR2 | IKQDGGEK |
| 4 | VH-338-CDR3 | ARDDNWNGHFDY |
| 5 | VK-338 | DIQMTQSPSTLSASVGDRVTITCRASQSISSWLAWYQQKPGKAPNLL IYKASSLESGVPSRFSGSGSGTEFTLTISSLQPDDFATYYCQQYYGSYI TFGQGTRLEIK |
| 6 | VK-338-CDR1 | QSISSW |
|  | VK-338-CDR2 | KAS |
| 7 | VK-338-CDR3 | QQYYGSYIT |
| 8 | VH-511 | QVQLVQSGSELKKPGASVMVSCKASGYTFTSYVMNWVRQAPGQGL EWMGWINSYTGNPTSAQGFTGRFVFSFDTSVNTAYLQISSLKAEDTA VYYCARGYCTSTSCYLDYWGQGTLVTVSS |
| 9 | VH-511-CDR1 | GYTFTSYV |
| 10 | VH-511-CDR2 | INSYTGNP |
| 11 | VH-511-CDR3 | ARGYCTSTSCYLDY |
| 12 | VL-511 | SYELTQPPSVSVSPGHTARITCSGDALPKKYACWFQQKSGQAPVLVI YEDSKRPSGIPERFSGSTSGTMATLTISGAQVEDETDYYCYSADTSG THRVFGGGTKLTVL |

(continued)

| SEQ ID NO | LABEL | SEQUENCE |
|---|---|---|
| 13 | VL-511-CDR1 | ALPKKY |
| | VL-511-CDR2 | EDS |
| 14 | VL-511-CDR3 | YSADTSGTHRV |
| 15 | VL-511-LC33S | SYELTQPPSVSVSPGHTARITCSGDALPKKYA**S**WFQQKSGQAPVLVI YEDSKRPSGIPERFSGSTSGTMATLTISGAQVEDETDYYCYSADTSG THRVFGGGTKLTVL |
| 16 | VL-511-LC33S-CDR1 | ALPKKY |
| | VL-511-LC33S-CDR2 | EDS |
| 17 | VL-511-LC33S-CDR3 | YSADTSGTHRV |
| 18 | VH-547 | EVQLLEPGGGLVQPGGSLRLSCEASGSTFSTYAMSWVRQAPGKGLE WVSGFSGSGGFTFYADSVRGRFTISRDSSKNTLFLQMSSLRAEDTAV YYCAIPARGYNYGSFQHWGQGTLVTVSS |
| 19 | VH-547-CDR1 | GSTFSTYA |
| 20 | VH-547-CDR2 | FSGSGGFT |
| 21 | VH-547-CDR3 | AIPARGYNYGSFQH |
| 22 | VL-547 | SYVLTQPPSVSVAPGQTARITCGGNNIGSKSVHWYQQKPGQAPVLV VYDDNDRPSGLPERFSGSNSGNTATLTISRVEAGDEADYYCQVWDS SSDHVVFGGGTKLTVL |
| 23 | VL-547-CDR1 | NIGSKS |
| | VL-547-CDR2 | DDN |
| 24 | VL-547-CDR3 | QVWDSSSDHVV |
| 25 | VH-huCD3-H1 | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRHGNFGNSYVSWFAYWGQGTLVTVSS |
| 26 | VH-huCD3-H1-CDR1 | GFTFNTYA |
| 27 | VH-huCD3-H1-CDR2 | IRSKYNNYAT |
| 28 | VH-huCD3-H1-CDR3 | VRHGNFGNSYVSWFAY |
| 29 | VL-huCD3-L1 | QAVVTQEPSFSVSPGGTVTLTCRSSTGAVTTSNYANWVQQTPGQAF RGLIGGTNKRAPGVPARFSGSLIGDKAALTITGAQADDESIYFCALWY SNLWVFGGGTKLTVL |
| 30 | VL-huCD3-L1-CDR1 | TGAVTTSNY |
| | VL-huCD3-L1-CDR2 | GTN |
| 31 | VL-huCD3-L1-CDR3 | ALWYSNLWV |

(continued)

| SEQ ID NO | LABEL | SEQUENCE |
|---|---|---|
| 32 | VH-321 | EVQLVESGGGLVQPGGSLRLSCAASGFTFSSYWMSWVRQAPGKGLE WVANIKQDGNEKYYVDSVKGRFTISRDNAKNSLYVQMNSLRAEDTA VYYCARDLYYGSGTYPPFDYWGQGTLVTVSS |
| 33 | VH-321-CDR1 | GFTFSSYW |
| 34 | VH-321-CDR2 | IKQDGNEK |
| 35 | VH-321-CDR3 | ARDLYYGSGTYPPFDY |
| 36 | VK-321 | DIQMTQSPSTLSASVGDRVTITCRASQSISSWLAWYLQKPGKAPKLL IYKASSLESGVPSRFSGSGSGTEFTLTISSLQPDDFATYYCQQYHSSS YTFGQGTKLEIK |
| 37 | VK-321-CDR1 | QSISSW |
|  | VK-321-CDR2 | KAS |
| 38 | VK-321-CDR3 | QQYHSSSYT |
| 39 | VH-huCD3-H1-T31P | EVKLVESGGGLVQPGGSLRLSCAASGFTFNPYAMNWVRQAPGKGLE WVARIRSKYNNYATYYADSVKDRFTISRDDSKSSLYLQMNNLKTEDT AMYYCVRHGNFGNSYVSWFAYWGQGTLVTVSS |
| 40 | VH-huCD3-H1-T31M | EVKLVESGGGLVQPGGSLRLSCAASGFTFNMYAMNWVRQAPGKGLE WVARIRSKYNNYATYYADSVKDRFTISRDDSKSSLYLQMNNLKTEDT AMYYCVRHGNFGNSYVSWFAYWGQGTLVTVSS |
| 41 | VH-huCD3-H1-Y114V | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRHGNFGNSYVSWFAYVWGQGTLVTVSS |
| 42 | VH-huCD3-H1-Y114M | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRHGNFGNSYVSWFAMWGQGTLVTVSS |
| 43 | VH-huCD3-H1-Y116R | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRHGNFGNSYVSWFARWGQGTLVTVSS |
| 44 | VH-huCD3-H1-S110A | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRHGNFGNSYVAWFAYWGQGTLVTVSS |
| 45 | VH-huCD3-H1-H101G | EVKLVESGGGLVQPGGSLRLSCAASGFTFNTYAMNWVRQAPGKGLE WVARIRSKYNNYATYYADSVKDRFTISRDDSKSSLYLQMNNLKTEDT AMYYCVRGGNFGNSYVSWFAYWGQGTLVTVSS |

(continued)

| SEQ ID NO | LABEL | SEQUENCE |
|---|---|---|
| 46 | VH-421 | EVQLVESGGGLVQPGRSLRLSCAASGFTFDDYAMHWVRQGPGKGLE WVSGIRWNSGSMHYADSVKGRFTISRDNAKSSLYLQMNSLRAEDTA LYYCARAPWYSGAWHPDYWGQGTLVTVSS |
| 47 | VH-421-CDR1 | GFTFDDYA |
| 48 | VH-421-CDR2 | IRWNSGSM |
| 49 | VH-421-CDR3 | ARAPWYSGAWHPDY |
| 50 | VL-421-C91S | QSALTQPRSVSGSPGQSVTISCTGTSSDVGTYNYVSWYQQHPGKAP KLMIYDVIKRPSGVPDRFSGSKSGNTASLTLSGLQAEDEADYYC**S**SY AGTYTLLFGGGTKLTVL |
| 51 | VL-421-C91S-CDR1 | SSDVGTYNY |
| | VL-421-C91S-CDR2 | DVI |
| 52 | VL-421-C91S-CDR3 | **S**SYAGTYTLL |
| 53 | VH-476-N101Q | EVQMLESGGGLVQPGGSLRLSCAASGFTFRSYAMSWVRQAPGKGLE WVSGIGDSGGSTYHADSVKGRFTISRDNSKNTLYLQMNSLRAEDTA VYYCAKLG**Q**SSGWYDHYYYYGMDVWGQGTTVTVSS |
| 54 | VH-476-N101Q-CDR1 | GFTFRSYA |
| 55 | VH-476-N101Q-CDR2 | IGDSGGST |
| 56 | VH-476-N101Q-CDR3 | AKLG**Q**SSGWYDHYYYYGMDV |
| 57 | VL-476-C33S | SYELTQPPSVSVSPGQTASITCSGDKLGNKYV**S**WFQQKPGQSPVLVI YRDSERPSGIPERFSGSNSGNTATLTISGTQAVDEADFYCQAWDSST VVFGGGTKLTVL |
| 58 | VL-476-C33S-CDR1 | KLGNKY |
| | VL-476-C33S-CDR2 | RDS |
| 59 | VL-476-C33S-CDR3 | QAWDSSTVV |
| 60 | VH-516 | QVQLQESGPGLVKPSDTLSLTCAVSDYSISSNDWWGWIRQPPGKGL EWIGYIYYSGTGYYNPSLKSRVTISIDTSKNQFSLKLNSVTAVDTAVY YCARTRVGARRAFDYWGQGTLVTVSS |
| 61 | VH-516-CDR1 | DYSISSNDW |
| 62 | VH-516-CDR2 | IYYSGTG |
| 63 | VH-516-CDR3 | ARTRVGARRAFDY |
| 64 | VL-516 | SYVLTQPPSVSVAPGQTARITCGGNNIGSKSVHWYQQKPGQAPVLV VYDDSDRPSGIPERFSGSNSGNTATLTISRVEAGDEADYYCQVWDS SSDHVVFGGGTKLTVL |
| 65 | VL-516-CDR1 | NIGSKS |
| | VL-516-CDR2 | DDS |

(continued)

| SEQ ID NO | LABEL | SEQUENCE |
|---|---|---|
| 66 | VL-516-CDR3 | QVWDSSSDHVV |
| 67 | VH-632 | QVQLVESGGGLVKPGGSLRLSCAASGFTFSDYYMSWIRQAPGKGLE WVSYIGSSSNTIYYADSVKGRFTISRDNAKNSLYLQMNSLRAEDTAV YSCARDRVKYGSPGSLFDYWGQGTLVTVSS |
| 68 | VH-632-CDR1 | GFTFSDYY |
| 69 | VH-632-CDR2 | IGSSSNTI |
| 703 | VH-632-CDR3 | ARDRVKYGSPGSLFDY |
| 71 | VL-632 | SYELTQPPSVSVSPGQTARITCSGDALPKKYAFWYQQKSGQAPVLVI YEDSKRPSGIPERFSGSSSGTMATLTISGAQVEDEADYYCYSTASSG DHRVFGGGTKLTVL |
| 72 | VL-632-CDR1 | ALPKKY |
| | VL-632-CDR2 | EDS |
| 73 | VL-632-CDR3 | YSTASSGDHRV |
| 74 | VH-MPDL3280A | EVQLVESGGGLVQPGGSLRLSCAASGFTFSDSWIHWYRQAPGKGLE WYAWISPYGGSTYYADSVKGRFTISADTSKNTAYLQMNSLRAEDTAV YYCARRHWPGGFDYWGQGTLVTVSS |
| 75 | VH-MPDL3280A-CDR1 | GFTFSDSW |
| 76 | VH-MPDL3280A-CDR2 | ISPYGGST |
| 77 | VH-MPDL3280A-CDR3 | ARRHWPGGFDY |
| 78 | VL-MPDL3280A | DIQMTQSPSSLSASVGDRVTITCRASQDVSTAVAWYQQKPGKAPKL LIYSASFLYSGVPSRFSGSGSGTDFTLTISSLQPEDFATYYCQQYLYHP ATFGQGTKVEIK |
| 79 | VL-MPDL3280A-CDR1 | QDVSTA |
| | VL-MPDL3280A-CDR2 | SAS |
| 80 | VL-MPDL3280A-CDR3 | QQYLYHPAT |
| 81 | VH-MEDI4736B | EVQLVESGGGLVQPGGSLRLSCAASGFTFSRYWMSWVRQAPGKGLE WVANIKQDGSEKYYVDSVKGRFTISRDNAKNSLYLQMNSLRAEDTA VYYCAREGGWFGELAFDYWGQGTLVTVSS |
| 82 | VH-MEDI4736B-CDR1 | GFTFSRYW |
| 83 | VH-MEDI4736B-CDR2 | IKQDGSEK |

(continued)

| SEQ ID NO | LABEL | SEQUENCE |
|---|---|---|
| 84 | VH-MEDI4736B-CDR3 | AREGGWFGELAFDY |
| 85 | VL-MEDI4736B | EIVLTQSPGTLSLSPGERATLSCRASQRVSSSYLAWYQQKPGQAPRL LIYDASSRATGIPDRFSGSGSGTDFTLTISRLEPEDFAVYYCQQYGSL PWTFGQGTKVEIK |
| 86 | VL-MEDI4736B-CDR1 | QRVSSSY |
| | VL-MEDI4736B-CDR2 | DAS |
| 88 | VL-MEDI4736B-CDR3 | QQYGSLPWT |
| 89 | IgG1-FEAR-Fc | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPE**FE**GGPSVFLFPPKPKDTLMISRTPEV TCVVV**A**VSHEDPEVKFNWYVDGVEVHNAKTKPREEQYNSTYRVVSV LTVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSFFLYS**R**LTVDKSRWQQGNVFSCSVMHEALHNHYTQKSLSLSPG K |
| 90 | IgG1-FEAL-Fc | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPE**FE**GGPSVFLFPPKPKDTLMISRTPEV TCVVV**A**VSHEDPEVKFNWYVDGVEVHNAKTKPREEQYNSTYRVVSV LTVLHQDWLNGKEYKCKVSNKALPAPIEKTISKAKGQPREPQVYTLPP SREEMTKNQVSLTCLVKGFYPSDIAVEWESNGQPENNYKTTPPVLDS DGSF**L**LYSKLTVDKSRWQQGNVFSCSVMHEALHNHYTQKSLSLSPG K |
| 91 | Kappa-C | RTVAAPSVFIFPPSDEQLKSGTASVVCLLNNFYPREAKVQWKVDNAL QSGNSQESVTEQDSKDSTYSLSSTLTLSKADYEKHKVYACEVTHQGL SSPVTKSFNRGEC |
| 92 | Lambda-C | GQPKAAPSVTLFPPSSEELQANKATLVCLISDFYPGAVTVAWKADSSP VKAGVETTTPSKQSNNKYAASSYLSLTPEQWKSHRSYSCQVTHEGS TVEKTVAPTECS |

(continued)

| SEQ ID NO | LABEL | SEQUENCE |
|---|---|---|
| 93 | IgGlm(f) - VH - aa 118-447 EU numbering | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVT CVVVDVSHEDPEVKFNWYVDGVEVHNAKTKPREEQYNSTYRVVSVL TVLHQDWLNGKEYKCKVSNKALPAPIEKTISKAKGQPREPQVYTLPPS REEMTKNQVSLTCLVKGFYPSDIAVEWESNGQPENNYKTTPPVLDSD GSFFLYSKLTVDKSRWQQGNVFSCSVMHEALHNHYTQKSLSLSPGK |
| 94 | PD-L1 (Genbank accession no. NP_054862.1) | MRIFAVFIFMTYWHLLNAFTVTVPKDLYVVEYGSNMTIECKFPVEKQL DLAALIVYWEMEDKNIIQFVHGEEDLKVQHSSYRQRARLLKDQLSLG NAALQITDVKLQDAGVYRCMISYGGADYKRITVKVNAPYNKINQRILV VDPVTSEHELTCQAEGYPKAEVIWTSSDHQVLSGKTTTTNSKREEKL FNVTSTLRINTTTNEIFYCTFRRLDPEENHTAELVIPELPLAHPPNERTH LVILGAILLCLGVALTFIFRLRKGRMMDVKKCGIQDTNSKKQSDTHLE ET |
| 95 | Mature human CD3ε | QDGNEEMGGITQTPYKVSISGTTVILTCPQYPGSEILWQHNDKNIGG DEDDKNIGSDEDHLSLKEFSELEQSGYYVCYPRGSKPEDANFYLYLR ARVCENCMEMDVMSVATIVIVDICITGGLLLLVYYWSKNRKAKAKPV TRGAGAGGRQRGQNKERPPPVPNPDYEPIRKGQRDLYSGLNQRRI |
| 96 | IgG1m(a) CH3 region | GQPREPQVYTLPPSRDELTKNQVSLTCLVKGFYPSDIAVEWESNGQP ENNYKTTPPVLDSDGSFFLYSKLTVDKSRWQQGNVFSCSVMHEALH NHYTQKSLSLSPGK |
| 97 | IgGlm(f) CH3 region | GQPREPQVYTLPPSREEMTKNQVSLTCLVKGFYPSDIAVEWESNGQP ENNYKTTPPVLDSDGSFFLYSKLTVDKSRWQQGNVFSCSVMHEALH NHYTQKSLSLSPGK |
| 98 | IgG1m(ax) CH3 region | GQPREPQVYTLPPSRDELTKNQVSLTCLVKGFYPSDIAVEWESNGQP ENNYKTTPPVLDSDGSFFLYSKLTVDKSRWQQGNVFSCSVMHEGLH NHYTQKSLSLSPGK |
| 99 | VH-huCD3-H1-CDR1-T31P | GFTFNPYA |
| 100 | VH-huCD3-H1-CDR1-T31M | GFTFNMYA |
| 101 | VH-huCD3-H1-CDR3-Y114V | VRHGNFGNSYVSWFAV |
| 102 | VH-huCD3-H1-CDR3-Y114M | VRHGNFGNSYVSWFAM |

(continued)

| SEQ ID NO | LABEL | SEQUENCE |
|---|---|---|
| 103 | VH-huCD3-H1-CDR3-Y116R | VRHGNFGNSYVSWFAR |
| 104 | VH-huCD3-H1-CDR3-S110A | VRHGNFGNSYVAWFAY |
| 105 | VH-huCD3-H1-CDR3-H101G | VRGGNFGNSYVSWFAY |
| 106 | VH-7717-625 | HMQLVESGGGVAQPGRSLRLSCAASGFTFSNYGMHWVRQAPGRGLEWLAVMSYDGETKYYADSVKGRFTISRDNSENTLFLQMNSLRAEDTAVYYCAKDTSNGWNYYFYGMDVWGQGTTVTVSS |
| 107 | VH-7717-625_CDR1 | GFTFSNYG |
| 108 | VH-7717-625_CDR2 | MSYDGETK |
| 109 | VH-7717-625_CDR3 | AKDTSNGWNYYFYGMDV |
| 110 | VL-7717-625 | SYELTQPPSVSVSPGQTARITCSGDALPKKFASWYQQKSGQAPVLVIYEDSKRPSGIPERVSGSSSGTMATLTISGAQTEDEADYYCYSTDRSGYHWVFGGGTKLTVL |
| 111 | VL-7717-625_CDR1 | ALPKKF |
| | VL-7717-625_CDR2 | EDS |
| 112 | VL-7717-625_CDR3 | YSTDRSGYHWV |
| 113 | IgG1m(f)_consta nt | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 114 | IgG1-K409R_constant domain | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYS**R**LTVDKSRWQQGNVFSCSVMHEALHNHYTQKSLSLSPGK |

(continued)

| SEQ ID NO | LABEL | SEQUENCE |
|---|---|---|
| 115 | IgG1-F405L_constant domain | ASTKGPSVFPLAPSSKSTSGGTAALGCLVKDYFPEPVTVSWNSGALT SGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVT CVVVDVSHEDPEVKFNWYVDGVEVHNAKTKPREEQYNSTYRVVSVL TVLHQDWLNGKEYKCKVSNKALPAPIEKTISKAKGQPREPQVYTLPPS REEMTKNQVSLTCLVKGFYPSDIAVEWESNGQPENNYKTTPPVLDSD GSF**L**LYSKLTVDKSRWQQGNVFSCSVMHEALHNHYTQKSLSLSPGK |

## Claims

1. An antibody comprising an antigen-binding region capable of binding to human PD-L1, wherein the antibody inhibits the binding of human PD-L1 to human PD-1 and

   (i) competes for binding to human PD-L1 with an antibody comprising a VH sequence as set forth in SEQ ID NO:8 and a VL sequence as set forth in SEQ ID NO:15 **[511],** but does not compete for binding to human PD-L1 with an antibody comprising a VH sequence as set forth in SEQ ID NO:18 and a VL sequence as set forth in SEQ ID NO:22 **[547],** or
   (ii) competes for binding to human PD-L1 with an antibody comprising a VH sequence as set forth in SEQ ID NO:18 and a VL sequence as set forth in SEQ ID NO:22 **[547],** but does not compete for binding to human PD-L1 with an antibody comprising a VH sequence as set forth in SEQ ID NO:8 and a VL sequence as set forth in SEQ ID NO:15 **[511].**

2. The antibody according to claim 1, wherein said antibody competes for binding to human PD-L1 with an antibody comprising a VH sequence as set forth in SEQ ID NO:1 and a VL sequence as set forth in SEQ ID NO:5 **[338].**

3. The antibody according to any of the preceding claims, wherein the binding of said antibody to human PD-L1 is not displaced by an antibody comprising a VH sequence as set forth in SEQ ID NO:53 and a VL sequence as set forth in SEQ ID NO:57[476].

4. The antibody according to any of the preceding claims, wherein the binding of said antibody to human PD-L1 is not blocked by binding of an antibody comprising a VH sequence as set forth in SEQ ID NO: 106 and a VL sequence as set forth in SEQ ID NO: 110 **[625].**

5. The antibody according to any of the preceding claims, wherein the binding of said antibody to human PD-L1 is blocked by an antibody comprising a VH sequence as set forth in SEQ ID NO:18 and a VL sequence as set forth in SEQ ID NO:22 **[547].**

6. The antibody according to claim 1, wherein said antibody:

   (i) is capable of binding to the same epitope of human PD-L1 as an antibody comprising a VH sequence as set forth in SEQ ID NO:1 and a VL sequence as set forth in SEQ ID NO:5 **[338],** or
   (ii) is capable of binding to the same epitope of human PD-L1 as an antibody comprising a VH sequence as set forth in SEQ ID NO:8 and a VL sequence as set forth in SEQ ID NO:15 **[511],** or
   (iii) is capable of binding to the same epitope of human PD-L1 as an antibody comprising a VH sequence as set forth in SEQ ID NO:18 and a VL sequence as set forth in SEQ ID NO:22 **[547].**

7. The antibody according to any one of claims 1-4 and 6, wherein binding of the antibody to a mutant PD-L1 in which any one or more of the amino acid residues at positions corresponding to positions 113 (R113), 123 (Y123) and 125 (R125) in SEQ ID NO: 94 have been substituted with alanines, is reduced as compared to binding to wild type PD-L1 having the amino acid sequence set forth in SEQ ID NO: 94; reduced binding being determined as fold change in binding of said antibody being less than mean fold change in binding over all alanine mutants - 1.5xSD , wherein SD is the standard deviation of all calculated fold changes for the antibody to the mutant PDL1 and fold change in binding is calculated as set forth in Example 13 **[338].**

8.  The antibody according to any one of claims 1-2 and 6, wherein said antibody binds to an epitope on PD-L1 (SEQ ID NO: 94), said epitope comprising the amino acid residues at position 113 (R113), the amino acid residue at position 123 (Y123) and/or the amino acid residue at position 125 (R125) of SEQ ID NO: 94.

9.  The antibody according to any one of claims 1, 3, 4 and 6, wherein binding of the antibody to a mutant PD-L1 in which any one or more of the amino acid residues at positions corresponding to positions 19 (F19), 42 (F42), 45 (E45), 46 (K46), 94 (L94) and 116 (I116) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13 **[511].**

10. The antibody according to any of claims 1, 3, 4 and 6, wherein said antibody binds to an epitope on PD-L1 (SEQ ID NO: 94), said epitope comprising one or more amino acid residues selected from the group consisting of: the amino acid residues at position 45 (E45), the amino acid residue at position 46 (K46; and/or the amino acid residue at position 94 (L94) of SEQ ID NO: 94.

11. The antibody according to any one of claims 1, 5 and 6, wherein binding of the antibody to a mutant PD-L1 in which any one or more of the amino acid residues at positions corresponding to positions 58 (E58) and 113 (R113) in SEQ ID NO: 94 has/have been substituted with alanines, is reduced as compared to wild type PD-L1 having the amino acid sequence set forth in SEQ ID NO: 94; reduced binding being determined as fold change in binding of said antibody being less than mean fold change in binding over all alanine mutants - 1.5xSD, wherein SD is the standard deviation of all calculated fold changes for the antibody to the mutant PDL1 and fold change in binding is calculated as set forth in Example 13 **[547].**

12. The antibody according to any of claims 1, 5 and 6, wherein said antibody binds to an epitope on PD-L1 (SEQ ID NO: 94), said epitope comprising the amino acid residue at position 58 (E58) and/or the amino acid residue at position 113 (R113) of SEQ ID NO: 94.

13. The antibody according to any one of the preceding claims, wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4, SEQ ID NO:11 and SEQ ID NO:21.

14. The antibody according to any one of the preceding claims, wherein said antigen-binding region capable of binding to human PD-L1 comprises:

    (i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively **[338],** or
    (ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively **[511],** or
    (iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively **[547].**

15. The antibody according to any one of the preceding claims, wherein said antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1, SEQ ID NO:8 and SEQ ID NO:18.

16. The antibody according to any one of the preceding claims, wherein said antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5, SEQ ID NO:15 and SEQ ID NO:22.

17. The antibody according to any one of the preceding claims, wherein said antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5 **[338],** or
(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15 **[511],** or
(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22 **[547].**

18. The antibody according to claim 17, wherein said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

19. The antibody according to any one of the preceding claims, wherein said antibody is capable of inducing dose-dependent lysis of epithelial cells of an adenocarcinoma, such MDA-MB-231 through antibody-dependent cell-mediated cytotoxicity (ADCC).

20. The antibody according to claim 19, wherein said antibody is capable of reducing the number of cells in a culture of said epithelial cells by at least 5%, such as at least 6%, 7%, 8%, 9% or at least 10% as a result of cell lysis.

21. The antibody according to claim 19 or 20, wherein ADCC is determined *in vitro* in a $^{51}$Cr release assay, such as the assay disclosed in example 14.

22. The antibody according to any one of claims 19 to 20, wherein ADCC is determined *in vitro,* by incubating said epithelial cells with a composition comprising the antibody and effector cells, such as peripheral blood mononuclear cells (PBMCs), for 4 hours at 37°C, 5% $CO_2$, the amount of antibody in said composition being within the range of 0.1-1 $\mu$g/mL and the ratio of effector cells to epithelial cells being 100:1.

23. The antibody according to claim 19 or 20, wherein said lysis of epithelial cells is determined *in vitro* in a luciferase reporter assay as a surrogate for ADCC, such as the luminescent ADCC reporter bioassay disclosed in example 14.

24. The antibody according to claim 23, wherein ADCC is determined *in vitro,* by

i) contacting a culture of said epithelial cells with a composition comprising the antibody and Jurkat human T-cells stably expressing FcγRIIIa (CD16) and firefly luciferase (effector cells), at an effector cell:epithelial cell ratio of 1:1.
ii) adjusting the culture of the epithelial cells and effector cells to room temperature for 15 minutes,
iii) incubating the culture of the epithelial cells and effector cells with a luciferase substrate, and
iv) determining luciferase production in said cell culture;

the amount of antibody in said composition being within the range of 0.5-250 ng/mL and the ratio of effector cells to epithelial cells being 1:1.

25. The antibody according to any one of claims 19, 20, 23 and 24, wherein when ADCC of said epithelial cells is determined in a luciferase reporter assay, such as a reporter assay defined in claim 23 or 24, then the ADCC observed after incubation of a culture of the epithelial cells with a test composition comprising said antibody is at least 1.5 times the ADCC observed after incubation of a culture of the epithelial cells with a composition comprising reference antibody; ADCC being determined as relative luminescence units (RLU), the concentration of antibody in said test composition and in said composition comprising a reference antibody being the same and within the range of 20 to 250 ng/ml, and the reference antibody being selected from:

a) an antibody comprising the VH sequence set forth in SEQ ID NO: 74 and the VL sequence set forth in SEQ ID NO: 78; and
b) an antibody comprising the VH sequence set forth in SEQ ID NO: 81 and the VL sequence set forth in SEQ ID NO: 85.

26. The antibody according to any one of the preceding claims, wherein said antigen-binding region capable of binding to human PD-L1 comprises:

(i) a VH sequence as set forth in SEQ ID NO:1 and a VL sequence as set forth in SEQ ID NO:5 **[338],** or
(ii) a VH sequence as set forth in SEQ ID NO:8 and a VL sequence as set forth in SEQ ID NO:15 **[511],** or
(iii) a VH sequence as set forth in SEQ ID NO:18 and a VL sequence as set forth in SEQ ID NO:22 **[547].**

27. An antibody comprising an antigen-binding region capable of binding to human PD-L1, wherein said antibody comprises:

(i) a heavy chain variable region (VH) comprising CDR1, CDR2, and CDR3 sequences as set forth in SEQ ID NOs: 33, 34 and 35, respectively, and a light chain variable region (VL) comprising CDR1, CDR2, and CDR3 having the sequences as set forth in SEQ ID NO:37, the sequence KAS, and the sequence as set forth in SEQ ID NO:38, respectively **[321],** or
(ii) a heavy chain variable region (VH) comprising CDR1, CDR2, and CDR3 sequences as set forth in SEQ ID NOs: 47, 48 and 49, respectively, and a light chain variable region (VL) comprising CDR1, CDR2, and CDR3 having the sequences as set forth in SEQ ID NO:51, the sequence DVI, and the sequence as set forth in SEQ ID NO:52, respectively **[421],** or
(iii) a heavy chain variable region (VH) comprising CDR1, CDR2, and CDR3 sequences as set forth in SEQ ID NOs: 54, 55 and 56, respectively, and a light chain variable region (VL) comprising CDR1, CDR2, and CDR3 having the sequences as set forth in SEQ ID NO:58, the sequence RDS, and the sequence as set forth in SEQ ID NO:59, respectively **[476],** or
(iv) a heavy chain variable region (VH) comprising CDR1, CDR2, and CDR3 sequences as set forth in SEQ ID NOs: 61, 62 and 63, respectively, and a light chain variable region (VL) comprising CDR1, CDR2, and CDR3 having the sequences as set forth in SEQ ID NO:65, the sequence DDS, and the sequence as set forth in SEQ ID NO:66, respectively **[516],** or
(v) a heavy chain variable region (VH) comprising CDR1, CDR2, and CDR3 sequences as set forth in SEQ ID NOs: 107, 108 and 109, respectively, and a light chain variable region (VL) comprising CDR1, CDR2, and CDR3 having the sequences as set forth in SEQ ID NO:111, the sequence EDS, and the sequence as set forth in SEQ ID NO:113, respectively **[625]**
(vi) a heavy chain variable region (VH) comprising CDR1, CDR2, and CDR3 sequences as set forth in SEQ ID NOs: 68, 69 and 70, respectively, and a light chain variable region (VL) comprising CDR1, CDR2, and CDR3 having the sequences as set forth in SEQ ID NO:72, the sequence EDS, and the sequence as set forth in SEQ ID NO:73, respectively **[632].**

28. The antibody according to claim 27, wherein said antibody comprises:

(i) a VH sequence as set forth in SEQ ID NO:32 and a VL sequence as set forth in SEQ ID NO:36 **[321],** or
(ii) a VH sequence as set forth in SEQ ID NO:46 and a VL sequence as set forth in SEQ ID NO:50 **[421],** or
(iii) a VH sequence as set forth in SEQ ID NO:53 and a VL sequence as set forth in SEQ ID NO:57 **[476],** or
(iv) a VH sequence as set forth in SEQ ID NO:60 and a VL sequence as set forth in SEQ ID NO:64 **[516],** or
a VH sequence as set forth in SEQ ID NO:106 and a VL sequence as set forth in SEQ ID NO:110 **[625]**

(v) a VH sequence as set forth in SEQ ID NO:67 and a VL sequence as set forth in SEQ ID NO:71**[632].**

29. The antibody according to any one of the preceding claims, wherein said antibody is monovalent.

30. The antibody according to any one of the preceding claims, wherein said antibody is a bivalent antibody having two antigen-binding regions capable of binding to human PD-L1 and wherein said two antigen-binding regions have identical variable region sequences.

31. The antibody according to any one of the preceding claims, wherein said antibody is a bivalent bispecific antibody, which, in addition to said (first) antigen-binding region capable of binding to human PD-L1, comprises a (second) antigen-binding region capable of binding to a second antigen or to a different epitope of human PD-L1, wherein said second antigen is not human CD3ε.

32. A bispecific antibody comprising an antigen-binding region capable of binding to human PD-L1 and an antigen-binding region capable of binding to human CD3ε (epsilon), wherein the antigen-binding region capable of binding to human PD-L1 has the features set forth in any one of the preceding claims.

33. The bispecific antibody according to claim 32, wherein the antigen-binding region capable of binding to human CD3ε comprises (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

34. The bispecific antibody according to claim 32 or 33, comprising:

(i) an antigen-binding region capable of binding to human PD-L1 comprising a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7 **[338],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or
(ii) an antigen-binding region capable of binding to human PD-L1 comprising a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17 **[338],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or
(iii) an antigen-binding region capable of binding to human PD-L1 comprising a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 sequences having the sequences as set forth in SEQ ID NO:23, the sequence DDN, and the sequence as set forth in SEQ ID NO:24 **[547],** respectively, and an antigen-binding region capable of binding to human CD3ε comprising (a) a heavy chain variable region (VH) comprising CDR1, CDR2, and CDR3 having the sequences as set forth in SEQ ID NOs: 26, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

35. The bispecific antibody according to any one of claims 32 to 34, wherein the antigen-binding region capable of binding to human CD3ε comprises a VH sequence as set forth in SEQ ID NO:25 and a VL sequence as set forth in SEQ ID NO:29.

36. The bispecific antibody according to claim 32, wherein said bispecific antibody:

(i) has a lower affinity for human CD3ε binding as compared to an antibody having an antigen-binding region capable comprising a VH sequence as set forth in SEQ ID NO:25 and a VL sequence as set forth in SEQ ID NO:29, preferably wherein said affinity is at least 2-fold lower, e.g. at least 5-fold lower, such as at least 10-fold lower, e.g. at least 25-fold lower, such as at least 50-fold lower, and

(ii) is capable of mediating concentration-dependent cytotoxicity of MDA-MB-231 cells, PC-3 cells and/or HELA cells when using PBMCs or purified T cells as effector cells, e.g. when assayed as described in Example 11 herein.

37. The bispecific antibody according to claim 36, wherein the antigen-binding region capable of binding to human CD3ε comprises:

(i) a heavy chain variable region (VH) comprising CDR1, CDR2, and CDR3 having the sequences as set forth in SEQ ID NOs: 99, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(ii) a heavy chain variable region (VH) comprising CDR1, CDR2, and CDR3 having the sequences as set forth in SEQ ID NOs: 100, 27, and 28, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iii) a heavy chain variable region (VH) comprising CDR1, CDR2, and CDR3 having the sequences as set forth in SEQ ID NOs: 26, 27, and 101, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(iv) a heavy chain variable region (VH) comprising CDR1, CDR2, and CDR3 having the sequences as set forth in SEQ ID NOs: 26, 27, and 102, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(v) a heavy chain variable region (VH) comprising CDR1, CDR2, and CDR3 having the sequences as set forth in SEQ ID NOs: 26, 27, and 103, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vi) a heavy chain variable region (VH) comprising CDR1, CDR2, and CDR3 having the sequences as set forth in SEQ ID NOs: 26, 27, and 104, respectively, and a light chain variable region (VL) comprising CDR1, CDR2, and CDR3 having the sequences as set forth in SEQ ID NO:30, the sequence GTN, and the sequence as set forth in SEQ ID NO:31, respectively, or

(vii) a heavy chain variable region (VH) comprising CDR1, CDR2, and CDR3 having the sequences as set forth in SEQ ID NOs: 26, 27, and 105, respectively, and a light chain variable region (VL) comprising CDR1, CDR2, and CDR3 having the sequences as set forth in SEQ ID NO:30, the sequence GTN, and the sequence as set forth in SEQ ID NO:31, respectively.

38. The bispecific antibody according to claim 36 or 37, wherein the antigen-binding region capable of binding to human CD3ε comprises:

(i) a VH sequence as set forth in SEQ ID NO:39 and a VL sequence as set forth in SEQ ID NO:29, or

(ii) a VH sequence as set forth in SEQ ID NO:40 and a VL sequence as set forth in SEQ ID NO:29, or

(iii) a VH sequence as set forth in SEQ ID NO:41 and a VL sequence as set forth in SEQ ID NO:29, or

(iv) a VH sequence as set forth in SEQ ID NO:42 and a VL sequence as set forth in SEQ ID NO:29, or

(v) a VH sequence as set forth in SEQ ID NO:43 and a VL sequence as set forth in SEQ ID NO:29, or

(vi) a VH sequence as set forth in SEQ ID NO:44 and a VL sequence as set forth in SEQ ID NO:29, or

(vii) a VH sequence as set forth in SEQ ID NO:45 and a VL sequence as set forth in SEQ ID NO:29.

39. A multispecific antibody comprising a first antigen-binding region capable of binding to human PD-L1 and a second antigen-binding region capable of binding to a second antigen or to a different epitope of human PD-L1, wherein said antigen-binding region capable of binding to human PD-L1 has the features set forth in any one of claims 1-31.

40. The multispecific antibody according to claim 39, wherein said antigen-binding region capable of binding to human PD-L1 comprises a heavy chain variable region (VH) comprising CDR1, CDR2, and CDR3 sequences and a light chain variable region (VL) comprising CDR1, CDR2, and CDR3 sequences, wherein the VH CDR3 sequence is selected from the group consisting of the sequences set forth in: SEQ ID NO:4 **[338]**, SEQ ID NO:11 **[511]** and SEQ ID NO:21 **[547].**

41. The multispecific antibody according to claim 40, wherein said first antigen-binding region capable of binding to human PD-L1 comprises:

(i) a heavy chain variable region (VH) comprising CDR1, CDR2, and CDR3 sequences as set forth in SEQ ID NOs: 2, 3 and 4, respectively, and a light chain variable region (VL) comprising CDR1, CDR2, and CDR3 having the sequences as set forth in SEQ ID NO:6, the sequence KAS, and the sequence as set forth in SEQ ID NO:7, respectively **[338],** or

(ii) a heavy chain variable region (VH) comprising CDR1, CDR2, and CDR3 sequences as set forth in SEQ ID NOs: 9, 10 and 11, respectively, and a light chain variable region (VL) comprising CDR1, CDR2, and CDR3 having the sequences as set forth in SEQ ID NO:16, the sequence EDS, and the sequence as set forth in SEQ ID NO:17, respectively **[511],** or

(iii) a heavy chain variable region (VH) comprising CDR1, CDR2, and CDR3 sequences as set forth in SEQ ID NOs: 19, 20 and 21, respectively, and a light chain variable region (VL) comprising CDR1, CDR2, and CDR3 having the sequences as set forth in SEQ ID NO:23, the sequence DDN, and the sequence as set forth in SEQ ID NO:24, respectively **[547].**

42. The multispecific antibody according to claim 40 or 41, wherein said first antigen-binding region capable of binding to human PD-L1 comprises a VH sequence which has at least 90%, at least 95%, at least 97%, at least 99%, or 100% amino acid sequence identity to a VH sequence selected from the group consisting of the sequences set forth in: SEQ ID NO:1 **[338],** SEQ ID NO:8 **[511]** and SEQ ID NO:18 **[547].**

43. The multispecific antibody according to any one of claims 40 to 42, wherein said first antigen-binding region capable of binding to human PD-L1 comprises a VL sequence which has at least 90%, at least 95%, at least 97%, at least 99%, or 100% amino acid sequence identity to a VL sequence selected from the group consisting of the sequences set forth in: SEQ ID NO:5 **[338],** SEQ ID NO:15 **[511]**and SEQ ID NO:22 **[547].**

44. The multispecific antibody according to any one of claims 40 to 43, wherein said first antigen-binding region capable of binding to human PD-L1 comprises:

(i) a VH sequence which has at least 90%, at least 95%, at least 97%, at least 99%, or 100% amino acid sequence identity to the VH sequence set forth in: SEQ ID NO:1 and a VL sequence which has at least 90%, at least 95%, at least 97%, at least 99% or 100% amino acid sequence identity to the VL sequence set forth in: SEQ ID NO:5 **[338],** or

(ii) a VH sequence which has at least 90%, at least 95%, at least 97%, at least 99%, or 100% amino acid sequence identity to the VH sequence set forth in: SEQ ID NO:8 and a VL sequence which has at least 90%, at least 95%, at least 97%, at least 99% or 100% amino acid sequence identity to the VL sequence set forth in: SEQ ID NO:15 **[511],** or

(iii) a VH sequence which has at least 90%, at least 95%, at least 97%, at least 99%, or 100% amino acid sequence identity to the VH sequence set forth in: SEQ ID NO:18 and a VL sequence which has at least 90%, at least 95%, at least 97%, at least 99% or 100% amino acid sequence identity to the VL sequence set forth in: SEQ ID NO:22 **[547].**

45. The multispecific antibody according to any one of claims 40 to 44, wherein said VH and VL sequences each comprise three CDR sequences, CDR1, CDR2 and CDR3, respectively, and four framework sequences, FR1, FR2, FR3 and FR4, respectively, and wherein the respective combined FR1, FR2, FR3 and FR4 framework sequences of the VH have at least 90%, at least 95%, at least 97%, or at least 99% amino acid sequence identity to the respective combined FR1, FR2, FR3 and FR4 framework sequences of said VH sequences and wherein the VH CDR sequences are not mutated and wherein the respective combined FR1, FR2, FR3 and FR4 framework sequences of the VL have at least 90%, at least 95%, at least 97%, or at least 99% amino acid sequence identity to the respective combined FR1, FR2, FR3 and FR4 framework sequences of said VL sequences and wherein the VL CDR sequences are not mutated.

46. The multispecific antibody according to any one of claims 40 to 45, wherein said first antigen-binding region capable of binding to human PD-L1 comprises:

(i) a VH sequence as set forth in SEQ ID NO: 1 and a VL sequence as set forth in SEQ ID NO:5 **[338],** or

(ii) a VH sequence as set forth in SEQ ID NO:8 and a VL sequence as set forth in SEQ ID NO: 15 **[511],** or

(iii) a VH sequence as set forth in SEQ ID NO:18 and a VL sequence as set forth in SEQ ID NO:22 **[547].**

47. A multispecific antibody comprising a first antigen-binding region capable of binding to human PD-L1 and a second antigen-binding region capable of binding to a second antigen or to a different epitope of human PD-L1, wherein said antibody:

(i) competes for binding to human PD-L1 with an antibody comprising a VH sequence as set forth in SEQ ID NO:8 and a VL sequence as set forth in SEQ ID NO:15, but does not compete for binding to human PD-L1 with an antibody comprising a VH sequence as set forth in SEQ ID NO:18 and a VL sequence as set forth in SEQ ID NO:22, or
(ii) competes for binding to human PD-L1 with an antibody comprising a VH sequence as set forth in SEQ ID NO:18 and a VL sequence as set forth in SEQ ID NO:22, but does not compete for binding to human PD-L1 with an antibody comprising a VH sequence as set forth in SEQ ID NO:8 and a VL sequence as set forth in SEQ ID NO:15.

48. The multispecific antibody according to claim 47, wherein said antibody competes for binding to human PD-L1 with an antibody comprising a VH sequence as set forth in SEQ ID NO: 1 and a VL sequence as set forth in SEQ ID NO:5.

49. A multispecific antibody comprising a first antigen-binding region capable of binding to human PD-L1 and a second antigen-binding region capable of binding to a second antigen or to a different epitope of human PD-L1, wherein the binding of said antibody to human PD-L1 is not displaced by an antibody comprising a VH sequence as set forth in SEQ ID NO:53 and a VL sequence as set forth in SEQ ID NO:57.

50. The multispecific antibody according to claim 49, wherein the antibody inhibits the binding of human PD-L1 to human PD-1.

51. The multispecific antibody according to claim 49 or 50, wherein the antibody competes for binding to human PD-L1 with an antibody comprising a VH sequence as set forth in SEQ ID NO:18 and a VL sequence as set forth in SEQ ID NO:22.

52. The mulitspecific antibody according to any of claims 49 to 51, wherein the binding of said antibody to human PD-L1 is blocked by an antibody comprising a VH sequence as set forth in SEQ ID NO:18 and a VL sequence as set forth in SEQ ID NO:22.

53. A multispecific antibody comprising a first antigen-binding region capable of binding to human PD-L1 and a second antigen-binding region capable of binding to a second antigen or to a different epitope of human PD-L1, wherein said first antigen-binding region:

(i) is capable of binding to the same epitope of human PD-L1 as an antibody comprising a VH sequence as set forth in SEQ ID NO:1 and a VL sequence as set forth in SEQ ID NO:5 **[338],** or
(ii) is capable of binding to the same epitope of human PD-L1 as an antibody comprising a VH sequence as set forth in SEQ ID NO:8 and a VL sequence as set forth in SEQ ID NO:15 **[511],** or
(iii) is capable of binding to the same epitope of human PD-L1 as an antibody comprising a VH sequence as set forth in SEQ ID NO:18 and a VL sequence as set forth in SEQ ID NO:22 **[547].**

54. The multispecific antibody according to any one of claims 40 to 53, wherein the antibody is bispecific.

55. The multispecific antibody according to claim 54, wherein the antbody is bivalent.

56. The multispecific antibody according to any one of claims 40 to 55, wherein the antibody is capable of binding a second antigen and said second antigen is not human CD3$\varepsilon$.

57. The antibody according to any one of the preceding claims, wherein the antibody is a full-length antibody.

58. The antibody according to claim 57, wherein the antibody is a full-length IgG1 antibody.

59. The antibody according to any one of the preceding claims, wherein the antibody is an antibody fragment.

60. The antibody according to any one of claims 32 to 59, wherein the antibody comprises two half-molecules each comprising an antigen-binding region, wherein

(i) the half-molecule(s) comprising the antigen-binding region capable of binding to human PD-L1 is/are chimeric, and/or
(ii) the half-molecule comprising the antigen-binding region capable of binding to human CD3ε (epsilon), if present, is chimeric.

61. The antibody according to any one of the preceding claims , wherein

(i) the antigen-binding region(s) capable of binding to human PD-L1 is/are humanized, and/or
(ii) the antigen-binding region capable of binding to human CD3ε (epsilon), if present, is humanized.

62. The antibody according to any one of the preceding claims, wherein

(i) the antigen-binding region(s) capable of binding to human PD-L1 is/are human, and/or
(ii) the antigen-binding region capable of binding to human CD3ε (epsilon), if present, is human.

63. The antibody according to any one of the preceding claims, wherein each of the antigen-binding regions comprises a heavy chain variable region (VH) and a light chain variable region (VL), and wherein said variable regions each comprise three CDR sequences, CDR1, CDR2 and CDR3, respectively, and four framework sequences, FR1, FR2, FR3 and FR4, respectively.

64. The antibody according to claim 63, wherein the antibody comprises two heavy chain constant regions (CH), and two chain constant regions (CL).

65. The antibody according to any one of the preceding claims, wherein the antibody comprises a first and second heavy chain, wherein each of said first and second heavy chains comprises at least a hinge region, a CH2 and a CH3 region, wherein in said first heavy chain at least one of the amino acids in a position corresponding to a position selected from the group consisting of T366, L368, K370, D399, F405, Y407, and K409 (according to EU numbering) has been substituted, and in said second heavy chain at least one of the amino acids in a position corresponding to a position selected from the group consisting of T366, L368, K370, D399, F405, Y407, and K409 (according to EU numbering) has been substituted, and wherein said first and said second heavy chains are not substituted in the same positions.

66. The antibody according to claim 65, wherein (i) the amino acid in the position corresponding to F405 (according to EU numbering) is L in said first heavy chain, and the amino acid in the position corresponding to K409 (according to EU numbering) is R in said second heavy chain, or (ii) the amino acid in the position corresponding to K409 (according to EU numbering) is R in said first heavy chain, and the amino acid in the position corresponding to F405 (according to EU numbering) is L in said second heavy chain.

67. The antibody according to any one of the preceding claims, wherein said antibody comprises a first and a second heavy chain and wherein one or both heavy chains are modified so that the antibody induces Fc-mediated effector function to a lesser extent relative to an antibody which is identical, except for comprising non-modified first and second heavy chains.

68. The antibody according to claim 67, wherein said Fc-mediated effector function is measured by determining Fc-mediated CD69 expression, by binding to Fcγ receptors, by binding to C1q, or by induction of Fc-mediated cross-linking of FcRs.

69. The antibody according to claim 67 or 68, wherein the heavy and light chain constant sequences have been modified so that said antibody reduces Fc-mediated CD69 expression by at least 50%, at least 60%, at least 70%, at least 80%, at least 90%, at least 99% or 100% when compared to a wild-type antibody wherein said Fc-mediated CD69 expression is determined in a PBMC-based functional assay.

70. The antibody according to any one of the preceding claims, wherein said antibody comprises a first and a second heavy chain, wherein in at least one of said first and second heavy chains one or more amino acids in the positions corresponding to positions L234, L235, D265, N297, and P331 in a human IgG1 heavy chain according to EU numbering, are not L, L, D, N, and P, respectively.

71. The antibody according to claim 70, wherein the positions corresponding to positions L234 and L235 in a human IgG1 heavy chain according to EU numbering are F and E, respectively, in said first and second heavy chains.

72. The antibody according to claim 71, wherein the antibody is a bispecific antibody comprising a first and second heavy chain and wherein the positions corresponding to positions L234 and L235 in a human IgG1 heavy chain according to EU numbering of both the first heavy chain and the second heavy chain are F and E, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

73. The antibody according to claim 70, wherein the positions corresponding to positions L234, L235, and D265 in a human IgG1 heavy chain according to EU numbering are F, E, and A, respectively, in said first and second heavy chains.

74. The antibody according to claim 73, wherein the antibody is a bispecific antibody comprising a first and second heavy chain and wherein the positions corresponding to positions L234, L235, and D265 in a human IgG1 heavy chain according to EU numbering of both the first heavy chain and the second heavy chain are F, E, and A, respectively, and wherein (i) the position corresponding to F405 in a human IgG1 heavy chain according to EU numbering of the first heavy chain is L, and the position corresponding to K409 in a human IgG1 heavy chain according to EU numbering of the second heavy chain is R, or (ii) the position corresponding to K409 in a human IgG1 heavy chain according to EU numbering of the first heavy chain is R, and the position corresponding to F405 in a human IgG1 heavy chain according to EU numbering of the second heavy chain is L.

75. The antibody according to any one of the preceding claims, wherein the antibody does not bind to human PD-L2.

76. The antibody according to any one of the preceding claims, wherein the antibody binds human PD-L1 with a $K_D$ of about $10^{-8}$ M or less, such as about $10^{-9}$ M or less, e.g. about $10^{-10}$ M or less, when determined as described in Example 8 herein.

77. The antibody according to any one of the preceding claims, wherein the antibody mediates concentration-dependent cytotoxicity of MDA-MB-231 cells, PC-3 cells and/or HELA cells when using purified T cells as effector cells, when assayed as described in Example 11 herein.

78. A nucleic acid construct comprising:

(i) a nucleic acid sequence encoding a heavy chain sequence of an antibody comprising an antigen-binding region capable of binding to human PD-L1 as defined in any one of claims 1 to 31, and/or
(ii) a nucleic acid sequence encoding a light chain sequence of an antibody comprising an antigen-binding region capable of binding to human PD-L1 as defined in any one of claims 1 to 31.

79. The nucleic acid construct according to claim 73, further comprising

(i) a nucleic acid sequence encoding a heavy chain sequence of an antibody comprising an antigen-binding region capable of binding to human CD3ε as defined in any one of claims 33 to 38, and
(ii) a nucleic acid sequence encoding a light chain sequence of an antibody comprising an antigen-binding region capable of binding to human CD3ε as defined in any one of claims 33 to 38.

80. An expression vector comprising a nucleic acid construct as defined in claim 78 or 79.

81. A host cell comprising a nucleic acid construct as defined in claim 78 or 79 or an expression vector as defined in claim 80.

82. The host cell according to claim 81, wherein said host cell is a mammalian cell, such as a Chinese hamster ovary cell.

83. A pharmaceutical composition comprising an antibody according to any one of claims 1 to 77 and a pharmaceutically-acceptable carrier.

84. The antibody according to any one of claims 1 to 77 or the pharmaceutical composition according to claim 83 for use as a medicament.

85. The antibody according to any one of claims 1 to 80 or the pharmaceutical composition according to claim 70 for use in the treatment of cancer.

86. The antibody according to any one of claims 1 to 77 or the pharmaceutical composition according to claim 70 for use in the treatment of a cancer disease **characterised by** the presence of solid tumors.

87. The antibody according to any one of claims 1 to 78 or the pharmaceutical composition according to claim 70 for use in the treatment of a cancer disease selected from the group consisting of: melanoma, ovarian cancer, lung cancer, colon cancer and head and neck cancer.

88. A method of treatment of a disease comprising administering an antibody according to any one of claims 1 to 75 or the pharmaceutical composition according to claim 83 to a subject in need thereof.

89. Use of an antibody according to any one of claims 1 to 77 for the manufacture of a medicament, such as a medicament for the treatment of cancer, e.g. a cancer disease **characterized by** the presence of solid tumors or a cancer disease selected from the group consisting of: melanoma, ovarian cancer, lung cancer, colon cancer and head and neck cancer.

90. The method or use according to any of claims 83 to 89 wherein the method or use comprises combination with one or more further therapeutic agent, such as a chemotherapeutic agent.

91. A method for producing an antibody according to any one of claims 1 to 77, comprising the steps of:

a) culturing a host cell producing a first antibody comprising an antigen-binding region capable of binding to human PD-L1 as defined in any one of claims 1 to 13 and purifying said first antibody from the culture;
b) culturing a host cell producing a second antibody comprising an antigen-binding region capable of binding to a different epitope of PD-L1 or a different antigen, e.g. a human CD3ε-binding region as defined in any one of claims 14 to 19, and purifying said second antibody from the culture;
c) incubating said first antibody together with said second antibody under reducing conditions sufficient to allow the cysteines in the hinge region to undergo disulfide-bond isomerization, and
d) obtaining said bispecific antibody.

92. An anti-idiotypic antibody which binds to the antigen-binding region capable of binding to human PD-L1 as defined in any one of claims 1 to 77.

A

● IgG1-338-FEAR
○ bsIgG1- huCD3-H1L1-FEALx338-FEAR

B

■ IgG1-547-FEAR
□ bsIgG1- huCD3-H1L1-FEALx547-FEAR

C

▲ IgG1-511-LC33S-FEAR
△ bsIgG1- huCD3-H1L1-FEALx511-LC33S-FEAR

Figure 1

Figure 2

D

E

F

Figure 2, cont'd

Figure 3

A

- IgG1-338-FEAR
- bsIgG1-b12-FEALx338-FEAR

B

- IgG1-547-FEAR
- bsIgG1-b12-FEALx547-FEAR

C

- IgG1-511-LC33S-FEAR
- bsIgG1-b12-FEALx511-LC33S-FEAR

Figure 4

A

● IgG1-338-FEAR

○ bsIgG1- huCD3-H1L1-FEALx338-FEAR

B

■ IgG1-547-FEAR

□ bsIgG1- huCD3-H1L1-FEALx547-FEAR

C

▲ IgG1-511-LC33S-FEAR

△ bsIgG1- huCD3-H1L1-FEALx511-LC33S-FEAR

**Figure 5**

D

E

F

Figure 5, cont'd

| Antibody | 321 | MEDI | 338 | MPDL | 421-LC19S | 547 | 511-LC-33S | 476-N101Q-LC33S | 632 | 516 |
|---|---|---|---|---|---|---|---|---|---|---|
| 321 | **0.0** | **0.0** | **0.0** | **0.0** | **0.0** | **0.0** | **0.0** | * | 0.6 | 0.6 |
| MEDI | **0.0** | **0.0** | **0.0** | **0.0** | **0.0** | **0.0** | **0.0** | * | 1.2 | 1.2 |
| 338 | **0.0** | **0.0** | **0.0** | **0.0** | **0.0** | **0.0** | **0.0** | * | 0.6 | 0.6 |
| MPDL | **0.0** | **0.0** | **0.0** | **0.0** | **0.0** | **0.0** | **0.0** | * | 1.0 | **0.0** |
| 421-LC91S | **-0.1** | **0.0** | **-0.1** | **0.0** | **0.0** | **0.0** | 0.3 | 0.4 | 0.2 | **0.0** |
| 547 | **0.0** | **0.0** | **0.0** | **0.0** | **0.0** | **0.0** | 0.7 | 0.8 | 0.7 | 0.7 |
| 511-LC33S | **0.0** | **0.0** | **0.0** | **-0.1** | 0.6 | 0.6 | **0.0** | **0.0** | **0.0** | 0.6 |
| 476-N101Q-LC33S | * | * | * | * | 0.8 | 0.7 | **0.0** | **0.0** | **0.0** | 0.7 |
| 632 | 0.6 | 0.5 | 0.6 | 0.4 | 0.6 | 0.6 | **0.0** | **0.0** | **0.0** | 0.6 |
| 516 | 1.1 | 1.0 | 1.1 | **0.0** | **-0.1** | 0.8 | 1.1 | 1.2 | 1.1 | **0.0** |

A

IgG1-PDL1-MEDI4736-FEAR x IgG1-476-N101Q-FEAR-LC33S

Figure 6

**B**

IgG1-PDL1-MEDI4736-FEAR x IgG1-547-FEAR

**C**

IgG1-PDL1-MEDI4736-FEAR x IgG1-632-FEAR

Figure 6, cont'd

A

● IgG1-338-FEAR

✕ bsIgG1- b12-FEALx338-FEAR

B

■ IgG1-547-FEAR

+ bsIgG1-b12-FEALx547-FEAR

C

▲ IgG1-511-LC33S-FEAR

✳ bsIgG1- b12-FEALx511-LC33S-FEAR

Figure 7

A

B

**Figure 8**

**PBMC donor 4, E:T=10:1**

- ● bsIgG1- huCD3-H1L1-FEALx338-FEAR
- ■ bsIgG1- huCD3-H1L1-FEALx547-FEAR
- ▲ bsIgG1- huCD3-H1L1-FEALx511-LC33S-FEAR

**Figure 8, cont'd**

A

B

Figure 9

**PBMC donor 6, E:T=10:1**

- bsIgG1- huCD3-H1L1-FEALx338-FEAR
- bsIgG1- huCD3-H1L1-FEALx547-FEAR
- bsIgG1- huCD3-H1L1-FEALx511-LC33S-FEAR

**Figure 9, cont'd**

A

**T cells donor 5, E:T=8:1**

- ● bsIgG1- huCD3-H1L1-FEALx338-FEAR
- ■ bsIgG1- huCD3-H1L1-FEALx547-FEAR
- ▲ bsIgG1-huCD3-H1L1-FEALx511-LC33S-FEAR

**T cells donor 6, E:T=8:1**

- ● bsIgG1- huCD3-H1L1-FEALx338-FEAR
- ■ bsIgG1- huCD3-H1L1-FEALx547-FEAR
- ▲ bsIgG1- huCD3-H1L1-FEALx511-LC33S-FEAR

B

**PBMC donor 5, E:T=10:1**

- ● bsIgG1- huCD3-H1L1-FEALx338-FEAR
- ■ bsIgG1- huCD3-H1L1-FEALx547-FEAR
- ▲ bsIgG1- huCD3-H1L1-FEALx511-LC33S-FEAR

**Figure 10**

PBMC donor 6, E:T=10:1

● bsIgG1- huCD3-H1L1-FEALx338-FEAR
■ bsIgG1- huCD3-H1L1-FEALx547-FEAR
▲ bsIgG1- huCD3-H1L1-FEALx511-LC33S-FEAR

Figure 10, cont'd

A

B

- ●
- ■
- ▲
- ✕ IgG1-b12

Figure 11

Figure 12

Figure 13

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004004771 A
- WO 2007005874 A
- WO 2010036959 A
- WO 2010077634 A
- WO 2013079174 A
- WO 2013164694 A
- WO 2013173223 A
- WO 2014022758 A
- WO 2007059782 A, Genmab
- WO 9222653 A
- EP 0629240 A
- WO 2015001085 A, Genmab
- WO 2017009442 A
- WO 2011131746 A, Genmab
- WO 2011069104 A, Roche
- WO 9850431 A
- WO 2011117329 A, Roche
- EP 1870459 A, Amgen
- WO 2009089004 A
- US 201000155133 A, Chugai
- WO 2010129304 A
- WO 2010134666 A
- WO 2014081202 A
- WO 2007110205 A
- WO 2013157953 A, Merus
- WO 201015792 A
- WO 11143545 A
- WO 2012058768 A
- WO 2011028952 A

- WO 2009080254 A
- WO 2009058383 A
- WO 2008003116 A
- US 7262028 B
- WO 2012023053 A
- US 7612181 B, Abbott
- WO 20100226923 A
- WO 2010111625 A
- US 007951918 B
- CN 102250246
- WO 2012025525 A, Roche
- WO 2012025530 A
- WO 2008157379 A
- WO 2010080538 A
- WO 2003074569 A
- WO 2005004809 A
- WO 2009040562 A1
- WO 2005061547 A
- WO 2010059315 A, Merrimack
- WO 2013060867 A, Genmab
- US 5731168 A
- WO 2008119353 A, Genmab
- US 6077 A
- US 835 A
- WO 0070087 A
- WO 200046147 A
- US 5589466 A
- US 5973972 A
- WO 2011066501 A

**Non-patent literature cited in the description**

- **RIETZ** ; **CHEN**. *Am J Transplant*, 2004, vol. 4, 8-14
- **ABIKO et al.** *Br J Cancer*, 2015, vol. 112, 1501-1509
- **DONG et al.** *Nature Medicine*, 2002, vol. 8 (8), 793-800
- **LATCHMAN et al.** *Proc Natl Acad Sci USA*, 2004, vol. 101, 10691-6
- **AZUMA et al.** *Blood*, 2008, vol. 111, 3635-43
- **BUTTE et al.** *Immunity*, 2007, vol. 27, 111-22
- **PARK et al.** *Blood*, 2010, vol. 116, 1291-8
- **FRANCISCO et al.** *J Exp Med*, 2009, vol. 206, 3015-29
- **RITTMEYER et al.** *Lancet*, 2017, vol. 389, 255-265

- **KAUFMAN et al.** *Lancet Oncol.*, 2016, vol. 17 (10), 1374-1385
- **MASSARD et al.** *J Clin Oncol.*, 2016, vol. 34 (26), 3119-25
- Fundamental Immunology. Raven Press, 1989
- **CHOTHIA** ; **LESK**. *J. Mol. Biol.*, 1987, vol. 196, 901-917
- **LEFRANC MP.** *Nucleic Acids Research*, 1999, vol. 27, 209-212
- **EHRENMANN F.** ; **KAAS Q.** ; **LEFRANC M.-P.** *Nucleic Acids Res.*, 2010, vol. 38, D301-307
- **EDELMAN et al.** *Proc Natl Acad Sci USA.*, May 1969, vol. 63 (1), 78-85

- **KABAT et al.** Sequences of Proteins of Immunological Interest. NIH Publication, 1991
- **WARD et al.** *Nature*, 1989, vol. 341, 544-546
- **HOLT et al.** *Trends Biotechnol.*, November 2003, vol. 21 (11), 484-90
- **REVETS et al.** *Expert Opin Biol Ther.*, January 2005, vol. 5 (1), 111-24
- **BIRD et al.** *Science*, 1988, vol. 242, 423-426
- **HUSTON et al.** *PNAS USA*, 1988, vol. 85, 5879-5883
- **E. MEYERS** ; **W. MILLER**. *Comput. Appl. Biosci*, 1988, vol. 4, 11-17
- **NEEDLEMAN** ; **WUNSCH**. *J. Mol. Biol.*, 1970, vol. 48, 444-453
- **ABDICHE et al.** *Plos One*, 2017, vol. 12 (1), e0169535
- **SAMBROOK et al.** Molecular Cloning: A laboratory Manual. Cold Spring Harbor Laboratory Press, 1989
- **KOHLER** ; **MILSTEIN**. *Nature*, 1975, vol. 256, 495
- **MENDEZ et al.** *Nat Genet.*, 1997, vol. 15 (2), 146-56
- **MØLHØJ et al.** *Molecular Immunology*, 2007, vol. 44
- **LABRIJN et al.** *Nat Biotechnol.*, 2009, vol. 27 (8), 767-71
- **KONTERMANN**. *Drug Discov Today*, July 2015, vol. 20 (7), 838-47
- *MAbs*, April 2012, vol. 4 (2), 182-97
- Generation and Characterization of a Dual Variable Domain Immunoglobulin (DVD-Ig™) Molecule. **WU et al.** Antibody Engineering. Springer Berlin Heidelberg, 2010
- **WRANIK et al.** *J. Biol. Chem.*, 01 November 2012, vol. 287 (52), 43331-9
- **BOSTROM et al.** *Science*, 2009, vol. 323, 1610-1614
- **LAFLEUR et al.** *MAbs*, April 2013, vol. 5 (2), 208-18
- **DOPPALAPUDI, V.R. et al.** *Bioorg. Med. Chem. Lett.*, 2007, vol. 17, 501-506
- **LEWIS et al.** *Nat Biotechnol.*, February 2014, vol. 32 (2), 191-8
- **DIMASI et al.** *J Mol Biol.*, 30 October 2009, vol. 393 (3), 672-92
- **PEARCE et al.** *Biochem Mol Biol Int.*, September 1997, vol. 42 (6), 1179-88
- **BLANKENSHIP JW et al.** *AACR 100th Annual meeting*, 2009
- **DEO et al.** *J Immunol.*, 15 February 1998, vol. 160 (4), 1677-86
- **SCHOONJANS**. *J Immunol.*, 15 December 2000, vol. 165 (12), 7050-7
- **LE GALL et al.** *Protein Eng Des Sel.*, April 2004, vol. 17 (4), 357-66
- **LAWRENCE**. *FEBS Lett.*, 03 April 1998, vol. 425 (3), 479-84
- **ZHU et al.** *Immunol Cell Biol.*, August 2010, vol. 88 (6), 667-75
- **HMILA et al.** *FASEB J.*, 2010
- **MARVIN** ; **ZHU**. *Acta Pharmacol Sin*, 2005, vol. 26, 649
- **LINDHOFER et al.** *J Immunol*, 1995, vol. 155, 219
- **SYKES** ; **JOHNSTON**. *Nat Biotech*, 1997, vol. 17, 355-59
- **SCHAKOWSKI et al.** *Mol Ther*, 2001, vol. 3, 793-800
- **BENVENISTY** ; **RESHEF**. *PNAS USA*, 1986, vol. 83, 9551-55
- **WIGLER et al.** *Cell*, 1978, vol. 14, 725
- **CORARO** ; **PEARSON**. *Somatic Cell Genetics*, 1981, vol. 7, 603
- **VAN HEEKE** ; **SCHUSTER**. *J Biol Chem*, 1989, vol. 264, 5503-5509
- Current Protocols in Molecular Biology. Greene Publishing and Wiley InterScience, 1987
- **GRANT et al.** *Methods in Enzymol*, 1987, vol. 153, 516-544
- **BEBBINGTON**. *Biotechnology (NY)*, 1992, vol. 10, 169-175
- **DALL'ACQUA WF et al.** *J Immunol.*, 2006, vol. 177 (2), 1129-1138
- **HEZAREH M**. *J Virol.*, 2001, vol. 75 (24), 12161-12168
- **XU et al.** *Cell Immunol.*, 2000, vol. 200 (1), 16-26
- **OGANESYAN et al.** *Acta Cryst.*, 2008, vol. D64, 700-4
- **CANFIELD et al.** *J. Exp. Med.*, 1991, vol. 173, 1483-91
- **DUNCAN et al.** *Nature*, 1988, vol. 332, 738-40
- **SHIELDS et al.** *J. Biol. Chem.*, 2001, vol. 276, 6591-604
- **IDUSOGIE EE et al.** *J Immunol.*, 2000, vol. 164, 4178-84
- **E LEABMAN et al.** *MAbs*, 2013, vol. 5 (6), 896-903
- **PARREN et al.** *J. Clin Invest.*, 1992, vol. 90, 1537-1546
- **BRUHNS et al.** *Blood*, 2009, vol. 113, 3716-3725
- **LIGHTLE, S. et al.** *Protein Science*, 2010, vol. 19, 753-62
- **BREKKE et al.** *J Immunol*, 2006, vol. 177, 1129-1138
- **DALL'ACQUA WF et al.** *J Immunol*, 2006, vol. 177, 1129-1138
- **DALL'ACQUA et al.** *J. Biol. Chem.*, 2006, vol. 281, 23514-24
- **HINTON et al.** *J. Immunol.*, 2006, vol. 176, 346-56
- **ZALEVSKY et al.** *Nat. Biotechnol.*, 2010, vol. 28, 157-9

- Remington: The Science and Practice of Pharmacy. Mack Publishing Co., 1995
- **ASLANIDIS, C.** ; **P.J. DE JONG**. *Nucleic Acids Res*, 1990, vol. 18 (20), 6069-74
- **LEFRANC MP. et al.** *Nucleic Acids Research*, 1999, vol. 27, 209-212
- **BROCHET X.** *Nucl. Acids Res.*, 2008, vol. 36, W503-508
- **KOZAK et al.** *Gene*, 1999, vol. 234, 187-208
- **BARBAS, CF.** *J Mol Biol.*, 05 April 1993, vol. 230 (3), 812-23
- **PONCELET** ; **CARAYON**. *J. Immunol. Meth.*, 1985, vol. 85, 65-74